# EUROPEAN PATENT SPECIFICATION

(11) **EP 1 725 538 B1**
(45) Date of publication and mention of the grant of the patent: **29.08.2007**
(21) Application number: 05722893.4
(22) Date of filing: 09.02.2005
(51) Int. Cl.: C07D 237/14, C07D 403/12, C07D 401/12, C07D 413/14, C07D 405/12, C07D 409/12, A61K 31/50, A61K 31/501, A61P 9/10, A61P 11/06, A61P 29/00, A61P 35/04, A61P 37/06, A61P 37/08, C07D 453/02

(54) **PYRIDAZINONE UREAS AS ANTAGONISTS OF A4 INTEGRINS**
PYRIDAZINONHARNSTOFFE ALS ANTAGONISTEN VON A4-INTEGRINEN
UREES DE PYRIDAZINONE UTILISEES EN TANT QU'ANTAGONISTES DES INTEGRINES _G(A)4

(30) Priority: 10.02.2004 US 543315 P
(43) Date of publication of application: 29.11.2006
(73) Proprietor: JANSSEN PHARMACEUTICA N.V., 2340 Beerse (BE)
(72) Inventor: BARBAY, Kent, Fort Washington, Pennsylvania 19034 (US); HE, Wei, Audubon, Pennsylvania 19403 (US); GONG, Yong, Warrington, Pennsylvania 18976 (US)
(74) Representative: Fisher, Adrian John
(86) International application number: PCT/US2005/004181
(87) International publication number: WO 2005/077914

(56) References cited:
- EP-A- 1 130 015
- WO-A-96/22966

## Description

### CROSS-REFERENCE TO RELATED APPLICATIONS

This Application claims priority to United States Provisional Patent Application No. 60/543,315, filed February 10, 2004, which is hereby incorporated by reference in its entirety.

### STATEMENT REGARDING FEDERALLY SPONSORED RESEARCH OR DEVELOPMENT

The research and development of the invention described below was not federally sponsored.

### FIELD OF THE INVENTION

The present invention relates to certain compounds, methods for preparing compounds, compositions, intermediates and derivatives thereof and for treating α4 integrin mediated disorders. More particularly, the pyridazinone urea compounds of the present invention are α4β7 integrin inhibitors useful for treating integrin mediated disorders.

### BACKGROUND OF THE INVENTION

The present invention relates to pyridazinone derivatives that inhibit α4 integrins. Many physiological processes require that cells come into close contact with other cells and/or extracellular matrix. Such adhesion events May be required for cell activation, migration, proliferation, and differentiation. -Cell-cell and cell-matrix interactions are mediated through several families of cell adhesion molecules (CAMs) including the selectins, integrins, cadherins, and immunoglobulins. CAMs play a role in both normal and pathophysiological processes. Therefore, the targeting of specific and relevant CAMs in certain disease conditions without interfering with normal cellular functions is essential for an effective and safe therapeutic agent that inhibits cell-cell and cell-matrix interactions.

The integrin superfamily is made up of structurally and functionally related glycoproteins consisting of α and β heterodimeric, transmembrane receptor molecules found in various combinations on nearly every mammalian cell type.

α4β7 is an integrin expressed on leukocytes and is a key mediator of leukocyte trafficking and homing in the gastrointestinal tract. The ligands for α4β7 include mucosal addressing cell adhesion molecule-1 (MAdCAM-1) and, upon activation of α4β7, VCAM-1 and fibronectin. MAdCAM-1 is a member of the lg superfamily and is expressed in vivo on endothelial cells of gut associated mucosal tissues of the small and large intestine ("Peyer's Patches") and lactating mammary glands. (See M. J. Briskin et al., Nature, 363, 461 (1993); A. Hamann et al., J. Immunol., 152, 3282 (1994)). MadCAM-1 can be induced in vitro by proinflammatory stimuli (See E. E. Sikorski et al. J. Immunol., 151, 5239 (1993)). MadCAM-1 is selectively expressed at sites of lymphocyte extravasation and specifically binds to the integrin α4β7.

The expression of α4β7 is elevated in animal models of inflammatory bowel diseases that are analogs of Crohn's disease, ulcerative colitis, hepatitis and pancreatitis in man (van Assche, G., and Rutgeerts, P. Antiadhesion molecule therapy in inflammatory bowel disease. Inflamm. Bowel Dis., 8: 291-300, 2002; Binion, DS. West, G. A., Volk, EE., et al. Acquired increase in leukocyte binding by intestinal microvascular endothelium in inflammatory bowel disease. Lancet, 352: 1742-1746, 1998; Souza, HS., Elia, CC., Spencer, J., and MacDonald, T. T. Expression of lymphocyte-endothelial receptor-ligand pairs, alpha4-beta7/MAdCAM-1 and OX40/OX40 ligand in the colon and jejunum of patients with inflammatory bowel diseae. Gut, 45: 856-863, 1999; Briskin, J., Winsor-Hines, D., Shyjan, A., Cochran. N., Bloom, S., Wilson, J., McEvoy, L. M., Butcher, E. C., Kassam, N., Mackay, C. R., Newman, W, and Ringler, D. J. Human mucosol addressin cell adhesion molecule-1 is preferentially expressed in intestinal tract and associated lymphoid tissue. Am. J.Pathol., 151: 97-110, 1997; Kawachi, S., Jennings, S., Panes, J., Cockrell, A., Laroux, F. S., Gray, L., Perry, M., van der Heyde, H., Balish, E., Granger, D. N., Specian, R. A., and Grisham. M. B. Cytokine and endothelial cell adhesion molecule expression in interleukin-10 deficient mice. Am J. Physiol. Gastrointest. Liver Phsyiol., 278: G734-G743, 2000).

Evidence for potential therapeutic application is offered by in vivo studies showing inhibition of cell recruitment by monoclonal antibodies to α4 (Issekutz, T. B., Inhibition of in vivo lymphocyte migration to inflammation and homing to lymphoid tissues by the TA-2 monoclonal antibody. A likely role for VLA-4 in vivo. J. Immunol., 146: 4178-4184, 1991; Richards, I. M., Kolbasa, K. P., Hatfield, C. A. Winterrowd, G. E., Vonderfecth, S. L., Fidler, S. F., Giriffin, R. L. Bradhler, J. R., Krzisicki, R. F., and Sly, L. M. Role of very late activation antigen-4 in the antigen induced accumulation of eosinophils and lymphocytes in the lungs and airway lumen of sensitised brown norway rats. Am. J. Respir. Cell. Mol. Biol., 15: 172, 1996; Issekutz, A. C. Ayer, L., Miyasaka, M., and lssekutz, T. B. Treatment of established adjuvant arthritis in rats with monoclonal antibody to CD18 and very late activation antigen-4 integrins suppressess neutrophils and T-lymphocyte migration to the joints and improves clinical disease. Immunology, 88: 659, 1996).

In vivo studies with monoclonal antibody to α4 integrin in animal models of IBD also demonstrate efficacy (Podolsky, D. K., Lobb, R.R., King, N., Benjamin, C. D., Pepinsky, B., Sehgal, P., deBeaumont, M.; J. Clin. Invest., 92: 372, 1993), but more critically, studies with monoclonal antibody to MAdCAM-1 or α4β7 demonstrate efficacy in IBD by decreasing the number of adherent cells in high endothelial venules in animals with colitis and by reducing disease severity (Shigematsu, T., Specian, R. D., Wolf, R. E., Grisham, M. B., and Granger, D. N. MADCAM mediates lymphocyte - endothelial cell adhesion in a murine model of chronic colitis; Am. J. Physiol. Gastrointest. Liver Physiol., 281: G1309-13015, 2001; Picarella, D., Hurlbut, P., Torrman, J., Shi, X., Butcher, E., and Ringler, D. J. Monoclonal antibodies specific for β7 integrin and mucosal addressin cell adhesion molecule-1 (MAdCAM-1) reduce inflammation in the colon of scid mice reconstituted with CD45RBhigh CD4+ T cells. J. Immuol., 158: 2099-2106. 1997).

A murine homolog to antibody LDP-02 is specific for α4β7 integrin and when administered to cotton-topped tamarins was found to prevent development of spontaneous gastroenteritis that is known to afflict these animals when in captivity. The α4β7 integrin is also seen to be required for recruitment of mast cell precursors to the intestine, but has no influence on their recruitment to the lung (Gurish, M. F., Tao, H., Abonia, P. J., Arya, A., Friend, D. S., Parker, C. M., and Austen, K. F. Intestinal mast cell progenitors require CD49dβ7 (α4β7 integrin) for tissue specific homing. J. Exp. Med., 194: 1243-1252,2001).

Antibodies or antisense olignucleotides to VCAM-1 and ICAM-1 have been reported by some not to have activity in animal models of IBD (30), although there are other reports showing efficacy. (Taniguchi,, T., Tsukada, H., Nakamura, J., Kodama, M., Fukuda, K., Saito, T., Miyuasaka, M., and Seino, Y. Effects of the anti-ICAM-1 monoclonal antibody on dextran sodium sulphate-induced colitis in rats. J. Gastroenterol. Hepatol., 13: 945-949, 1998; Sans, M., Panes, J., Ardite, E., Elizalde, J. L., Arce, Y., Elena, M., Palacin, A., Fernandez-Checa, J. C., Anderson, D. C., Lobb, R., and Pique, J. M. VCAM-1 and ICAM-1 mediate leukocyte-endothelial cell adhesion in rat experimental colitis. Gastroenterology, 116: 874-883, 1999; Bums, R C., Rivera-Nieves, J., Moskaluk, C.A., et al., Antibody blockade of ICAM-1 and VCAM-1 ameloriates inflammation in the SAMP-1/Yit adoptive transfer model of Crohn's disease in mice. Gastroenterology 121: 1428-1436, 2001; Bennet, C. F., Hall, W., Jacoby, H. I. An ICAM-1 antisense oligonucleotide prevents and reverses dextran sulfate sodium-induced colitis in mice. J. Pharmacol Exp Ther., 280: 988-1000, 1999). The fact that α4β7-MAdCAM-1 interactions are essentially confined to the Gl tract and mesenteric system strongly suggests that an antagonist of this cell-cell adhesion pair will provide a specific anti-inflammatory response confined to the Gl tract and mesenteric systems without the potential for systemic immune suppression.

There still remains a need for low molecular weight, specific inhibitors of α4-dependent cell adhesion that have improved pharmacokinetic and pharmacodynamic properties such as oral bioavailability and significant duration of action. Such compounds would prove useful for the treatment, prevention, or suppression of various pathologies mediated by α4β7 binding and cell adhesion and activation.

It is an object of the present invention to provide pyridazinone urea compounds that are integrin inhibitors, in particular, inhibitors of α4β7, useful for treating inflammatory, immunological, and integrin-mediated disorders. It is another object of the invention to provide a process for preparing pyridazinone urea compounds, compositions, intermediates and derivatives thereof. It is a further object of the invention to provide methods for treating inflammatory and α4β7 integrin-mediated disorders.

### SUMMARY OF THE INVENTION

The present invention is directed to a compound of Formula (I) 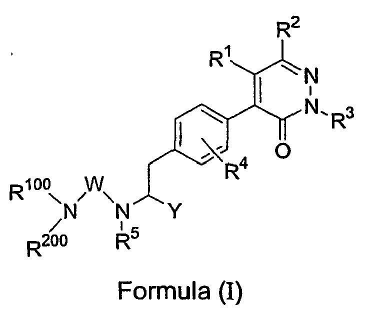
- **R¹** is: independently selected from the group consisting of hydrogen, C₁₋₆alkyl, C₁₋₆alkoxy, aryl, heteroaryl, heterocyclyl, benzo fused heterocyclyl, benzo fused cycloalkyl, heteroaryl fused heterocyclyl, heteroaryl fused cycloalkyl, aryloxy, heteroaryloxy, heterocyclyloxy, cycloalkyloxy, -NR¹⁰R²⁰, halogen, hydroxy, and -S(C₁₋₆)alkyl; wherein C₁₋₆alkoxy is optionally substituted with one to four substituents independently selected from R^{a}; wherein **R^{a}** is independently selected from the group consisting of aryl, heteroaryl, heterocyclyl, cycloalkyl, (C₁₋₆)alkoxycarbonyl, carboxy, amino, alkylamino, dialkylamino, hydroxy(C₁₋₆)alkoxy, one to three halogen atoms, and hydroxy;
wherein **R¹⁰** and **R²⁰** are independently selected from the group consisting of hydrogen, C₁₋₆alkyl, allyl, halogenated C₁₋₆alkyl, hydroxy, hydroxy(C₁₋₄)alkyl, aryl(C₁₋₄)alkyl, aryl, and cycloalkyl; additionally, R¹⁰ and R²⁰ are optionally taken together with the atoms to which they are attached to form a five to seven membered monocyclic ring;
wherein the aryl and aryloxy substituents of R¹ are optionally substituted with a substituent independently selected from the group consisting of one to three C₁₋₆alkyl substituents, hydroxy(C₁₋₆)alkyl, aryl(C₁₋₆)alkyl, C₁₋₆alkoxy, aryl, heteroaryl, C₁₋₆alkoxycarbonyl, aryl(C₁₋₆)alkoxycarbonyl, C₁₋₆alkylcarbonyl, aminocarbonyl, alkylaminocarbonyl, dialkylaminocarbonyl, hydroxy, cyano, nitro, -SO₂(C₁₋₃)alkyl, -SO₂aryl, -SO₂heteroaryl, trifluoromethyl, trifluoromethoxy, and one to three halogen atoms;
and wherein the heteroaryl and heterocyclyl substituents of R¹ are optionally substituted with one to three substituents independently selected from the group consisting of C₁₋₆alkyl, C₁₋₆alkoxy, aryl, heteroaryl, halogen, and hydroxy;
additionally, R¹ and R² are optionally taken together with the atoms to which they are attached to form a five to seven membered carbocyclic or heterocyclic ring;
- **R²** is: independently selected from the group consisting of hydrogen, C₁₋₆alkyl, C₁₋₆alkoxy, C₂₋₆alkenyloxy, hydroxy, amino, alkylamino, dialkylamino, and halogen; wherein R¹ and R² are optionally taken together with the atoms to which they are attached to form a five to seven membered carbocyclic or heterocyclic ring;
- **R³** is: independently selected from the group consisting of hydrogen, C₁₋₆alkyl, C₂₋₆alkenyl, C₂₋₆alkynyl, aryl, heteroaryl, heterocyclyl, and cycloalkyl; wherein alkyl, alkenyl, and alkynyl are optionally substituted with a substituent independently selected from the group consisting of aminocarbonyl, alkylaminocarbonyl, dialkylaminocarbonyl, aryl, heteroaryl, heterocyclyl, cycloalkyl, carboxy, one to three halogen atoms, hydroxy, and -C(=O)C₁₋₆alkyl;
- **R⁴** is: independently selected from the group consisting of hydrogen, fluorine, chlorine, and methyl;
- **R⁵** is: hydrogen or C₁₋₃alkyl, provided that R⁵ is C₁₋₃alkyl only when taken with Y and the atoms to which R⁵ and Y are attached to form a five to seven membered heterocycle;
- **Y** is: independently selected from the group consisting of hydroxymethyl, -C(=O)NH₂, -C(=O)NH(OH), -C(=O)NH(C₁₋₆alkyl), -C(=O)NH(hydroxy(C₁₋₆)alkyl), -C(=O)N(C₁₋₆alkyl)₂, -C(=O)NHSO₂(C₁₋₄)alkyl, carboxy, tetrazolyl, and -C(=O)C₁₋₆alkoxy; wherein said alkoxy is optionally substituted with one to two substituents independently selected from the group consisting of hydroxy, -NR³⁰R⁴⁰, heterocyclyl, heteroaryl, halogen, and -OCH₂CH₂OCH₃; wherein R³⁰ and R⁴⁰ are independently selected from the group consisting of hydrogen, C₁₋₆alkyl, hydroxy, and hydroxy(C₁₋₄)alkyl, wherein said R³⁰ and R⁴⁰ are optionally taken together with the atoms to which they are attached to form a five to ten membered monocyclic ring;
- **W** is: independently selected from the group consisting of -C(=O)- and -C(=S)-;
- **R¹⁰⁰** and **R²⁰⁰** are: independently selected from the group consisting of hydrogen, C₁₋₆alkyl, C₁₋₆alkenyl, C₁₋₆alkynyl, aryl, heteroaryl, cycloalkyl, polycycloalkyl, heterocyclyl, hydroxy, and arylamino;
wherein the alkyl, C₁₋₆alkenyl, C₁₋₆alkynyl, and alkoxy substituents of R¹⁰⁰ and R²⁰⁰ are optionally substituted with a substituent independently selected from the group consisting of aryl, heteroaryl, heterocyclyl, hydroxy, one to three halogen atoms, amino, C₁₋₆alkylamino, hydroxy(C₁₋₆)alkylamino, di(C₁₋₆)alkylamino, -C(=O)amino, and -C(=O)C₁₋₆alkoxy; wherein said aryl, heteroaryl, and heterocyclyl substituents may be optionally substituted with up to four substituents selected from the group consisting of C₁₋₆alkyl, C₁₋₆alkoxy, halogen, aryl, amino, alkylamino, dialkylamino, hydroxy, polycycloalkyl, and heteroaryl; and said heterocyclyl is optionally substituted with one to three oxo substituents;
wherein said aryl, heteroaryl, and heterocyclyl of R¹⁰⁰ and R²⁰⁰ are optionally substituted with one to four substituents independently selected from the group consisting of C₁₋₆alkyl, trifluoroalkyl, C₁₋₆alkoxy, trifluoroalkoxy, halogen, aryl, amino, alkylamino, dialkylamino, arylamino, hydroxy, polycycloalkyl, and heteroaryl; additionally heterocyclyl is optionally substituted with one to three oxo substituents;
and wherein R¹⁰⁰ and R²⁰⁰ are optionally taken with the nitrogen atom to which they are both attached to form a five to ten membered heterocycle or a nine to ten membered benzo-fused heterocycle; wherein the heterocycle is optionally substituted with up to four substituents independently selected from the group consisting of C₁₋₆alkyl, C₁₋₆alkoxy, halogen, aryl, heteroaryl, amino, alkylamino, dialkylamino, hydroxy, polycycloalkyl, and oxo;
and an optical isomer, enantiomer, diastereomer, racemate, or pharmaceutically acceptable salt thereof.

Illustrative of the invention is a pharmaceutical composition comprising a pharmaceutically acceptable carrier and any of the compounds described above.

The present invention is also directed to methods for producing the instant pyridazinone urea compounds and pharmaceutical compositions and medicaments thereof.

The present invention is further directed to the present compounds for use in treating or ameliorating an α4 integrin-mediated disorder. In particular, the compounds of the present invention are directed for use in treating or ameliorating an α4 integrin mediated disorder such as, multiple sclerosis, asthma, allergic rhinitis, allergic conjunctivitis, inflammatory lung disease, rheumatoid arthritis, septic arthritis, type I diabetes, organ transplantation rejection, restenosis, autologous bone marrow transplantation, inflammatory sequelae of viral infections, myocarditis, inflammatory bowel disease including ulcerative colitis and Crohn's disease, certain types of toxic and immune based nephritis, contact dermal hypersensitivity psoriasis, tumor metastasis, atherosclerosis and hepatitis.

### DETAILED DESCRIPTION OF THE INVENTION

An embodiment of the present invention includes compounds of Formula (I): 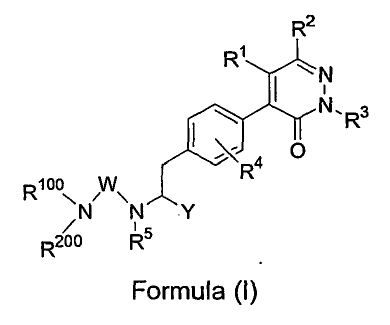 wherein:
- R¹ is: independently selected from the group consisting of hydrogen, C₁₋₄alkyl, C₁₋₄alkoxy, aryl, heteroaryl, heterocyclyl, benzo fused cycloalkyl benzo fused heterocyclyl, aryloxy, heteroaryloxy, heterocyclyloxy, cycloalkyloxy, -NR¹⁰R²⁰, halogen, hydroxy, and -S(C₁₋₆)alkyl; wherein C₁₋₄alkoxy of R¹ is optionally substituted with one to three substituents independently selected from R^{a};
wherein R^{a} is independently selected from the group consisting of aryl, heteroaryl, heterocyclyl, cycloalkyl, dialkylamino, hydroxy(C₁₋₆)alkoxy, one to three halogen atoms, or hydroxy;
wherein R¹⁰ and R²⁰ are independently selected from the group consisting of hydrogen, C₁₋₆alkyl, allyl, halogenated C₁₋₆alkyl, and cycloalkyl; additionally, R¹⁰ and R²⁰ are optionally taken together with the atoms to which they are attached to form a five to seven membered monocyclic ring;
wherein the aryl and aryloxy substituents of R¹ are optionally substituted with a substituent independently selected from the group consisting of C₁₋₆alkyl, C₁₋₆alkoxy, phenyl, heteroaryl, aminocarbonyl, alkylaminocarbonyl, dialkylaminocarbonyl, hydroxy, cyano, nitro, -SO₂(C₁₋₃)alkyl, -SO₂aryl, trifluoromethyl, trifluoromethoxy, and halogen;
and wherein the heteroaryl and heterocyclyl substituents of R¹ are optionally substituted with one to three substituents independently selected from the group consisting of C₁₋₆alkyl, halogen, and hydroxy;
additionally, R¹ and R² are optionally taken together with the atoms to which they are attached to form a five to seven membered carbocyclic or heterocyclic ring.

An embodiment of the present invention includes compounds of Formula (I) wherein:
- R¹ is: independently selected from the group consisting of hydrogen, ethyl, methoxy, ethoxy, 2-hydroxyeth-1-oxy, iso-propoxy, iso-butoxy, difluoromethoxy, 2,2,2-trifluoro-eth-1-oxy, benzyloxy, cyclopropylmethoxy, pyridin-3-ylmethoxy, (1-methyl)-pyrrolidinyl-3-oxy, cyclobutyloxy, cyclopentyloxy, cyclohexyloxy, indazol-1-yl, thiophen-3-yl, [1,3]benzodioxol-5-yl, (2-methyl)-imidazol-1-yl, (1-methyl)-piperidin-4-yloxy, 2-(morpholin-4-yl)-ethoxy, (4-bromo)-pyrazol-1-yl, N-pyrrolidinyl, (3, 5-dimethyl)-pyrazol-1-yl, morpholin-4-yl, hydroxy, -(OCH₂CH₂)₂OH, phenyl (optionally substituted with a substituent independently selected from the group consisting of -SO₂Me, -C(=O)NH₂, -OCF₃, -CF₃, cyano, fluoro, and methoxy), amino, cyclopropylamino, allylamino, methylamino, hydroxy, chloro, and -SMe;
and wherein R¹ is optionally taken together with R² to form a 1,4-dioxanyl or a oxazinyl.

Another embodiment of the present invention includes compounds of Formula (I) wherein R¹ is methoxy.

An embodiment of the present invention includes compounds of Formula (I) wherein R² is independently selected from the group consisting of hydrogen, C₁₋₄alkyl, C₁₋₄alkoxy, C₂₋₄alkenyloxy, hydroxy, amino, and halogen; wherein R¹ and R² are optionally taken together with the atoms to which they are attached to form a five to seven membered carbocyclic or heterocyclic ring.

An embodiment of the present invention includes compounds of Formula (I) wherein R² is independently selected from the group consisting of hydrogen, C₁₋₄alkyl, C₁₋₄alkoxy, hydroxy, amino, alkylamino, and halogen; wherein R² is optionally taken together with R¹ to form a 1,4-dioxanyl or a oxazinyl ring.

An embodiment of the present invention includes compounds of Formula (I) wherein R² is independently selected from the group consisting of hydrogen, C₁₋₄alkoxy, amino, and alkylamino; wherein R² is optionally taken together with R¹ to form a 1,4-dioxanyl or a oxazinyl ring.

An embodiment of the present invention includes compounds of Formula (I) wherein R³ is independently selected from the group consisting of hydrogen, C₁₋₆alkyl, aryl, heteroaryl, heterocyclyl, and cycloalkyl; wherein C₁₋₆alkyl is optionally substituted with a substituent independently selected from the group consisting of -C(=O)NH₂, aryl, heteroaryl, heterocyclyl, cycloalkyl, carboxy, one to three halogen atoms, hydroxy, and -C(=O)C₁₋₆alkyl.

An embodiment of the present invention include compounds of Formula (I) wherein R³ is independently selected from the group consisting of hydrogen, C₁₋₄alkyl, cycloalkyl, and aryl; wherein C₁₋₄alkyl is optionally substituted with a substituent independently selected from the group consisting of -C(=O)C₁₋₄alkyl, -C(=O)NH₂, carboxy, heterocyclyl, phenyl, cyclopropyl, hydroxy, and one to three fluorine atoms.

An embodiment of the present invention includes compounds of Formula (I) wherein R³ is independently selected from the group consisting of hydrogen, C₁₋₄alkyl, and phenyl; wherein C₁₋₄alkyl is optionally substituted with a substituent independently selected from the group consisting of -C(=O)C₁₋₄alkyl, -C(=O)NH₂, carboxy, morpholinyl, cyclopropyl, hydroxy, and one to three fluorine atoms.

An embodiment of the present invention includes compounds of Formula (I) wherein R³ is independently selected from the group consisting of hydrogen, methyl, ethyl, and phenyl; wherein methyl and ethyl are optionally substituted with a substituent independently selected from the group consisting of -C(=O)C₁₋₄alkyl, -C(=O)NH₂, carboxy, morpholinyl, cyclopropyl, hydroxy, and one to three fluorine atoms.

An embodiment of the present invention includes compounds of Formula (I) wherein R³ is independently selected from hydrogen or 2-hydroxy-eth-1-yl.

An embodiment of the present invention includes compounds of Formula (I) wherein R⁴ is independently selected from the group consisting of hydrogen, fluorine, and chlorine.

Another embodiment of the present invention includes compounds of Formula (I) wherein R⁴ is independently selected from hydrogen or fluorine.

Another embodiment of the present invention includes compounds of Formula (I) wherein R⁴ is hydrogen.

An embodiment of the present invention includes compounds of Formula (I) wherein R⁵ is selected from the group consisting of hydrogen and C₁₋₃alkyl, provided that R⁵ is C₁₋₃alkyl only when taken with Y and the atoms to which R⁵ and Y are attached to form a five to seven membered heterocycle.

Another embodiment of the present invention includes compounds of Formula (I) wherein R⁵ is selected from the group consisting of hydrogen and methylene, provided that R⁵ is methylene only when taken with Y and the atoms to which R⁵ and Y are attached to form a five membered heterocycle.

Another embodiment of the present invention includes compounds of Formula (I) wherein R⁵ is hydrogen.

Another embodiment of the present invention includes compounds of Formula (I) wherein Y is independently selected from the group consisting of hydroxymethyl, -C(=O)NH₂, -C(=O)NH(OH), -C(=O)NH(2-hydroxyeth-1-yl), carboxy, tetrazolyl, -C(=O)NHSO₂(C₁₋₄)alkyl, and -C(=O)C₁₋₆alkoxy; wherein said alkoxy is optionally substituted with one to two substituents independently selected from hydroxy, -NR³⁰R⁴⁰, heterocyclyl, heteroaryl, halogen, or - OCH₂CH₂OCH₃; wherein R³⁰ and R⁴⁰ are independently selected from the group consisting of hydrogen and C₁₋₆alkyl.

An embodiment of the present invention includes compounds of Formula (I) wherein Y is independently selected from the group consisting of carboxy, tetrazolyl, -C(=O)NH(2-hydroxyeth-1-yl) and -C(=O)C₁₋₄alkoxy; wherein said alkoxy is optionally substituted with one to two substituents independently selected from the group consisting of hydroxy, -NH₂, -NH(C₁₋₄)alkyl, -N(C₁₋₄alkyl)₂, heterocyclyl, halogen, and -OCH₂CH₂OCH₃.

An embodiment of the present invention includes compounds of Formula (I) wherein Y is independently selected from the group consisting of carboxy, 1*H*-tetrazol-5-yl, and -C(=O)C₁₋₄alkoxy; wherein said alkoxy is optionally substituted with a substituent independently selected from the group consisting of hydroxy, -NMe₂, morpholin-1-yl, chloro, and -OCH₂CH₂OCH₃.

Another embodiment of the present invention includes compounds of Formula (I) wherein Y is independently selected from the group consisting of carboxy, 1*H*-tetrazol-5-yl, or -C(=O)ethoxy; wherein ethoxy is optionally substituted with a substituent independently selected from the group consisting of hydroxy, chlorine, -NMe₂, and -OCH₂CH₂OCH₃.

An embodiment of the present invention includes compounds of Formula (I) wherein W is independently selected from -C(=O)-or-C(=S)-

An embodiment of the present invention includes compounds of Formula (I) wherein W is -C(=O)-.

An embodiment of the present invention includes compounds of Formula (I) wherein R¹⁰⁰ and R²⁰⁰ are substituents independently selected from the group consisting of hydrogen, C₁₋₄alkyl, C₁₋₄alkynyl, aryl, heteroaryl, cycloalkyl, bridged-polycycloalkyl, heterocyclyl, phenylamino, and hydroxy; wherein said aryl, heteroaryl, and heterocyclyl may be optionally substituted with up to four substituents independently selected from the group consisting of C₁₋₆alkyl, hydroxy(C₁₋₄)alkyl, halogen, aryl, trifluoromethyl, trifluoromethoxy, heteroaryl, and hydroxy;
wherein C₁₋₄alkyl and C₁₋₄alkynyl of R¹⁰⁰ and R²⁰⁰ are optionally substituted with a substituent independently selected from the group consisting of heteroaryl, aryl, hydroxy, one to three halogen atoms, amino, alkylamino, and dialkylamino;
wherein R¹⁰⁰ and R²⁰⁰ are optionally taken with the nitrogen atom to which they are both attached to form a five to seven membered heterocycle or nine to ten membered benzo-fused heterocycle; optionally substituted with up to four substituents selected from C₁₋₆alkyl, halogen, aryl, and heteroaryl.

An embodiment of the present invention includes compounds of Formula (I) wherein R¹⁰⁰ and R²⁰⁰ are independently selected from the group consisting of hydrogen, C₁₋₄alkyl, aryl, cycloalkyl, and heterocyclyl; wherein said aryl and heterocyclyl are optionally substituted with up to four substituents independently selected from the group consisting of C₁₋₃alkyl, halogen, trifluoromethyl, trifluoromethoxy, heteroaryl, and hydroxy;
wherein R¹⁰⁰ and R²⁰⁰ are optionally taken with the nitrogen atom to which they are both attached to form a five to seven membered heterocycle optionally substituted with up to four substituents independently selected from the group consisting of C₁₋₆alkyl, halogen, aryl, and heteroaryl.

Another embodiment of the present invention includes compounds of Formula (I) wherein R¹⁰⁰ and R²⁰⁰ are independently selected from the group consisting of hydrogen, C₁₋₄alkyl, aryl, cycloalkyl, and a five to seven membered heterocyclyl , wherein said heterocyclyl is formed when R¹⁰⁰ and R²⁰⁰ are taken together with the nitrogen atom to which they are both attached);
wherein said aryl and heterocyclyl are optionally substituted with up to four substituents independently selected from the group consisting of C₁₋₃alkyl, halogen, trifluoromethyl, trifluoromethoxy, and hydroxy.

Another embodiment of the present invention includes compounds of Formula (I) wherein R¹⁰⁰ and R²⁰⁰ are substituents independently selected from the group consisting of hydrogen, methyl, ethyl, *I*-but-1-yl, cyclohexyl, 2-methylpiperadin-1-yl, and phenyl; wherein said phenyl is substituted with one to two substitutents independently selected from the group consisting of methyl, chloro, hydroxy, trifluoromethyl, and trifluoromethoxy.
and an optical isomer, enantiomer, diastereomer, racemate, or pharmaceutically acceptable salt thereof.

Another aspect of the present invention is directed to a compound of Formula (1a): 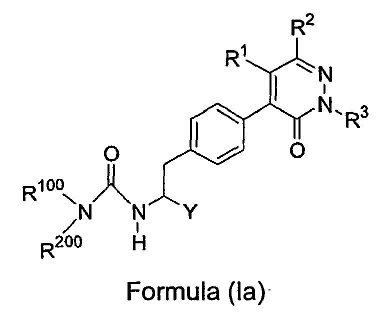 wherein:
- R¹ is: independently selected from the group consisting of hydrogen, C₁₋₄alkyl, C₁₋₄alkoxy, aryl, heteroaryl, heterocyclyl, benzo fused cycloalkyl,benzo fused heterocyclyl, aryloxy, heteroaryloxy, heterocyclyloxy, cycloalkyloxy, -NR¹⁰R²⁰, halogen, hydroxy, and -S(C₁₋₆)alkyl; wherein C₁₋₄alkoxy of R¹ is optionally substituted with one to three substituents independently selected from R^{a};
wherein R^{a} is independently selected from the group consisting of aryl, heteroaryl, heterocyclyl, cycloalkyl, dialkylamino, hydroxy(C₁₋₆)alkoxy, one to three halogen atoms, and hydroxy;
wherein R¹⁰ and R²⁰ are independently selected from the group consisting of hydrogen, C₁₋₆alkyl, allyl, halogenated C₁₋₆alkyl, and cycloalkyl; additionally, R¹⁰ and R²⁰ are optionally taken together with the atoms to which they are attached to form a five to seven membered monocyclic ring;
wherein the aryl and aryloxy substituents of R¹ are optionally substituted with a substituent independently selected from the group consisting of C₁₋₆alkyl, C₁₋₆alkoxy, phenyl, heteroaryl, aminocarbonyl, alkylaminocarbonyl, dialkylaminocarbonyl, hydroxy, cyano, nitro, -SO₂(C₁₋₃)alkyl, -SO₂aryl, trifluoromethyl, trifluoromethoxy, and halogen;
and wherein the heteroaryl and heterocyclyl substituents of R¹ are optionally substituted with one to three substituents independently selected from the group consisting of C₁₋₆alkyl, halogen, and hydroxy;
additionally, R¹ and R² are optionally taken together with the atoms to which they are attached to form a five to seven membered carbocyclic or heterocyclic ring;
- R² is: independently selected from the group consisting of hydrogen, C₁₋₄alkyl, C₁₋₄alkoxy, C₂₋₄alkenyloxy, hydroxy, amino, and halogen; wherein R¹ and R² are optionally taken together with the atoms to which they are attached to form a five to seven membered carbocyclic or heterocyclic ring;
- R³ is: independently selected from the group consisting of hydrogen, C₁₋₄alkyl, cycloalkyl, and aryl; wherein C₁₋₄alkyl is optionally substituted with a substituent independently selected from the group consisting of -C(=O)C₁₋₄alkyl, -C(=O)NH₂, carboxy, heterocyclyl, phenyl, cyclopropyl, hydroxy, and one to three fluorine atoms;
- Y is: independently selected from the group consisting of carboxy, tetrazolyl, -C(=O)NH(2-hydroxyeth-1-yl) and -C(=O)C₁₋₄alkoxy; wherein said alkoxy is optionally substituted with one to two substituents independently selected from the group consisting of hydroxy, -NH₂, -NH(C₁₋₄)alkyl, -N(C₁₋₄alkyl)₂, heterocyclyl, halogen, and -OCH₂CH₂OCH₃;
- R¹⁰⁰ and R²⁰⁰ are: independently selected from the group consisting of hydrogen, C₁₋₄alkyl, aryl, cycloalkyl, and heterocyclyl; wherein said aryl and heterocyclyl are optionally substituted with up to four substituents independently selected from the group consisting of C₁₋₃alkyl, halogen, trifluoromethyl, trifluoromethoxy, heteroaryl, and hydroxy;
wherein R¹⁰⁰ and R²⁰⁰ are optionally taken with the nitrogen atom to which they are both attached to form a five to seven membered heterocycle optionally substituted with up to four substituents independently selected from the group consisting of C₁₋₆alkyl, halogen, aryl, and heteroaryl;
and an optical isomer, enantiomer, diastereomer, racemate, or pharmaceutically acceptable salt thereof.

Another aspect of the present invention is directed to a compound of Formula (1a): 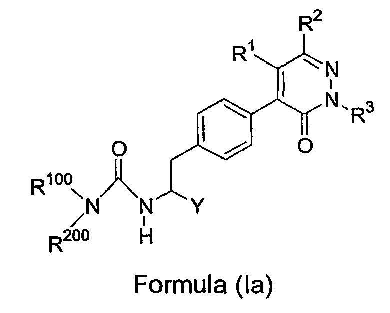 wherein:
- R¹ is: independently selected from the group consisting of hydrogen, ethyl, methoxy, ethoxy, 2-hydroxyeth-1-oxy, iso-propoxy, iso-butoxy, difluoromethoxy, 2,2,2-trifluoro-eth-1-oxy, benzyloxy, cyclopropylmethoxy, pyridin-3-ylmethoxy, (1-methyl)-pyrrolidinyl-3-oxy, cyclobutyloxy, cyclopentyloxy, cyclohexyloxy, indazol-1-yl, thiophen-3-yl, [1,3]benzodioxol-5-yl, (2-methyl)-imidazol-1-yl, (1-methyl)-piperidin-4-yloxy, 2-(morpholin-4-yl)-ethoxy, (4-bromo)-pyrazol-1-yl, *N*-pyrrolidinyl, (3, 5-dimethyl)-pyrazol-1-yl, morpholin-4-yl, hydroxy, -(OCH₂CH₂)₂OH, phenyl (optionally substituted with a substituent independently selected from the group consisting of -SO₂Me, -C(=O)NH₂, -OCF₃, -CF₃, cyano, fluoro, and methoxy), amino, cyclopropylamino, allylamino, methylamino, hydroxy, chloro, and -SMe; and wherein R¹ is optionally taken together with R² to form a 1,4-dioxanyl or a, oxazinyl ring.
- R² is: independently selected from the group consisting of hydrogen, C₁₋₄alkyl, C₁₋₄alkoxy, hydroxy, amino, alkylamino, and halogen; wherein R² is optionally taken together with R¹ to form a 1,4-dioxanyl or an oxazinyl ring.
- R³ is: independently selected from the group consisting of hydrogen, C₁₋₄alkyl, and phenyl; wherein C₁₋₄alkyl is optionally substituted with a substituent independently selected from the group consisting of -C(=O)C₁₋₄alkyl, -C(=O)NH₂, carboxy, morpholinyl, cyclopropyl, hydroxy, and one to three fluorine atoms;
- Y is: independently selected from the group consisting of carboxy, 1 H-tetrazol-5-yl, and -C(=O)C₁₋₄alkoxy; wherein said alkoxy is optionally substituted with a substituent independently selected from the group consisting of hydroxy, -NMe₂, morpholin-1-yl, chloro, and -OCH₂CH₂OCH₃;
- R¹⁰⁰ and R²⁰⁰ are: independently selected from the group consisting of hydrogen, C₁₋₄alkyl, aryl, cycloalkyl, and a five to seven membered heterocyclyl, said heterocyclyl being formed from R¹⁰⁰ and R²⁰⁰ taken together with the nitrogen atom to which they are both attached;
wherein said aryl and heterocyclyl are optionally substituted with up to four substituents independently selected from the group consisting of C₁₋₃alkyl, halogen, trifluoromethyl, trifluoromethoxy, and hydroxy; and an optical isomer, enantiomer, diastereomer, racemate, or pharmaceutically acceptable salt thereof.

Another aspect of the present invention is directed to a compound of Formula (Ia): 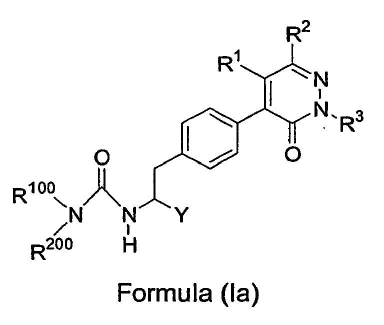 wherein:
- R¹ is: methoxy;
- R² is: independently selected from the group consisting of hydrogen, C₁₋₄alkoxy, amino, or alkylamino; wherein R² is optionally taken together with R¹ to form a 1,4-dioxanyl or a oxazinyl ring;
- R³ is: independently selected from the group consisting of hydrogen, methyl, ethyl, and phenyl; wherein methyl and ethyl are optionally substituted with a substituent independently selected from the group consisting of -C(=O)C₁₋₄alkyl, -C(=O)NH₂, carboxy, morpholinyl, cyclopropyl, hydroxy, and one to three fluorine atoms;
- Y is: independently selected from the group consisting of carboxy, 1*H* tetrazol-5-yl, or -C(=O)ethoxy; wherein ethoxy is optionally substituted with a substituent independently selected from the group consisting of hydroxy, chlorine, -NMe₂, and -OCH₂CH₂OCH₃;
- R¹⁰⁰ and R²⁰⁰ are: independently selected from the group consisting of hydrogen, methyl, ethyl, *i*-but-1-yl, cyclohexyl, 2-methyl-piperadin-1-yl, and phenyl; wherein said phenyl is substituted with one to two substitutents independently selected from the group consisting of methyl, chloro, hydroxy, trifluoromethyl, and trifluoromethoxy;
and an optical isomer, enantiomer, diastereomer, racemate, or pharmaceutically acceptable salt thereof.

Another embodiment of the present invention is further directed to compounds of Formula (Ib) wherein the substituents are as previously defined (including the previously listed preferred substitutions for R¹, R², R³, W, Y, and Z in any combination). Examples of embodiments of the present invention are shown in Table I: 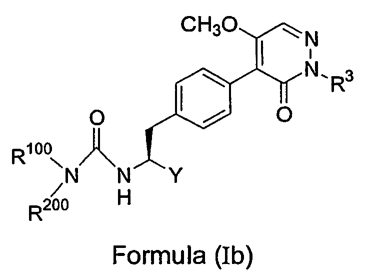 wherein Y, R³, R¹⁰⁰, and R²⁰⁰ are dependently selected from the group consisting of:

**Table I**

| (d denotes a diastereomeric mixture; * denotes a prodrug) | | | | | |
|---|---|---|---|---|---|
| **Cpd** | **R³** | Y | _{R}100 | **R²⁰⁰** | **Stereochem. of R¹⁰⁰** |
| **1** | CH₃ | CO₂H | *i*-propyl | *i*-propyl | |
| **2** | (2-OH)-eth-1-yl | CO₂H | (2,6-Cl₂)phenyl | H | |
| **3** | (2-OH)-eth-1-yl | CO₂H | cyclohexyl | H | |
| **4** | (2-OH)-eth-1-yl | CO₂H | (2-F)phenyl | H | |
| **5** | CH₃ | CO₂H | (4-Me)piperazin-1-yl | | |
| **6** | CH₃ | CO₂H | (3-OH)pyrrolidin-1-yl | | d |
| **7** | CH₃ | CO₂H | (2-OH)-eth-1-yl | H | |
| **8** | CH₃ | CO₂H | (2-hydroxy methyl)-pyrrolidin-1-yl | H | S |
| **9** | CH₃ | CO₂H | benzyl | H | |
| **10** | CH₃ | CO₂H | phenyl | methyl | |
| **11** | CH₃ | CO₂H | cyclohexyl | methyl | |
| **12** | CH₃ | CO₂H | benzyl | methyl | |
| **13** | CH₃ | CO₂H | methyl | *i*-butyl | |
| **14** | CH₃ | CO₂H | (2-OH)-eth-1-yl | methyl | |
| **15** | CH₃ | CO₂H | benzyl | (2-OH)-eth-1-yl | |
| **16** | CH₃ | CO₂H | phenyl | (2-OH)-eth-1-yl | |
| **17** | CH₃ | CO₂H | cyclohexyl | (2-OH)-eth-1-yl | |
| **18** | CH₃ | CO₂H | *i*-butyl | benzyl | |
| **19** | CH₃ | CO₂H | methyl | methyl | |
| **20** | CH₃ | CO₂H | cyclohexyl | H | |
| **21** | CH₃ | 2-OH-ethoxy carbonyl | *i*-butyl | methyl | |
| **22** | CH₃ | CO₂H | 1-methyl-piperidin-4-yl | cyclopropyl | |
| **23** | CH₃ | CO₂H | norbornan-2-yl | H | S |
| **24** | CH₃ | CO₂H | 4-(3-chloro-5-trifluoromethyl-pyridin-2-yl)-piperazin-1-yl | | |
| **25** | CH₃ | CO₂H | 4-(adamantan-2-yl)-piperazin-1-yl | | |
| **26** | CH₃ | CO₂H | cyclohexyl | hydroxy | |
| **27** | CH₃ | CO₂H | benzyl | *i*-propyl | |
| **28** | CH₃ | CO₂H | 2-methyl-piperidin-1-yl | | d |
| **29** | CH₃ | CO₂H | (1-phenyl)-pyrazol-4-ylmethyl | methyl | |
| **30** | CH₃ | CO₂H | 4-(Benzooxazol-2-yl)-[1,4]diazepan-1-yl | | |
| **31** | CH₃ | CO₂H | furan-2-ylmethyl | but-2-yn-1-yl | |
| **32** | CH₃ | CO₂H | 10-methoxy-3,4,5,6-tetrahydro-2*H*-benzo[b][1,5]oxacinyl | | |
| **33** | CH₃ | CO₂H | cyclohexyl | *i*-propyl | |
| **34** | CH₃ | CO₂H | 4-(2-hydroxy-eth-1 -yl)-piperazin-1-ylamino | H | |
| **35** | CH₃ | CO₂H | 1-benzyl-pyrrolidin-3-yl | methyl | |
| **36** | CH₃ | CO₂H | (4-Hydroxy-1,1-dioxo-tetrahydro-1λ⁶-thiopheny-3-yl | H | 4S,3S |
| **37** | CH₃ | CO₂H | 2-(dimethylami no)-eth-1-yl | ethyl | |
| **38** | CH₃ | CO₂H | 2-(phenylamino-methyl)-pyrrolidin-1-yl | | R |
| **39** | CH₃ | CO₂H | 2-(pyrrolidin-1-ylmethyl)-pyrrolidin-1-yl | | S |
| **40** | CH₃ | CO₂H | 1-Aza-bicyclo [2.2.2]oct-3-yl | pyridin-3-ylmethyl | d |
| **41** | CH₃ | CO₂H | phenyl | *i*-butyl | |
| **42** | CH₃ | CO₂H | phenyl | *i*-propyl | |
| **43** | CH₃ | CO₂H | *4-(i-*propylamino )-phenyl | *i*-propyl | |
| **44** | CH₃ | CO₂H | (2-methyl-5-hydroxy)-phenyl | ethyl | |
| **45** | CH₃ | CO₂H | naphth-1-yl | ethyl | |
| **46** | CH₃ | CO₂H | phenyl | phenylamino | |
| **47** | CH₃ | CO₂H | phenyl | phenyl | |
| **48** | CH₃ | CO₂H | benzyl | phenyl | |
| **49** | CH₃ | CO₂H | (2-methyl)-indolin-1-yl | | d |
| **50** | CH₃ | CO₂H | phenyl | n-propyl | |
| **51** | CH₃ | CO₂H | (2-trifluorometh oxy)-phenyl | methyl | |
| **52** | CH₃ | CO₂H | (2-trifluorometh yl)-phenyl | methyl | |
| **53** | CH₃ | CO₂H | 6-fluoro-2-methyl-3,4-dihydro-2H-quinolin-1-yl | | d |
| **54** | CH₃ | CO₂H | pyridin-2-yl | methyl | |
| ***55** | CH₃ | -C(=O) O(CH₂)₂OH | s-but-1-yl | methyl | |

A further embodiment of the present invention includes representative compounds shown in Table II:

**Table II**

| **Cpd** | |
|---|---|
| **1** | 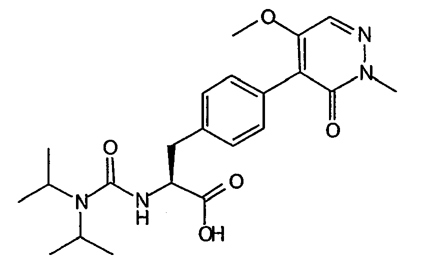 |
| **2** | 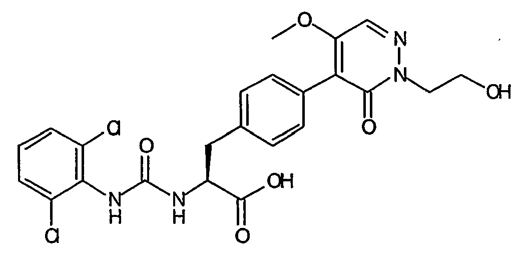 |
| **10** | 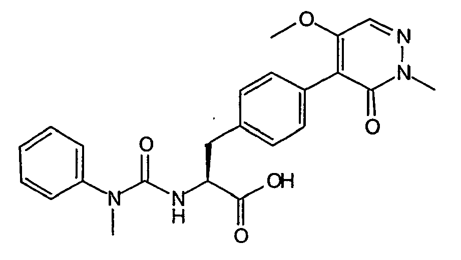 |
| **11** | 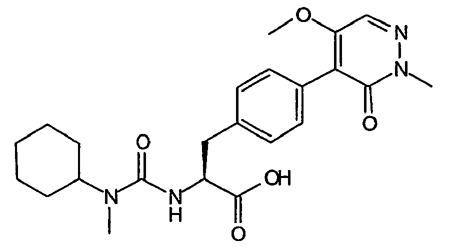 |
| **13** | 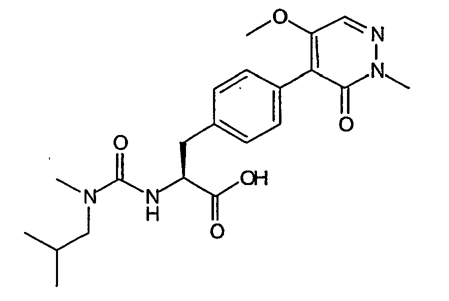 |
| **14** | 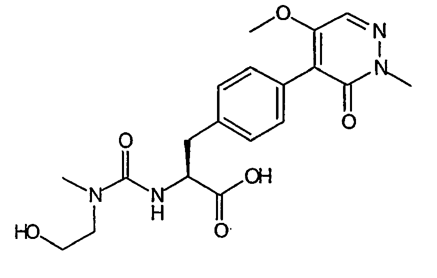 |
| **16** | 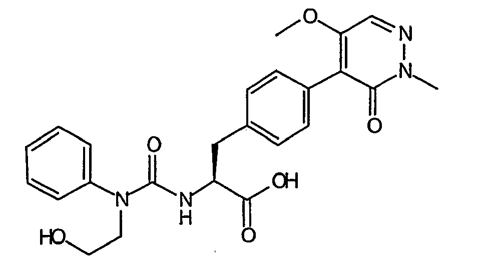 |
| **21** | 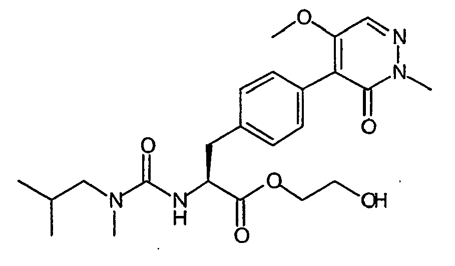 |
| **22** | 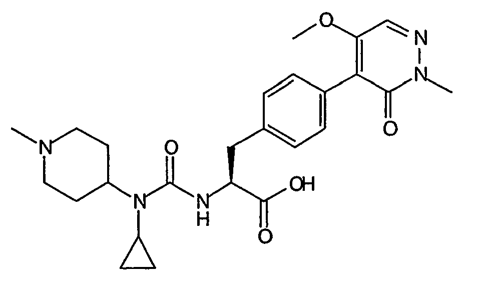 |
| **23** | 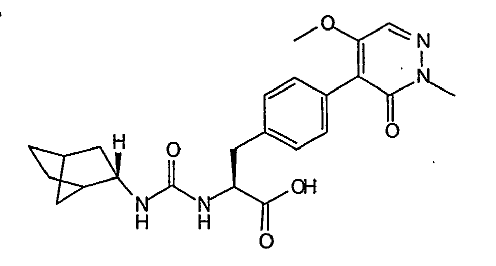 |
| **28** | 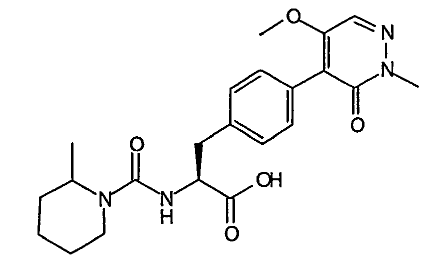 |
| **30** | 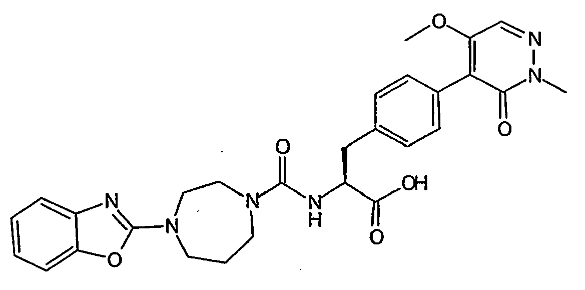 |
| **41** | 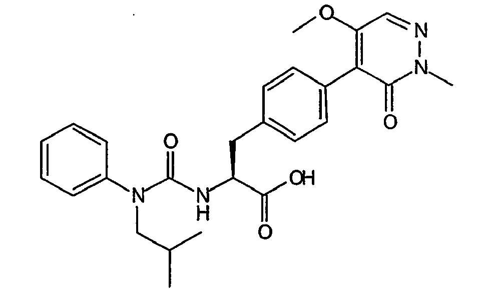 |
| **42** | 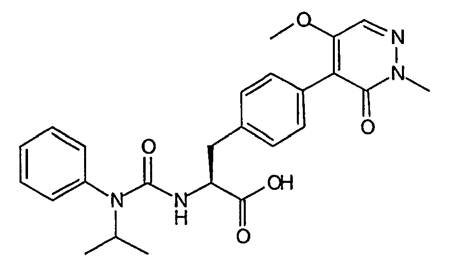 |
| **44** | 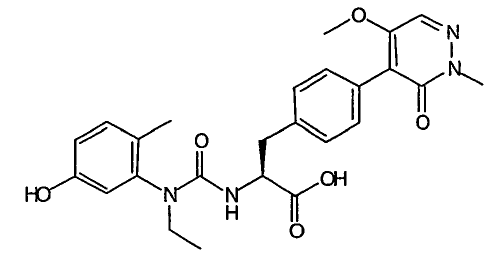 |
| **48** | 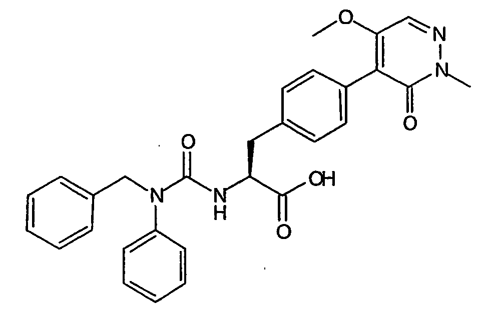 |
| **51** | 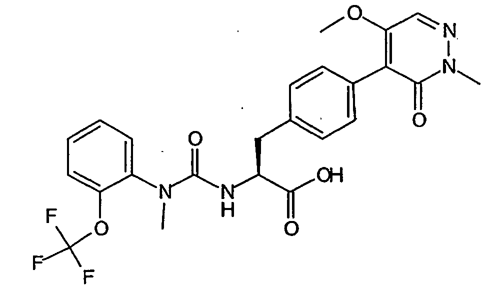 |
| **52** | 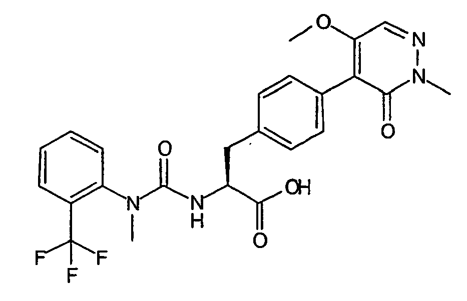 |
| **53** | 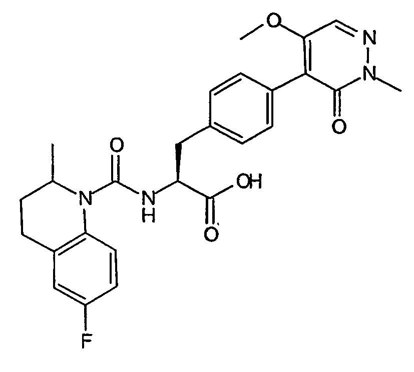 |

The compounds of the present invention may also be present in the form of pharmaceutically acceptable salts. For use in medicine, the salts of the compounds of this invention refer to non-toxic "pharmaceutically acceptable salts" *(*Ref. International J. Pharm., 1986, 33, 201-217; J. Pharm. Sci., 1997 (Jan), 66, 1, 1). Other salts may, however, be useful in the preparation of compounds according to this invention or of their pharmaceutically acceptable salts. Representative organic or inorganic acids include, but are not limited to, hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toluenesulfonic, cyclohexanesulfamic, salicylic, saccharinic or trifluoroacetic acid. Representative organic or inorganic bases include, but are not limited to, basic or cationic salts such as benzathine, chloroprocaine, choline, diethanolamine, ethylenediamine, meglumine, procaine, aluminum, calcium, lithium, magnesium, potassium, sodium and zinc.

The present invention includes within its scope prodrugs of the compounds of this invention. In general, such prodrugs will be functional derivatives of the compounds which are readily convertible *in vivo* into the required compound. Thus, in the methods of treatment of the present invention, the term "administering" shall encompass the treatment of the various disorders described with the compound specifically disclosed or with a compound which may not be specifically disclosed, but which converts to the specified compound *in vivo* after administration to the subject. Conventional procedures for the selection and preparation of suitable prodrug derivatives are described, for example, in "Design of Prodrugs", ed. H. Bundgaard, Elsevier, 1985.

Where the compounds according to this invention have at least one chiral center, they may accordingly exist as enantiomers. Where the compounds possess two or more chiral centers, they may additionally exist as diastereomers. Where the processes for the preparation of the compounds according to the invention give rise to mixtures of stereoisomers, these isomers may be separated by conventional techniques such as preparative chromatography. The compounds may be prepared in racemic form or as individual enantiomers or diasteromers by either stereospecific synthesis or by resolution. The compounds may, for example, be resolved into their component enantiomers or diastereomers by standard techniques, such as the formation of stereoisomeric pairs by salt formation with an optically active base, followed by fractional crystallization and regeneration of the free acid. The compounds may also be resolved by formation of stereoisomeric esters or amides, followed by chromatographic separation and removal of the chiral auxiliary. Alternatively, the compounds may be resolved using a chiral HPLC column. It is to be understood that all stereoisomers, racemic mixtures, diastereomers and enantiomers thereof are encompassed within the scope of the present invention.

During any of the processes for preparation of the compounds of the present invention, it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known in the art.

Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

As used herein, unless otherwise noted, "alkyl" and "alkoxy" whether used alone or as part of a substituent group refers to straight and branched carbon chains having 1 to 8 carbon atoms or any number within this range. Similarly, alkenyl and alkynyl groups include straight and branched chain alkenes and alkynes having 2 to 8 carbon atoms or any number within this range, wherein an alkenyl chain has at least one double bond in the chain and an alkynyl chain has at least one triple bond in the chain. Alkoxy radicals are oxygen ethers formed from the previously described straight or branched chain alkyl groups.

As used herein, unless otherwise noted "oxo" whether used alone or as part of a substituent group refers to an O= to either a carbon or a sulfur atom. For example, phthalimide and saccharin are examples of compounds with oxo substituents.

The term "cycloalkyl," as used herein, refers to an optionally substituted, stable, saturated or partially saturated monocyclic or bicyclic ring system containing from 3 to 8 ring carbons and preferably 5 to 7 ring carbons. Examples of such cyclic alkyl rings include cyclopropyl, cyclobutyl, cyclopentyl, cyclohexyl or cycloheptyl.

The term "benzo fused cycloalkyl" shall mean an optionally substituted stable ring system wherein one of the rings is phenyl and the other is a cycloalkyl as previously defined. Examples of such benzo fused cycloalkyl includes, but is not limited to, indane, dihydronaphthalene, and 1,2,3,4-tetrahydronaphthalene

The term "polycycloalkyl" as used herein refers to an optionally substituted stable, saturated or partially saturated polycyclic ring system containing three or more rings with from 8 to 12 carbons. Examples of such polycyclic alkyl rings include, but are not limited to, tetracyclo [5.2.2.0.0] undecanyl and adamantyl.

The term "heterocyclyl" as used herein refers to an optionally substituted, stable, saturated or partially saturated 5 or 6 membered monocyclic or bicyclic ring system which consists of carbon atoms and from one to three heteroatoms selected from N, O, or S. Examples of heterocyclyl groups include, but are not limited to, pyrrolinyl (including 2*H*-pyrrole, 2-pyrrolinyl or 3-pyrrolinyl), pyrrolidinyl, dioxolanyl, 2-imidazolinyl, imidazolidinyl, 2-pyrazolinyl, pyrazolidinyl, piperidinyl, dioxanyl, morpholinyl, dithianyl, thiomorpholinyl or piperazinyl. The heterocyclyl group may be attached at any heteroatom or carbon atom, which results in the creation of a stable structure.

The term "benzo fused heterocycle" or the radical "benzo fused heterocyclyl" as used herein refers to a optionally substituted, stable ring structure wherein one of the rings is phenyl and the other is stable, saturated or partially saturated 5 or 6 membered monocyclic or 8 to 10 membered bicyclic ring system which consists of carbon atoms and from one to three heteroatoms selected from N, O, or S. Examples of benzo fused heterocyclyl groups include, but are not limited to,indoline, isoindoline, and 1,2,3,4-tetrahydroquinoline.

The term "aryl", as used herein, refers to optionally substituted aromatic groups comprising a stable six membered monocyclic or ten membered bicyclic aromatic ring system which consists of carbon atoms. Examples of aryl groups include, but are not limited to, phenyl or naphthalenyl.

The term "heteroaryl" as used herein represents a stable five or six membered monocyclic aromatic ring system or a nine or ten membered benzo-fused heteroaromatic ring system which consists of carbon atoms and from one to three heteroatoms selected from N, O or S. The heteroaryl group may be attached at any heteroatom or carbon atom which results in the creation of a stable structure.

The term "heteroaryl fused heterocyclyl" as used herein represents a optionally substituted stable bicyclic ring structure in which one ring is an aromatic five or six membered ring which consists of carbon atoms and from one to three heteroatoms selected from N, O or S and the second ring is a stable, saturated or partially saturated 5 or 6 membered ring which consists of carbon atoms and from one to three heteroatoms selected from N, O or S.

The term "heteroaryl fused cycloalkyl" as used herein represents an optionally substituted stable bicyclic ring structure in which one ring is an aromatic five or six membered ring which consists of carbon atoms and from one to three heteroatoms selected from N, O or S and the other ring is a saturated or partially saturated ring containing from 3 to 8 ring carbons and preferably 5 to 7 ring carbons.

The term "arylalkyl" means an alkyl group substituted with an aryl group (e.g., benzyl, phenethyl). The term "arylalkoxy" indicates an alkoxy group substituted with an aryl group (e.g., benzyloxy, phenethoxy, etc.). Similarly, the term "aryloxy" indicates an oxy group substituted with an aryl group (e.g., phenoxy).

Whenever the term "alkyl" or "aryl" or either of their prefix roots appear in a name of a substituent (*e.g.*, aralkyl, alkylamino) it shall be interpreted as including those limitations given above for "alkyl" and "aryl." Designated numbers of carbon atoms (e.g., C₁₋₆) shall refer independently to the number of carbon atoms in an alkyl or cycloalkyl moiety or to the alkyl portion of a larger substituent in which alkyl appears as its prefix root.

The term "cycloalkyloxy" and "polycycloalkyloxy" whether used alone or as part of a substituent group, denotes an oxygen ether radical of the above described cycloalkyl or polycyloalkyl groups.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques known in the art as well as those methods set forth herein.

The pyridazinone urea compounds of the present invention are useful α4 integrin receptor antagonists and, more particularly α4β7 integrin receptor antagonists for treating a variety of integrin mediated disorders that are ameliorated by inhibition of the α4β7 integrin receptor including, but not limited to, inflammatory, autoimmune and cell-proliferative disorders.

Illustrative of the invention is a pharmaceutical composition comprising a pharmaceutically acceptable carrier and any of the compounds described above. Also illustrative of the invention is a pharmaceutical composition made by mixing any of the compounds described above and a pharmaceutically acceptable carrier. A further illustration of the invention is a process for making a pharmaceutical composition comprising mixing any of the compounds described above and a pharmaceutically acceptable carrier. The present invention also provides pharmaceutical compositions comprising one or more compounds of this invention in association with a pharmaceutical acceptable carrier.

An example of the invention is a method for the treatment of integrin mediated disorders in a subject in need thereof comprising administering to the subject a therapeutically effective amount of any of the compounds or pharmaceutical compositions described above. Also included in the invention is the use of a compound of Formula (I) for the preparation of a medicament for treating an integrin mediated disorder in a subject in need thereof.

Further exemplifying the invention is the method for the treatment of integrin mediated disorders, wherein the therapeutically effective amount of the compound is from about 0.01 mg/kg/day to about 120 mg/kg/day.

In accordance with the methods of the present invention, the individual components of the pharmaceutical compositions described herein can be administered separately at different times during the course of therapy or concurrently in divided or single combination forms. The instant invention is therefore to be understood as embracing all such regimes of simultaneous or alternating treatment and the term "administering" is to be interpreted accordingly.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human, that is being sought by a researcher, veterinarian, medical doctor, or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.

The utility of the compounds to treat integrin mediated disorders can be determined according to the procedures herein. The present invention therefore provides a method for the treatment of integrin mediated disorders in a subject in need thereof which comprises administering any of the compounds as defined herein in a quantity effective to inhibit the α4β7 integrin receptor including, but not limited to, inflammatory, autoimmune and cell-proliferative disorders.

The ability of the compounds of Formula I to antagonize the actions of *VLA-4* and/or α4β7 integrin makes them useful for preventing or reversing the symptoms, disorders or diseases induced by the binding of *VLA-4* and or *α*4*β*7 to their various respective ligands. Thus, these antagonists will inhibit cell adhesion processes including cell activation, migration, proliferation and differentiation. Accordingly, another aspect of the present invention provides a method for the treatment (including prevention, alleviation, amelioration or suppression) of diseases or disorders or symptoms mediated by *VLA-4* and/or *α*4β7 binding and cell adhesion and activation, which comprises administering to a mammal an effective amount of a compound of Formula I. Such diseases, disorders, conditions or symptoms are for example (1) multiple sclerosis, (2) asthma, (3) allergic rhinitis, (4) allergic conjunctivitis, (5) inflammatory lung diseases, (6) rheumatoid arthritis, (7) septic arthritis, (8) type I diabetes, (9) organ transplantation rejection, (10) restenosis, (11) autologous bone marrow transplantation, (12) inflammatory sequelae of viral infections, (13) myocarditis, (14) inflammatory bowel disease including ulcerative colitis and Crohn's disease, (15) certain types of toxic and immune-based nephritis, (16) contact dermal hypersensitivity, (17) psoriasis, (18) tumor metastasis, (19) atherosclerosis, and (20) hepatitis.

The utilities of the present compounds in these diseases or disorders may be demonstrated in animal disease models that have been reported in the literature. The following are examples of such animal disease models:
i) experimental allergic encephalomyelitis, a model of neuronal demyelination resembling multiple sclerosis (for example, see T. Yednock et al., "Prevention of experimental autoimmune encephalomyelitis by antibodies against α4β1 integrin." Nature, 356, 63 (1993) and E. Keszthelyi et al., "Evidence for a prolonged role of α4 integrin throughout active experimental allergic encephalomyelitis." Neurology, 47, 1053 (1996));
ii) bronchial hyperresponsiveness in sheep and guinea pigs as models for the various phases of asthma (for example, see W. M. Abraham et al., "α4 -Integrins mediate antigen-induced late bronchial responses and prolonged airway hyperresponsiveness in sheep." J. Clin. Invest. 93, 776 (1993) and A. A. Y. Milne and P. P. Piper, "Role of VLA-4 integrin in leucocyte recruitment and bronchial hyperresponsiveness in the guinea-pig." Eur. J. Pharmacol., 282, 243 (1995));
iii) adjuvant-induced arthritis in rats as a model of inflammatory arthritis (see C. Barbadillo et al., "Anti-VLA-4 mAb prevents adjuvant arthritis in Lewis rats." Arthr. Rheuma. (Suppl.), 36 95 (1993) and D. Seiffge, "Protective effects of monoclonal antibody to VLA-4 on leukocyte adhesion and course of disease in adjuvant arthritis in rats." J. Rheumatol., 23, 12 (1996));
iv) adoptive autoimmune diabetes in the NOD mouse (see J. L. Baron et al., "The pathogenesis of adoptive murine autoimmune diabetes requires an interaction between α4 -integrins and vascular cell adhesion molecule-1.", J. Clin. Invest., 93, 1700 (1994), A. Jakubowski et al., "Vascular cell adhesion molecule-lg fusion protein selectively targets activated α4-integrin receptors in vivo: Inhibition of autoimmune diabetes in an adoptive transfer model in nonobese diabetic mice." J. Immunol., 155, 938 (1995), and X. D. Yang et al., "Involvement of β7 integrin and mucosal addressin cell adhesion molecule-1 (MadCAM-1) in the development of diabetes in nonobese diabetic mice", Diabetes, 46, 1542 (1997));
v) cardiac allograft survival in mice as a model of organ transplantation (see M. Isobe et al., "Effect of anti-VCAM-1 and anti-VLA-4 monoclonal antibodies on cardiac allograft survival and response to soluble antigens in mice.", Tranplant. Proc., 26, 867 (1994) and S. Molossi et al., "Blockade of very late antigen-4 integrin binding to fibronectin with connecting segment-1 peptide reduces accelerated coronary arteripathy in rabbit cardiac allografts." J. Clin Invest., 95, 2601 (1995));
vi) spontaneous chronic colitis in cotton-top tamarins which resembles human ulcerative colitis, a form of inflammatory bowel disease (see D. K. Podolsky et al., "Attenuation of colitis in the Cotton-top tamarin by anti-α4 integrin monoclonal antibody.", J. Clin. Invest., 92, 372 (1993));
vii) contact hypersensitivity models as a model for skin allergic reactions (see T. A. Ferguson and T. S. Kupper, "Antigen-independent processes in antigen-specific immunity.", J. Immunol., 150, 1172 (1993) and P. L. Chisholm et al., "Monoclonal antibodies to the integrin α-4 subunit inhibit the murine contact hypersensitivity response." Eur. J. Immunol., 23, 682 (1993));
viii) acute nephrotoxic nephritis (see M. S. Mulligan et al., "Requirements for leukocyte adhesion molecules in nephrotoxic nephritis.", J. Clin. Invest., 91, 577 (1993));
ix) tumor metastasis (for examples, see M. Edward, "Integrins and other adhesion molecules involved in melanocytic tumor progression.", Curr. Opin. Oncol., 7, 185 (1995));
x) experimental autoimmune thyroiditis (see R. W. McMurray et al., "The role of α4 integrin and intercellular adhesion molecule-1 (ICAM-1) in murine experimental autoimmune thyroiditis." Autoimmunity, 23, 9 (1996);
xi) ischemic tissue damage following arterial occlusion in rats (see F. Squadrito et al., "Leukocyte integrin very late antigen-4/vascular cell adhesion molecule-1 adhesion pathway in splanchnic artery occlusion shock." Eur. J. Pharmacol., 318, 153 (1996; and
xii) inhibition of TH2 T-cell cytokine production including IL-4 and IL-5 by *VLA-4* antibodies which would attenuate allergic responses (J. Clinical Investigation 100, 3083 (1997).
xiii) Shigematsu, T., Specian, R. D., Wolf, R. E., Grisham, M. B., and Granger, D. N. MADCAM mediates lymphocyte - endothelial cell adhesion in a murine model of chronic colitis. Am. J. Physiol. Gastrointest.. Liver Physiol., 281: G1309-13015, 2001.
xiv) Picarella, D., Hurlbut, P., Torrman, J., Shi, X., Butcher, E., and Ringler, D. J. Monoclonal antibodies specific for β7 integrin and mucosal addressin cell adhesion molecule-1 (MAdCAM-1) reduce inflammation in the colon of scid mice reconstituted with CD45RBhigh CD4+ T cells. J. Immuol., 158: 2099-2106. 1997.
xv) Hesterberg, P. E., Winsor-Hines, D., Briskin, M. J., et al., Rapid resolution of chronic colitis in the cotton-top tamarin with an antibody to a gut-homing integrin α4β7. Gastroenterology, 111:1373-1380, 1996.
xvi) Gordon, F. H., Lai, C. W. Y., Hamilton, M. I., Allison, M. C., Srivastava, E. D., Foutweather, M. G., Donoghue, S., Greenlee, C., Subhani, J., Amlot, P. L., and Pounder, R. E. A randomized placebo-controlled trial of a humanized monoclonal antibody to α4 integrin in active Crohn's disease. Gastroenterology, 121:268-274, 2001.
xvii) Ghosh, S., Goldin, e., Gordon, F. H., Malchow, H. A., Rask-madsen, J., Rutgeerts, P., Vyhnalek, P., Zadorova, Z, Palmer, T, and Donoghue, S. Natalizumab for active Crohn's disease. New Engl. J. Med., 348: 24-32, 2003.

Compounds of Formula I may be used in combination with other drugs that are used in the treatment/prevention/suppression or amelioration of the diseases or conditions for which compounds of Formula I are useful. Such other drugs may be administered, by a route and in an amount commonly used therefor, contemporaneously or sequentially with a compound of Formula I. When a compound of Formula I is used contemporaneously with one or more other drugs, a pharmaceutical composition containing such other drugs in addition to the compound of Formula I is preferred. Accordingly, the pharmaceutical compositions of the present invention include those that also contain one or more other active ingredients, in addition to a compound of Formula I. Examples of other active ingredients that may be combined with a compound of Formula I, either administered separately or in the same pharmaceutical compositions, include, but are not limited to: (a) other VLA-4 antagonists such as those described in U.S. Pat. No. 5,510,332, WO97/03094, WO97/02289, WO96/40781, WO96/22966, WO96/20216, WO96/01644, WO96/06108, WO95/15973 and WO96/31206; (b) steroids such as beclomethasone, methylprednisolone, betamethasone, prednisone, dexamethasone, and hydrocortisone; (c) immunosuppressants such as FK-506 type immunosuppressants; (d) antihistamines (H1-histamine antagonists) such as bromopheniramine, chlorpheniramine, dexchlorpheniramine, triprolidine, clemastine, diphenhydramine, diphenylpyraline, tripelennamine, hydroxyzine, methdilazine, promethazine, trimeprazine, azatadine, cyproheptadine, antazoline, pheniramine pyrilamine, astemizole, terfenadine, loratadine, cetirizine, fexofenadine, descarboethoxyloratadine, and the like; (e) non-steroidal anti-asthmatics such as b2-agonists (terbutaline, metaproterenol, fenoterol, isoetharine, albuterol, bitolterol, salmeterol and pirbuterol), theophylline, cromolyn sodium, atropine, ipratropium bromide, leukotriene antagonists (zafirlukast, montelukast, pranlukast, iralukast, pobilukast, SKB-106,203), leukotriene biosynthesis inhibitors (zileuton, BAY-1005); (f) non-steroidal antiinflammatory agents (NSAIDs) such as propionic acid derivatives (alminoprofen, benoxaprofen, bucloxic acid, carprofen, fenbufen, fenoprofen, fluprofen, flurbiprofen, ibuprofen, indoprofen, ketoprofen, miroprofen, naproxen, oxaprozin, pirprofen, pranoprofen, suprofen, tiaprofenic acid, and tioxaprofen), acetic acid derivatives (indomethacin, acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirac), fenamic acid derivatives (flufenamic acid, meclofenamic acid, mefenamic acid, niflumic acid and tolfenamic acid), biphenylcarboxylic acid derivatives (diflunisal and flufenisal), oxicams (isoxicam, piroxicam, sudoxicam and tenoxican), salicylates (acetyl salicylic acid, sulfasalazine) and the pyrazolones (apazone, bezpiperylon, feprazone, mofebutazone, oxyphenbutazone, phenylbutazone); (g) cyclooxygenase-2 (COX-2) inhibitors such as celecoxib, rofecoxib, and parecoxib; (h) inhibitors of phosphodiesterase type IV (PDE-IV); (i) antagonists of the chemokine receptors, especially CCR-1, CCR-2, and CCR-3; (j) cholesterol lowering agents such as HMG-CoA reductase inhibitors (lovastatin, simvastatin, pravastatin, fluvastatin, atorvastatin, and other statins), sequestrants (cholestyramine and colestipol), nicotinic acid, fenofibric acid derivatives (gemfibrozil, clofibrat, fenofibrate and benzafibrate), and probucol; (k) anti-diabetic agents such as insulin, sulfonylureas, biguanides (metformin), a-glucosidase inhibitors (acarbose) and glitazones (troglitazone, pioglitazone, englitazone, MCC-555, BRL49653 and the like); (1) agents that interfere with TNF such as antibodies to TNF (REMICADE®) or soluble TNF receptor (e.g. ENBREL®); (m) anticholinergic agents such as muscarinic antagonists (ipratropium nad tiatropium); (n) agents that slow gut motility such as opiate agonist (i.e. LOPERAMIDE®), serotonin receptor receptor anagonists (ALOSERTON, ODANSETRON, ect.) (o) other compounds such as 5-aminosalicylic acid and prodrugs thereof, antimetabolites such as azathioprine and 6-mercaptopurine, and cytotoxic cancer chemotherapeutic agents.

The weight ratio of the compound of the Formula I to the second active ingredient may be varied and will depend upon the effective dose of each ingredient. Generally, an effective dose of each will be used. Thus, for example, when a compound of the Formula I is combined with an NSAID the weight ratio of the compound of the Formula I to the NSAID will generally range from about 1000:1 to about 1:1000, preferably about 200:1 to about 1:200. Combinations of a compound of the Formula I and other active ingredients will generally also be within the aforementioned range, but in each case, an effective dose of each active ingredient should be used.

Accordingly, a compound of the present invention may be administered by any conventional route of administration including, but not limited to oral, nasal, pulmonary, sublingual, ocular; transdermal, rectal, vaginal and parenteral (i.e. subcutaneous, intramuscular, intradermal, intravenous etc.).

To prepare the pharmaceutical compositions of this invention, one or more compounds of Formula (I) or salt thereof as the active ingredient, is intimately admixed with a pharmaceutical carrier according to conventional pharmaceutical compounding techniques, which carrier may take a wide variety of forms depending of the form of preparation desired for administration (e.g. oral or parenteral). Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain.

Methods of formulating pharmaceutical compositions have been described in numerous publications such as Pharmaceutical Dosage Forms: Tablets. Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

In preparing a pharmaceutical composition of the present invention in liquid dosage form for oral, topical and parenteral administration, any of the usual pharmaceutical media or excipients may be employed. Thus, for liquid dosage forms, such as suspensions (i.e. colloids, emulsions and dispersions) and solutions, suitable carriers and additives include but are not limited to pharmaceutically acceptable wetting agents, dispersants, flocculation agents, thickeners, pH control agents (i.e. buffers), osmotic agents, coloring agents, flavors, fragrances, preservatives (i.e. to control microbial growth, etc.) and a liquid vehicle may be employed. Not all of the components listed above will be required for each liquid dosage form.

In solid oral preparations such as, for example, dry powders for reconstitution or inhalation, granules, capsules, caplets, gelcaps, pills and tablets (each including immediate release, timed release and sustained release formulations), suitable carriers and additives include but are not limited to diluents, granulating agents, lubricants, binders, glidants, disintegrating agents and the like. Because of their ease of administration, tablets and capsules represent the most advantageous oral dosage unit form, in which case solid pharmaceutical carriers are obviously employed. If desired, tablets may be sugar coated, gelatin coated, film coated or enteric coated by standard techniques.

The pharmaceutical compositions herein will contain, per dosage unit, e.g., tablet, capsule, powder, injection, teaspoonful and the like, an amount of the active ingredient necessary to deliver an effective dose as described above. The pharmaceutical compositions herein will contain, per unit dosage unit, e.g., tablet, capsule, powder, injection, suppository, teaspoonful and the like, of from about 0.01 mg/kg to about 300 mg/kg (preferably from about 0.01 mg/kg to about 100 mg/kg; and, more preferably, from about 0.01 mg/kg to about 30 mg/kg) and may be given at a dosage of from about 0.01 mg/kg/day to about 300 mg/kg/day (preferably from about 0.01 mg/kg/day to about 100 mg/kg/day and more preferably from about 0.01 mg/kg/day to about 30 mg/kg/day). Preferably, the method for the treatment of integrin mediated disorders described in the present invention using any of the compounds as defined herein, the dosage form will contain a pharmaceutically acceptable carrier containing between from about 0.01 mg to about 100 mg; and, more preferably, from about 5 mg to about 50 mg of the compound, and may be constituted into any form suitable for the mode of administration selected. The dosages, however, may be varied depending upon the requirement of the subjects, the severity of the condition being treated and the compound being employed. The use of either daily administration or post-periodic dosing may be employed.

Preferably these compositions are in unit dosage forms from such as tablets, pills, capsules, dry powders for reconstitution or inhalation, granules, lozenges, sterile parenteral solutions or suspensions, metered aerosol or liquid sprays, drops, ampoules, autoinjector devices or suppositories for administration by oral, intranasal, sublingual, intraocular, transdermal, parenteral, rectal, vaginal, dry powder inhaler or other inhalation or insufflation means. Alternatively, the composition may be presented in a form suitable for once-weekly or once-monthly administration; for example, an insoluble salt of the active compound, such as the decanoate salt, may be adapted to provide a depot preparation for intramuscular injection.

For preparing solid pharmaceutical compositions such as tablets, the principal active ingredient is mixed with a pharmaceutical carrier, e.g. conventional tableting ingredients such as diluents, binders, adhesives, disintegrants, lubricants, antiadherents and gildants. Suitable diluents include, but are not limited to, starch (i.e. corn, wheat, or potato starch, which may be hydrolized), lactose (granulated, spray dried or anhydrous), sucrose, sucrose-based diluents (confectioner's sugar; sucrose plus about 7 to 10 weight percent invert sugar; sucrose plus about 3 weight percent modified dextrins; sucrose plus invert sugar, about 4 weight percent invert sugar, about 0.1 to 0.2 weight percent cornstarch and magnesium stearate), dextrose, inositol, mannitol, sorbitol, microcrystalline cellulose (i.e. AVICEL^{™} microcrystalline cellulose available from FMC Corp.), dicalcium phosphate, calcium sulfate dihydrate, calcium lactate trihydrate and the like. Suitable binders and adhesives include, but are not limited to acacia gum, guar gum, tragacanth gum, sucrose, gelatin, glucose, starch, and cellulosics (i.e. methylcellulose, sodium carboxymethylcellulose, ethylcellulose, hydroxypropylmethylcellulose, hydroxypropylcellulose, and the like), water soluble or dispersible binders (i.e. alginic acid and salts thereof, magnesium aluminum silicate, hydroxyethylcellulose [i.e. TYLOSE^{™} available from Hoechst Celanese], polyethylene glycol, polysaccharide acids, bentonites, polyvinylpyrrolidone, polymethacrylates and pregelatinized starch) and the like. Suitable disintegrants include, but are not limited to, starches (corn, potato, etc.), sodium starch glycolates, pregelatinized starches, clays (magnesium aluminum silicate), celluloses (such as crosslinked sodium carboxymethylcellulose and microcrystalline cellulose), alginates, pregelatinized starches (i.e. com starch, etc.), gums (i.e. agar, guar, locust bean, karaya, pectin, and tragacanth gum), cross-linked polyvinylpyrrolidone and the like. Suitable lubricants and antiadherents include, but are not limited to, stearates (magnesium, calcium and sodium), stearic acid, talc waxes, stearowet, boric acid, sodium chloride, DL-leucine, carbowax 4000, carbowax 6000, sodium oleate, sodium benzoate, sodium acetate, sodium lauryl sulfate, magnesium lauryl sulfate and the like. Suitable gildants include, but are not limited to, talc, cornstarch, silica (i.e. CAB-O-SIL^{™} silica available from Cabot, SYLOID^{™} silica available from W.R. Grace/Davison, and AEROSIL^{™} silica available from Degussa) and the like. Sweeteners and flavorants may be added to chewable solid dosage forms to improve the palatability of the oral dosage form. Additionally, colorants and coatings may be added or applied to the solid dosage form for ease of identification of the drug or for aesthetic purposes. These carriers are formulated with the pharmaceutical active to provide an accurate, appropriate dose of the pharmaceutical active with a therapeutic release profile.

Generally these carriers are mixed with the pharmaceutical active to form a solid preformulation composition containing a homogeneous mixture of the pharmaceutical active of the present invention, or a pharmaceutically acceptable salt thereof. Generally the preformulation will be formed by one of three common methods: (a) wet granulation, (b) dry granulation and (c) dry blending. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from about 0.1 mg to about 500 mg of the active ingredient of the present invention. The tablets or pills containing the novel compositions may also be formulated in multilayer tablets or pills to provide a sustained or provide dual-release products. For example, a dual release tablet or pill can comprise an inner dosage and an outer dosage component, the latter being in the form of an envelope over the former. The two components can be separated by an enteric layer, which serves to resist disintegration in the stomach and permits the inner component to pass intact into the duodenum or to be delayed in release. A variety of materials can be used for such enteric layers or coatings, such materials including a number of polymeric materials such as shellac, cellulose acetate (i.e. cellulose acetate phthalate, cellulose acetate trimetllitate), polyvinyl acetate phthalate, hydroxypropyl methylcellulose phthalate, hydroxypropyl methylcellulose acetate succinate, methacrylate and ethylacrylate copolymers, methacrylate and methyl methacrylate copolymers and the like. Sustained release tablets may also be made by film coating or wet granulation using slightly soluble or insoluble substances in solution (which for a wet granulation acts as the binding agents) or low melting solids a molten form (which in a wet granulation may incorporate the active ingredient). These materials include natural and synthetic polymers waxes, hydrogenated oils, fatty acids and alcohols (i.e. beeswax, carnauba wax, cetyl alcohol, cetylstearyl alcohol, and the like), esters of fatty acids metallic soaps, and other acceptable materials that can be used to granulate, coat, entrap or otherwise limit the solubility of an active ingredient to achieve a prolonged or sustained release product.

The liquid forms in which the novel compositions of the present invention may be incorporated for administration orally or by injection include, but are not limited to aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable suspending agents for aqueous suspensions, include synthetic and natural gums such as, acacia, agar, alginate (i.e. propylene alginate, sodium alginate and the like), guar, karaya, locust bean, pectin, tragacanth, and xanthan gum, cellulosics such as sodium carboxymethylcellulose, methylcellulose, hydroxymethylcellulose, hydroxyethylcellulose, hydroxypropyl cellulose and hydroxypropyl methylcellulose, and combinations thereof, synthetic polymers such as polyvinyl pyrrolidone, carbomer (i.e. carboxypolymethylene), and polyethylene glycol; clays such as bentonite, hectorite, attapulgite or sepiolite; and other pharmaceutically acceptable suspending agents such as lecithin, gelatin or the like. Suitable surfactants include but are not limited to sodium docusate, sodium lauryl sulfate, polysorbate, octoxynol-9, nonoxynol-10, polysorbate 20, polysorbate 40, polysorbate 60, polysorbate 80, polyoxamer 188, polyoxamer 235 and combinations thereof. Suitable deflocculating or dispersing agent include pharmaceutical grade lecithins. Suitable flocculating agent include but are not limited to simple neutral electrolytes (i.e. sodium chloride, potassium, chloride, and the like), highly charged insoluble polymers and polyelectrolyte species, water soluble divalent or trivalent ions (i.e. calcium salts, alums or sulfates, citrates and phosphates (which can be used jointly in formulations as pH buffers and flocculating agents). Suitable preservatives include but are not limited to parabens (i.e. methyl, ethyl, *n*-propyl and *n*-butyl), sorbic acid, thimerosal, quaternary ammonium salts, benzyl alcohol, benzoic acid, chlorhexidine gluconate, phenylethanol and the like. There are many liquid vehicles that may be used in liquid pharmaceutical dosage forms, however, the liquid vehicle that is used in a particular dosage form must be compatible with the suspending agent(s). For example, nonpolar liquid vehicles such as fatty esters and oils liquid vehicles are best used with suspending agents such as low HLB (Hydrophile-Lipophile Balance) surfactants, stearalkonium hectorite, water insoluble resins, water insoluble film forming polymers and the like. Conversely, polar liquids such as water, alcohols, polyols and glycols are best used with suspending agents such as higher HLB surfactants, clays silicates, gums, water soluble cellulosics, water soluble polymers and the like. For parenteral administration, sterile suspensions and solutions are desired. Liquid forms useful for parenteral administration include sterile solutions, emulsions and suspensions. Isotonic preparations which generally contain suitable preservatives are employed when intravenous administration is desired.

Furthermore, compounds of the present invention can be administered in an intranasal dosage form via topical use of suitable intranasal vehicles or via transdermal skin patches, the composition of which are well known to those of ordinary skill in that art. To be administered in the form of a transdermal delivery system, the administration of a therapeutic dose will, of course, be continuous rather than intermittent throughout the dosage regimen.

Compounds of the present invention can also be administered in the form of liposome delivery systems, such as small unilamellar vesicles, large unilamellar vesicles, multilamellar vesicles and the like. Liposomes can be formed from a variety of phospholipids, such as cholesterol, stearylamine, phosphatidylcholines and the like.

Compounds of the present invention may also be delivered by the use of monoclonal antibodies as individual carriers to which the compound molecules are coupled. The compounds of the present invention may also be coupled with soluble polymers as targetable drug carriers. Such polymers can include, but are not limited to polyvinylpyrrolidone, pyran copolymer, polyhydroxypropylmethacrylamidephenol, polyhydroxy-ethylaspartamidephenol, or polyethyl eneoxidepolylysine substituted with palmitoyl residue. Furthermore, the compounds of the present invention may be coupled to a class of biodegradable polymers useful in achieving controlled release of a drug, for example, to homopolymers and copolymers (which means polymers containing two or more chemically distinguishable repeating units) of lactide (which includes lactic acid d-, l- and meso lactide), glycolide (including glycolic acid), ε-caprolactone, p-dioxanone (1,4-dioxan-2-one), trimethylene carbonate (1,3-dioxan-2-one), alkyl derivatives of trimethylene carbonate, δ-valerolactone, β-butyrolactone, γ-butyrolactone, ε-decalactone, hydroxybutyrate, hydroxyvalerate, 1,4-dioxepan-2-one (including its dimer 1,5,8,12-tetraoxacyclotetradecane-7,14-dione), 1,5-dioxepan-2-one, 6,6-dimethyl-1,4-dioxan-2-one, polyorthoesters, polyacetals, polydihydropyrans, polycyanoacrylates and cross-linked or amphipathic block copolymers of hydrogels and blends thereof.

Compounds of this invention may be administered in any of the foregoing compositions and dosage regimens or by means of those compositions and dosage regimens established in the art whenever treatment of integrin mediated disorders is required for a subject in need thereof.

The daily dose of a pharmaceutical composition of the present invention may be varied over a wide range from about 0.7 mg to about 21,000 mg per adult human per day; preferably, the dose will be in the range of from about 0.7 mg to about 7000 mg per adult human per day; most preferably the dose will be in the range of from about 0.7 mg to about 2100 mg per adult human per day. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250 and 500 milligrams of the active ingredient for the symptomatic adjustment of the dosage to the subject to be treated. An effective amount of the drug is ordinarily supplied at a dosage level of from about 0.01 mg/kg to about 300 mg/kg of body weight per day. Advantageously, a compound of the present invention may be administered in a single daily dose or the total daily dosage may be administered in divided doses of two, three or four times daily.

Optimal dosages to be administered may be readily determined by those skilled in the art, and will vary with the particular compound used, the mode of administration, the strength of the preparation, and the advancement of the disease condition. In addition, factors associated with the particular subject being treated, including subject age, weight, diet and time of administration, will result in the need to adjust the dose to an appropriate therapeutic level.

Representative IUPAC names for the compounds of the present invention were derived using the ACD/LABS SOFTWARE^{™} Index Name Pro Version 4.5 nomenclature software program provided by Advanced Chemistry Development, Inc., Toronto, Ontario, Canada.

Abbreviations used in the instant specification, particularly the Schemes and Examples, are as follows:
- Boc =: *tert*-butoxycarbonyl
- BuLi =: *n-*butyllithium
- *t*-BuOH =: *tert*-butanol
- CDI =: 1,1'-carbonyldiimidazole
- Cpd or Cmpd =: compound
- d =: day/ days
- DCM =: dichloromethane
- EDC =: 1-(3-Dimethylaminopropyl)-3-ethylcarbodiimide hydrochloride
- EtOAc=: ethyl acetate
- EtOH =: ethanol
- h =: hour/hours
- HOBt / HOBT=: hydroxybenzotriazole
- M=: molar
- MeCN =: acetonitrile
- MeOH =: methanol
- min =: minutes
- NMM =: N-methylmorpholine
- NT =: not tested
- rt/RT =: room temperature
- THF =: tetrahydrofuran
- TFA =: trifluoroacetic acid
- TsOH =: para-toluenesulfonic acid

### GENERAL SYNTHETIC METHODS

Representative compounds of the present invention can be synthesized in accordance with the general synthetic methods described below and are illustrated more particularly in the examples that follow. The schemes are for illustration only. The preparation of the various starting materials used in the schemes is well within the skill of persons versed in the art.

The following schemes describe general synthetic methods whereby intermediate and target compounds of the present invention may be prepared. Additional representative compounds and stereoisomers, racemic mixtures, diastereomers and enantiomers thereof can be synthesized using the intermediates prepared in accordance to the general schemes and other materials, compounds and reagents known to those skilled in the art. All such compounds, stereoisomers, racemic mixtures, diastereomers and enantiomers thereof are intended to be encompassed within the scope of the present invention.

Compounds of the present invention may be prepared by the method shown in Scheme A. Phenyl boronic acid **A1** (Samanen, et al. J. Med. Chem. 1988, 31, 510-516) may be coupled with 4-halo-pyridazinone Compound **A2** in the presence of a palladium catalyst to yield Compound **A3.** Compound **A3** may be converted to its methyl ester, Compound **A4,** by reaction with trimethylsilyldiazomethane. The Boc-group may be removed from Compound **A4** under acidic conditions to provide the corresponding amine, which may then be acylated with a reagent such as CDI to give Compound **A5.**

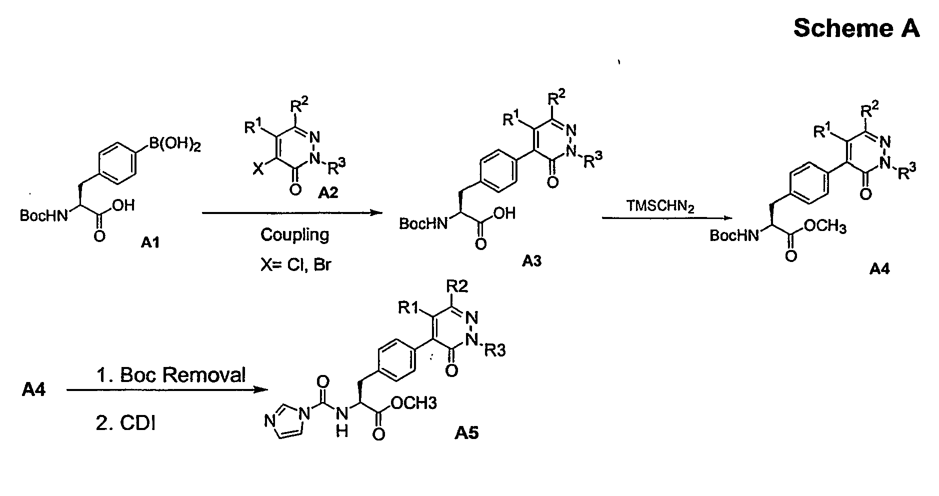

As shown in Scheme B, Compound **A5** may be reacted with amine Compound **B1** to form a urea. Subsequent saponification of the methyl ester provides compounds of Formula **B2.**

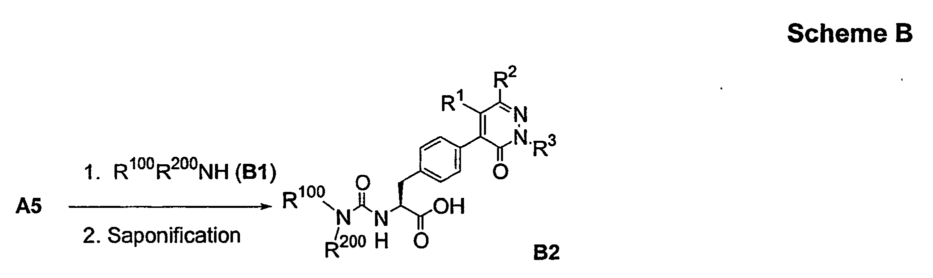

In the case wherein R¹⁰⁰ and/or R²⁰⁰ are optionally substituted aryl groups, the reaction of Compound **A5** with R¹⁰⁰R²⁰⁰NH may be promoted by microwave irradiation. Saponification of the methyl ester intermediate provides compounds of Formula **B2**.

Scheme C illustrates the synthesis of ureas of the present invention by reaction of an amine, Compound **C1**, with an isocyanate Compound **C2** under basic conditions. The subsequent coupling of the intermediate urea to pyridazinone Compound **A2** under microwave irradiation provides Compound **C3**.

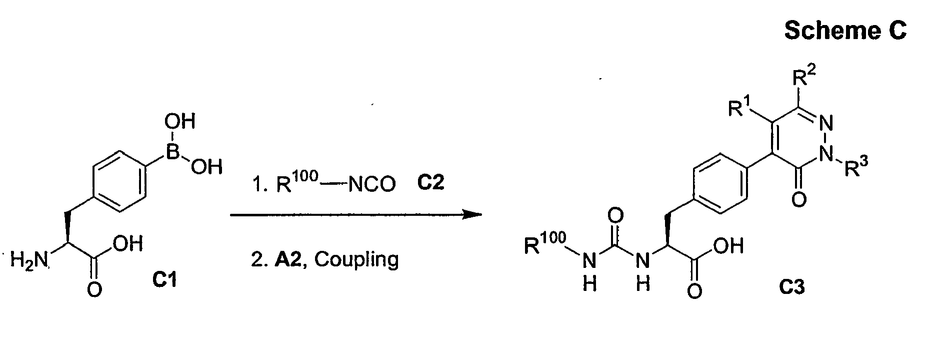

### SPECIFIC SYNTHETIC METHODS

Specific compounds which are representative of this invention were prepared as per the following examples and reaction sequences; the examples and the diagrams depicting the reaction sequences are offered by way of illustration, to aid in the understanding of the invention. The instant compounds may also be used as intermediates in subsequent examples to produce additional compounds of the present invention. No attempt has been made to optimize the yields obtained in any of the reactions. One skilled in the art would know how to increase such yields through routine variations in reaction times, temperatures, solvents and/or reagents.

Reagents were purchased from commercial sources. Nuclear magnetic resonance (NMR) spectra for hydrogen atoms were measured in the indicated solvent with (TMS) as the internal standard on a Bruker Avance 300 (300 MHz) spectrometer. The values are expressed in parts per million down field from TMS. The mass spectra (MS) were determined on a Micromass Platform LC spectrometer using electrospray techniques as either (ESI) *m*/*z* (M+H⁺) or (ESI) *m*/*z* (M-H⁻). Microwave accelerated reactions were performed using either a CEM Discover or a Personal Chemistry Smith Synthesizer microwave instrument. Stereoisomeric compounds may be characterized as racemic mixtures or as separate diastereomers and enantiomers thereof using X-ray crystallography and other methods known to one skilled in the art. Unless otherwise noted, the materials used in the examples were obtained from readily available commercial suppliers or synthesized by standard methods known to one skilled in the art of chemical synthesis. The substituent groups, which vary between examples, are hydrogen unless otherwise noted.

### EXAMPLE 1

### 2-[(Imidazole-1-carbonyl)-amino]-3-[4-(5-methoxy-2-methyl-3-oxo-2,3-dihydro-pyridazin-4-yl)-phenyl]-propionic acid methyl ester

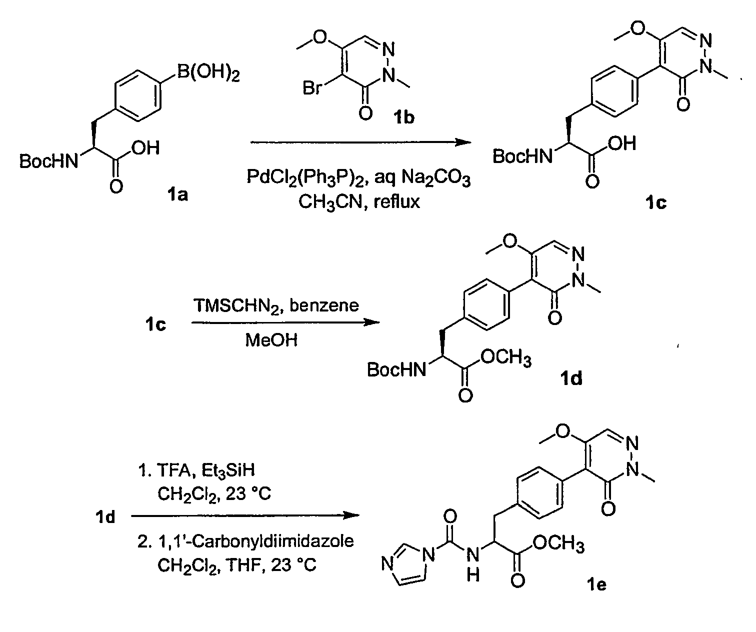

Compound **1a** was prepared from 4-borono-L-phenylalanine by the method of Samanen, et al. J. Med. Chem. 1988, 31, 510-516.

To a mixture of Compound **1a** (12.86 g, 41.6 mmol), Compound **1b** (Cho, S.-D.; Choi, W.-Y.; Yoon, Y.-J. J. Heterocycl. Chem. 1996, 33, 1579-1582) (10.02 g, 45.8 mmol) and *trans*-dichloro(bistriphenylphosphine)palladium (II) (1.46 g, 2.08 mmol) were sequentially added a solution of Na₂CO₃ (aq)(2 M, 84 mL, 168 mmol) and CH₃CN (84 mL). The resulting suspension was heated at reflux under N₂ for 1 h, then was allowed to cool to 23 °C. The mixture was partially concentrated to remove volatile solvent. The resulting mixture was diluted with one-quarter saturated NaHCO₃ (aq) (200 mL) and was washed with Et₂O (200 mL). The organic phase was back-extracted with one-quarter saturated NaHCO₃ (aq) (200 mL). The combined aqueous extracts were cooled to 0 °C and were acidified to pH 2 by addition of 2 N aqueous HCl. The precipitated solid was collected by vacuum filtration, affording crude Compound 1c (15.18g). A sample of purified Compound 1c was obtained by reverse-phase HPLC (YMC Pack ODS-A column, gradient elution from 23 to 43% CH₃CN-water, both containing 0.1% TFA);MS ES+ m/z 426 (M+Na)⁺.

Trimethylsilyldiazomethane (2 M solution in hexanes, 28.0 mL, 56.0 mmol) was added to a solution of crude Compound 1c (15.18 g) in benzene:MeOH (7:2, 135 mL). The resulting mixture was stirred at 23 °C for 17 h. The mixture was concentrated and the residue was purified by column chromatography (gradient elution from 50 to 90% EtOAc-hexanes), yielding Compound **1d** as a white foam (7.83 g); (TOF MS ES+) m/z 440 (M+Na)⁺.

TFA (819 µL, 10.6 mmol) was added to a solution of Compound 1d (443 mg, 1.06 mmol) in CH₂Cl₂. The resulting solution was stirred at 23 °C for 3 h. The solution was concentrated and the residue was purified by flash column chromatography (silica gel, gradient elution from 2 to 10% MeOH- CH₂Cl₂) to yield a white foam (539 mg); (TOF MS ES+) m/z 318 (M+H)⁺.

To a solution of the foam in CH₂Cl₂:THF (5:1, 6 mL) was added 1,1'-carbonyldiimidazole (259 mg, 1.59 mmol). The resulting solution was stirred at 23 °C for 1 h. The mixture was concentrated and the residue was purified by column chromatography (silica gel, gradient elution from 2 to 10% MeOH-CH₂Cl₂). The title Compound **1e** was obtained as a white solid (355 mg). (TOF MS ES+) *mlz* 412 (M+H)⁺.

### EXAMPLE 2

### 2-[(2-Hydroxymethyl-pyrrolidine-1-carbonyl)-amino]-3-[4-(5-methoxy-2-methyl-3-oxo-2,3-dihydro-pyridazin-4-yl)-phenyl]-propionic acid, Cpd 8

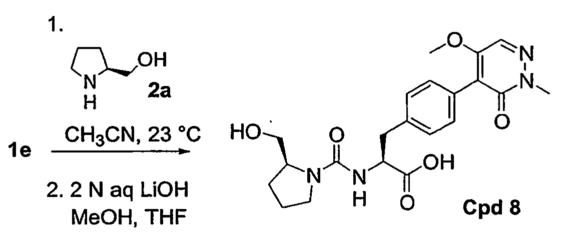

(S)-(+)-2-pyrrolidinemethanol (Compound **2a**)(5.8 µL, 58.3 µmol) was added to a solution of Compound **1e** (20.0 µg, 48.6 µmol) in CH₃CN (200 µL). The resulting solution was stirred at 23 °C for 20 h. To the reaction mixture was added 2 N aqueous LiOH (200 µL). The resulting solution was stirred at 23 °C for 3 h. The mixture was diluted with water (400 µL) and was acidified to pH 2 by addition of TFA (100 µL). The acidified solution was purified by reverse-phase HPLC (YMC Pack ODS-A column, gradient elution from 20 to 40% CH₃CN-water, both containing 0.1% TFA). Compound **8** was obtained as a white powder (12.6 mg). (TOF MS ES+) m/z 431 (M+H)⁺.

Other compounds of the present invention may be prepared by those skilled in the art by varying the starting materials, reagent(s) and conditions used. Using the procedure of Example 2, the following compounds were prepared:

| **Cpd** | **MS (M+H)⁺** | **Cpd** | **MS (M+H)⁺** |
|---|---|---|---|
| **5** | 430 | **24** | 595 |
| **6** | 417 | **25** | 550 |
| **7** | 391 | **26** | 445 |
| **9** | 437 | **27** | 479 |
| **10** | 437 | **28** | 429 |
| **11** | 443 | **29** | 517 |
| **12** | 451 | **30** | 547 |
| **13** | 417 | **31** | 479 |
| **14** | 405 | **32** | 523 |
| **15** | 481 | **33** | 471 |
| **16** | 467 | **34** | 475 |
| **17** | 473 | **35** | 520 |
| **18** | 493 | **36** | 481 |
| **19** | 375 | **37** | 446 |
| **20** | 429 | **38** | 506 |
| **22** | 484 | **39** | 484 |
| **23** | 441 | **40** | 547 |

### EXAMPLE 3

### 2-(3-Benzyl-3-phenyl-ureido)-3-[4-(5-methoxy-2-methyl-3-oxo-2,3-dihydro-pyridazin-4-yl)-phenyl]-propionic acid, Cpd 48

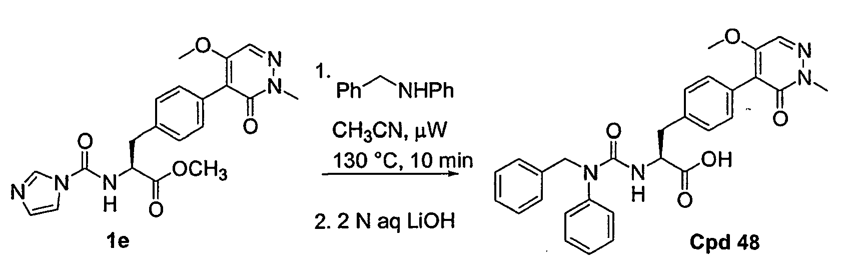

*N*-Phenylbenzylamine (17.4 mg, 94.8 µmol) was added to a solution of Compound **1e** (32.5 mg, 79.0 µmol) in acetonitrile (200 µL). The resulting solution was heated by microwave irradiation (CEM Explorer, 130 °C, 60-W, 10 min). To the resulting mixture was added an aqueous solution of LiOH (2 N, 200 µL). The mixture was stirred at 23 °C for 3.5 h. The mixture was acidified to pH 2 by the addition of trifluoroacetic acid (62 µL). The resulting mixture was filtered through a plug of Varian Chem Tube Hydromatrix, which was washed with 1% acetic acid-CH₂Cl₂ (6 × 500 µL). The filtrate was concentrated and the residue was purified by reverse-phase HPLC, affording Compound **48** as a white solid (9.6 mg). (MS ES+) *m*/*z* 514(M+H)⁺.

Other compounds of the present invention may be prepared by those skilled in the art by varying the starting materials, reagent(s) and conditions used. Using the procedure of Example 3, the following compounds were prepared:

| **Cpd** | **MS (M+H)⁺** | **Cpd** | **MS (M+H)⁺** |
|---|---|---|---|
| **41** | 479 | **49** | 463 |
| **42** | 465 | **50** | 465 |
| **43** | 523 | **51** | 521 |
| **44** | 481 | **52** | 505 |
| **45** | 502 | **53** | 495 |
| **46** | 514 | **54** | 438 |
| **47** | 499 | | |

### EXAMPLE 4

### 2-[3-(2-Fluoro-phenyl-ureido]-3-{4-[2-(2-hydroxy-ethyl)-5-methoxy-3-oxo-2,3-dihydro-pyridazin-4-yl]-phenyl}-propionic acid, Cpd 4

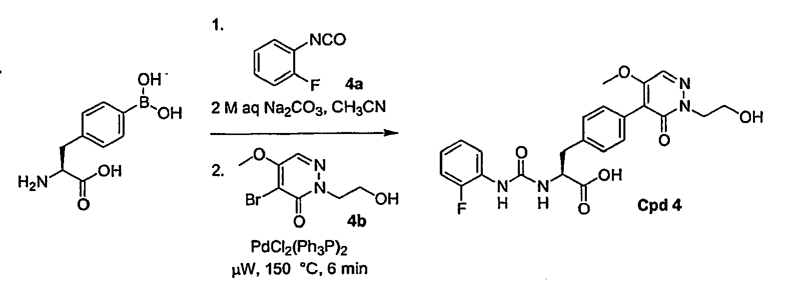

To a mixture of 4-borono-L-phenylalanine (105 mg, 0.500 mmol), acetonitrile (1 mL), and a 2 M aqueous solution of sodium carbonate (1 mL) was added Compound **4a** (2-fluorophenyl isocyanate) (61.7 µL, 0.550 mmol). The resulting mixture was stirred at 23 °C for 23 h. To the reaction mixture was added Compound **4b** (137.0 mg, 0.550 mmol) and *trans-*dichloro(bistriphenylphosphine)palladium (II) (17.5 mg, 25 µmol). The resulting mixture was heated by microwave irradiation (CEM Explorer, 150 °C, 6 min, 60 Watts). The reaction mixture was acidified to pH 2 by the addition of trifluoroacetic acid (1 mL). The resulting mixture was filtered and the filtrate was purified by reverse-phase HPLC (YMC Pack ODS-A column, gradient elution from 28 to 48% acetonitrile-water, both containing 0.1% TFA). To the collected HPLC fractions was added a 1 M aqueous solution of ammonium formate until the solution reached pH 5. The resulting solution was lyophilized and Compound **4** was obtained as a white powder (35.4 mg); (TOF MS ES+) m/z 471 (M+H)⁺.

Other compounds of the present invention may be prepared by those skilled in the art by varying the starting materials, reagent(s) and conditions used. Using the procedure of Example 4, the following compounds were prepared:

| **Cpd** | **MS (M+H)⁺** | **Cpd** | **MS (M+H)⁺** |
|---|---|---|---|
| **2** | 521 | **3** | 459 |

### EXAMPLE 5

### 2-(3-lsobutyl-3-methyl-ureido)-3-[4-(5-methoxy-2-methyl-3-oxo-2,3-dihydro-pyridazin-4-yl)-phenyl]-propionic acid 2-hydroxy-ethyl ester

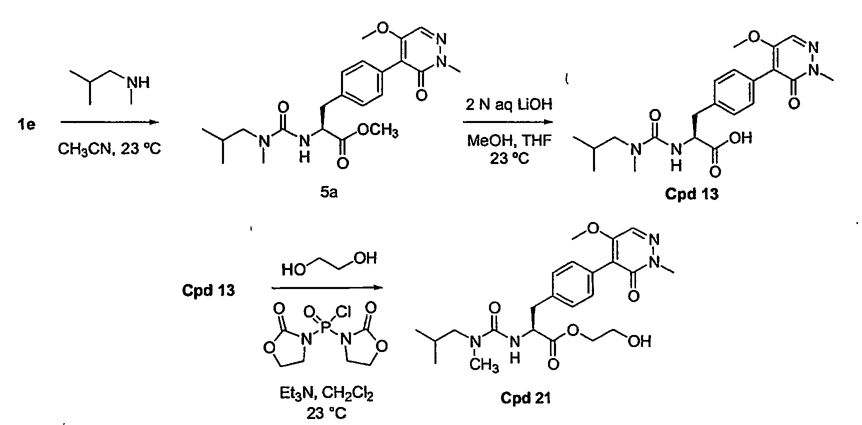

To a solution of Compound **1e** (1.18 g, 2.88 mmol, 1 equiv) in acetonitrile (15 mL) was added *N*-methylisobutylamine (0.412 mL, 3.45 mmol, 1.2 equiv). The resulting mixture was stirred at 23 °C for 18 h and was concentrated. The residual colorless oil was purified by flash column chromatography (silica gel, gradient elution from 75 to 100% ethyl acetate-hexanes), yielding Compound **5a** as a white foam (694 mg). (TOF MS ES+) *m*/*z* 431 (M+H)⁺. ¹H NMR (CDCl₃) 7.87 (s, 1H), 7.47 (d, 2H, *J* = 8.2 Hz), 7.17 (d, 2H, *J* = 8.2 Hz), 4.78-4.81 (m, 2H), 3.89 (s, 3H), 3.81 (s, 3H), 3.73 (s, 3H), 3.15 (d, 2H, *J* = 5.0 Hz), 3.03 (d, 2H, *J* = 7.3 Hz), 2.86 (s, 3H), 1.82-1.92 (m, 1H), 0.87 (d, 3H, *J* = 6.7 Hz), 0.86 (d, 3H, *J* = 6.7 Hz).

To a solution of Compound **5a** (694 mg, 1.61 mmol, 1 equiv) in a mixture of methanol (4 mL) and tetrahydrofuran (4 mL) was added a 2 N aqueous solution of lithium hydroxide (4.03 mL, 8.08 mmol, 5.0 equiv). The resulting cloudy, colorless mixture was stirred at 23 °C for 2 h. The solution was partially concentrated, to remove organic solvents and the residual mixture was acidified to pH 2 by addition of 2 N aqueous hydrochloric acid. The resulting suspension was extracted with dichloromethane (3 × 20 mL). The combined organic extracts were dried over sodium sulfate, were filtered, and were concentrated, yielding Compound **13** as a white foam (648 mg). (TOF MS ES+) *m*/*z* 417 (M+H)⁺. ¹H NMR (CD₃OD) 8.17 (s, 1 H), 7.38 (d, 2H, J = 8.3 Hz), 7.28 (d, 2H, J = 8.2 Hz), 4.56 (dd, 1H, J = 8.7, 5.0 Hz), 3.93 (s, 3H), 3.78 (s, 3H), 2.94-3.26 (m, 4H), 2.85 (s, 3H), 1.86-1.93 (m, 1 H), 0.84 (d, 3H, J = 6.6 Hz), 0.83 (d, 3H, J = 6.7 Hz).

Ethylene glycol (434 µL, 7.78 mmol), bis(2-oxo-3-oxazolidinyl)phosphinic chloride (515 mg, 2.02 mmol), and triethylamine (564 µL, 4.05 mmol) were added in sequence to a solution of Compound **13** (648 mg, 1.56 mmol) in CH₂Cl₂ (7.8 mL). The resulting mixture was stirred at 23 °C for 5 d. The mixture was concentrated and the residue was purified by reverse-phase HPLC (YMC Pack ODS-A column, gradient elution from 20 to 40% acetonitrile-water, both containing 0.1% TFA) to yield Compound **21** as a white powder (448 mg). (TOF MS ES+) m/z 461 (M+H)⁺. ¹H NMR (CD₃OD) 8.17 (s, 1H), 7.38 (d, 2H, J = 8.2 Hz), 7.28 (d, 2H, J = 8.4 Hz), 6.21 (d, 1 H, J = 7.9 Hz), 4.52-4.60 (m, 1 H), 4.11-4.24 (m, 2H), 3.93 (s, 3H), 3.77 (s, 3H), 3.68-3.72 (m, 2H), 2.96-3.25 (m, 4H), 2.86 (s, 3H), 1.80-1.94 (m, 1 H), 0.84 (d, 3H, J = 6.4 Hz), 0.83 (d, 3H, J = 6.7 Hz).

### EXAMPLE 6

### 2-(3,3-Diisopropyl-ureido)-3-[4-(5-methoxy-2-methyl-3-oxo-2,3-dihydro-pyridazin-4-yl)-phenyl]-propionic acid, Cpd 1

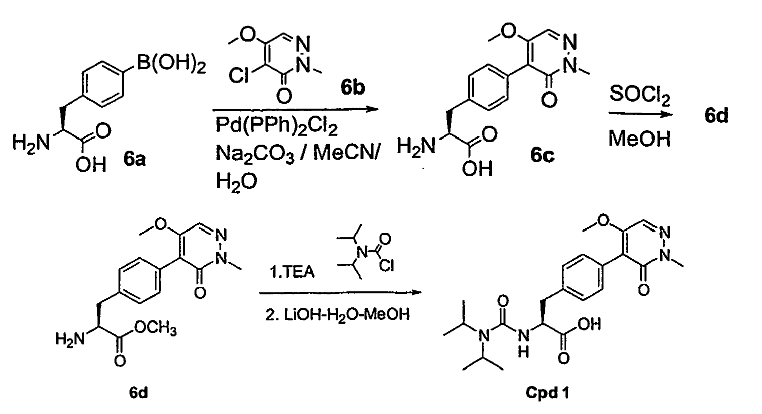

A 10 mL vial containing a magnetic stir bar was charged with Compound **6a** (4-borono-L-phenylalanine) (110 mg, 0.50 mmol), 4-chloro-5-methoxy-2-methyl-2*H*-pyridazin-3-one (Compound **6b)** (79 mg, 0.45 mmol), dichlorobis(triphenylphosphine)palladium (II) (18 mg, 0.025 mmol), 1.0 M sodium carbonate (1.0 mL, 1.0 mmol) and acetonitrile (1.0 mL). The vial was sealed and the mixture was heated under microwave irradiation at 150 °C for 10 min. The crude mixture, upon acidification with TFA and removal of the solvents, was purified by reverse phase HPLC (0.1 % TFA H₂O/MeCN, 0-20% gradient) to yield Compound **6c** as a white solid (TFA salt, 125 mg). ¹H NMR (CD₃OD) δ: 8.20 (s, 1H), 7.42 (d, 2H), 7.35 (d, 2H), 4.24, (dd, 1 H), 3.92 (s, 3H), 3.80 (s, 3H), 3.37 (dd, 1H), 3.14 (dd, 1H). MS *m*/*z:* M+1 = 304.

Compound 6c (TFA salt, 0.20 g, 0.48 mmol) was dissolved in MeOH (8 mL) and heated in the presence of SOCl₂ (0.2 mL) at 80 °C for 2 h. The solution was concentrated, and the resulting solid was treated with saturated NaHCO₃ (aq) and extracted with CH₂Cl₂ (3 x 2 mL). The organic phase was dried (MgSO₄), filtered, and concentrated to a clear gum to give Compound **6d** (0.10 g). ¹H NMR (CDCl₃) δ: 7.88 (s, 1H), 7.48 (d, 2H), 7.24 (d, 2H), 3.90 (s, 3H), 3.80 (s, 3H), 3.80 (s, 3H), 3.77 (dd, 1 H), 3.73 (s, 3H), 3.15 (dd, 1H), 2.86 (dd, 1H). MS *m*/*z*: M+1 = 318.

To a solution of Compound **6d** (32 mg, 0.10 mmol) in DCM (1 mL) was added diisopropylcarbamyl chloride (25 mg, 0.15 mmol) and TEA (0.056 mL, 0.40 mmol). The resulting mixture was stirred at rt overnight, then washed with aq NaHCO₃, and concentrated. The residue was treated with 1.0 M LiOH (0.30 mL) in MeOH (1 mL) for 2 h. The residue was acidified with 2 M HCl (aq), and then purified by reverse phase HPLC (20 to 40% MeCN-H₂O gradient containing 0.1% TFA) to yield Compound **1** as a white solid (15 mg). MS *m*/*z* 430 (M⁺). ¹H NMR (CD₃OD) δ 8.17 (s, 1 H), 7.38 (d, 2H), 7.25 (d, 2H), 4.60 (dd, 1 H, J = 8.7, 5.0 Hz), 3.89 (s, 3H), 3.82 (m, 2H), 3.77 (s, 3H), 3.27 (dd, 1 H), 3.12 (dd, 1H), 1.16 (d, 12H).

### Biological Experimental Examples

As demonstrated by biological studies described hereinafter, and shown in Table III and Table IV, the compounds of the present invention are α4β1 and α4β7 integrin receptor antagonists useful in treating integrin mediated disorders including, but not limited to, inflammatory, autoimmune and cell-proliferative disorders.

### Example 1

### Ramos Cell Adhesion Assay (α₄β₁ Mediated Adhesion / VCAM-1)

Immulon 96 well plates (Dynex) were coated with 100 µL recombinant hVCAM-1 at 4.0 µg/mL in 0.05 M NaCO₃ buffer pH 9.0 overnight at 4°C (R&D Systems). Plates were washed 2 times in PBS with 1 % BSA and blocked for 1 h @ room temperature in this buffer. PBS was removed and compounds to be tested (50 µL) were added at 2X concentration. Ramos cells, (50 µL at 2 X 10⁶/mL) labeled with 5 µM Calcein AM (Molecular Probes) for 1 h at 37 °C, were added to each well and allowed to adhere for 1 h at room temperature. Plates were washed 4 X in PBS + 1 % BSA and cells were lysed for 15 minutes in 100 µL of 1 M Tris pH 8.0 with 1 % SDS. The plate was read at 485 nm excitation and 530 nm emission. The resultant data is shown in Table III.

### Example 2

### α₄β₇ -K562 Cell Adhesion Assay (α₄β₇ Mediated Adhesion / MAdCAM-1)

M2 anti-FLAG Antibody Coated 96-well plates (Sigma) were coated for 1 hour at 4 °C with 2-8 µL / well recombinant FLAG-hMAdCAM-1 contained in 100 µL of Dulbecco's PBS, pH 7.4, with 1% BSA and 1 mM Mn²⁺ (PBS-BSA-Mn). Plates were washed once with PBS-BSA-Mn. Buffer was removed and compounds to be tested (50 µL) were added at 2x concentration. Stably transfected K562 cells expressing human α4β7 integrin, (50 µL at 2 X 10⁶/mL) that had been labeled with 100 µg / mL carboxymethyl fluorescein diacetate succinimidyl ester (CFDA-SE; Molecular Probes) for 15 min at 37 °C were added to each well and allowed to adhere for 1 h at room temperature. Plates were washed 4x in PBS-BSA-Mn and then cells were lysed for 2 min by addtion of 100 µL of PBS without Ca²⁺, Mg²⁺ supplemented with 0.1 M NaOH. The plate was read on a 96-well fluorescent plate reader at 485 nm excitation and 530 nm emission. The resultant data is shown in Table IV.

**Table III**

| **Cpd** | **α4β1 IC₅₀ (µM)** | **Cpd** | **α4β1 IC₅₀ (µM)** |
|---|---|---|---|
| **1** | > 1 | **28** | 1.80 |
| **2** | 2.87 | **29** | >5 |
| **3** | >5 | **30** | >5 |
| **4** | >5 | **31** | >5 |
| **5** | >5 | **32** | >5 |
| **6** | >5 | **33** | >5 |
| **7** | >5 | **34** | >5 |
| **8** | >5 | **35** | >5 |
| **9** | >5 | **36** | >5 |
| **10** | >5 | **37** | >5 |
| **11** | >5 | **38** | >5 |
| **12** | >5 | **39** | >5 |
| **13** | 1.75 | **40** | >5 |
| **14** | >5 | **41** | >5 |
| **15** | >5 | **42** | >5 |
| **16** | >5 | **43** | >5 |
| **17** | >5 | **44** | >5 |
| **18** | 2.65 | **45** | >5 |
| **19** | >5 | **46** | >5 |
| **20** | >5 | **47** | >5 |
| ***21** | >5 | **48** | >5 |
| **22** | >5 | **49** | >5 |
| **23** | >5 | **50** | >5 |
| **24** | >5 | **51** | >5 |
| **25** | >5 | **52** | 4.17 |
| **26** | >5 | **53** | >5 |
| **27** | >5 | **54** | >5 |

| | | | |
|---|---|---|---|
| * indicates a prodrug | | | |

**Table IV**

| **Cpd** | **α4β7 IC₅₀ (µm)** | **Cpd** | **α4β7 IC₅₀ (µM)** |
|---|---|---|---|
| **1** | 0.124 | **28** | 0.029 |
| **2** | 0.028 | **29** | 0.694 |
| **3** | 0.311 | **30** | 0.156 |
| **4** | 2.780 | **31** | 0.414 |
| **5** | 0.469 | **32** | 0.428 |
| **6** | 0.380 | **33** | 0.310 |
| **7** | 0.789 | **34** | 0.382 |
| **8** | 0.341 | **35** | 1.828 |
| **9** | 0.370 | **36** | 0.674 |
| **10** | 0.063 | **37** | 0.297 |
| **11** | 0.053 | **38** | 0.967 |
| **12** | 0.601 | **39** | 0.871 |
| **13** | 0.017 | **40** | 1.006 |
| **14** | 0.094 | **41** | 0.213 |
| **15** | 0.310 | **42** | 0.092 |
| **16** | 0.069 | **43** | 0.238 |
| **17** | 0.282 | **44** | 0.024 |
| **18** | 0.395 | **45** | 0.446 |
| **19** | 1.549 | **46** | 0.575 |
| **20** | 0.497 | **47** | 1.451 |
| ***21** | 2.896 | **48** | 0.067 |
| **22** | 0.135 | **49** | 0.390 |
| **23** | 0.183 | **50** | 0.354 |
| **24** | 0.391 | **51** | 0.052 |
| **25** | 2.018 | **52** | 0.037 |
| **26** | 0.598 | **53** | 0.128 |
| **27** | 0.218 | **54** | 0.880 |

| | | | |
|---|---|---|---|
| * indicates a prodrug | | | |

### Example 3

### In Vivo Model for Leukocytosis

Leukocytosis is the increase in circulating white blood cells (leukocytes). This can be brought about by preventing leukocyte binding to integrin counter-receptor adhesion molecules expressed on high endothelial venules. This cell adhesion occurs between immunoglobulin superfamily molecules and integrins. Relevant examples of these paired interactions include Intracellular Adhesion Molecule-1 and AlphaL Beta2 integrin, Vascular Cell Adhesion Molecule-1 and α4β1 integrin, and Mucosal Addressin Cell Adhesion Molecule-1 and α4β7 integrin, respectively.

In this model, a compound that antagonizes these leukocyte-endothelial interactions will cause an increase in circulating leukocytes, defined as leukocytosis, as measured at 1 -1.5 h post-administration. This leukocytosis is indicative that normal lymphocyte or leukocyte emigration from the peripheral circulation was prevented. Similar emigration of cells out of the circulation into inflamed tissues is responsible for the progression and maintainance of the inflammatory state. Leukocytosis is an indication that lymphocyte and leukocyte extravasation is prevented, and is predictive of general anti-inflammatory activity.

### Procedure

One week prior to being tested, 7-10 week old female Balb/c mice, n = 8 per group, were bled and randomized according to leukocyte counts. One week later, the mice were adminstered test compound orally or subcutaneously and then bled 1 - 1.5 h after drug administration, approximately 1 h after the peak blood concentration of the compound has occurred. Whole blood, 250 - 350 microliters, was collected from each mouse into potassium-EDTA serum collection tubes (Becton-Dickenson) and mixed to prevent clotting.

Cell counts and differential counts on the whole blood preparation were performed using an Advia 120 Hematology System (Bayer Diagnostics). Cell counts as total leukocytes and as total lympohcytes were made and compared to counts made from mice dosed with vehicle only. Data were reported as percent of vehicle control for lymphocyte counts and total leukocyte counts.

Statistical analyses were performed using ANOVA with Dunnet's multiple comparison test. Resulting data is shown in Table V.

**Table V**

| | | Lymphoycte Counts | | | | | | Total Leukocyte Count |
|---|---|---|---|---|---|---|---|---|
| **Cpd** | Rte | % of Vehicle Control | | | | | | % of Vehicle Control |

| | | 3 mg/kg | 10 mg/kg | 30 mg/kg | | 3 mg/kg | 10 mg/kg | 30 mg/kg |
|---|---|---|---|---|---|---|---|---|
| **13** | sc | 84.4 | 133.6 | 154.3 | | 84.4 | 133.5 | 149.5 |
| **21** | po | 148.1 | 156.8 | 189.6 | | 121.9 | 118.7 | 131.0 |

### Example 4

### In Vivo Model for Colitis: Dextran Sulfate Sodium (DSS) Induced Colitis

Inflammatory bowel diseases such as ulcerative colitis and Crohn's disease are characterized by diminished intestinal barrier function, apparent inflammatory damage that may include erosive loss of intestinal mucosa, and inflammatory infiltrates in the mucosa and submucosa.

Chemically induced models of experimental of colitis are used to mimic various aspects of these diseases. Among the many possible chemicals used are dextran sulfate sodium (DSS) and trinitrobenzene sulfonic acid (TNBS). The dextran sulfate sodium model of experimental colitis is characterized by a shrinkage of the colon's length, macrosopic inflammatory damage, diarrhea, a discontinuous pattern of mucosal epithelial damage in the distal colon with infiltration of inflammatory cells that include macrophages and neutrophils into the mucosa and submucosa (Blumberg, R. S., Saubermann, L. J., and Strober, W. Animal models of mucosal inflammation and their relation to human inflammatory bowel disease. Current Opinion in Immunology, 11: 648-656, 1999; Okayasu, I., Hatakeyama, S., Yamada, M., Ohkusa, T., Inagaki, Y., and Nakaya, R. A novel method of induction of reliable experimental acute and chronic colitis in mice. Gastroenterology, 98: 694-702, 1990; Cooper, H. S., Murthy, S. N. S., Shah, R. S., and Sedergran, D. J. Clinicopathologic study of dextran sulfate sodium experimental murine colitis. Lab Invest., 69: 238-249, 1993.; Egger, B., Bajaj-Elliott, M., MacDonald, T. T., Inglin, R., Eysselein, V. E., and Buchler, M. W. Characterization of acute murine dextran sodium sulphate colitis: Cytokine profile and dose dependency. Digestion, 62: 240-248, 2000.; Stevceva, L., Pavli, P., Husband, A. J., and Doe, W. F. The inflammatory infiltrate in the acute stage of the dextran sulphate sodium induced colitis: B cell response differs depending on the percentage of DSS used to induce it. BMC Clinical Pathology, 1: 3-13, 2001.; and Diaz-Granados, Howe, K., Lu, J, and McKay, D. M. Dextran sulfate sodium-induced colonic histopathology, but not altered epithelial ion transport, is reduced by inhibition of phosphodiesterase activity. Amer. J. Pathology, 156: 2169-2177, 2000.).

### Methodology

Balb/c female mice and C57Black/6 mice were used in these studies. The Balb/c mice were provided with a solution of tap water containing 5% DSS (ICN chemicals) ad libitum over a 7-day period. When C57/Black 6 mice were used, a solution of tap water containing 4% DSS was used. During the ensuing 7-day period, test animals were administered a preparation of an experimental compound. This material may be administered orally or intraperitoneally or subcutaneously, once or twice daily. At the end of this period, the animals were euthanized and their colons were collected for further analysis. Among the parameters analyzed were the length of the colon starting from the anus to the top of the cecum, the consistency of any stools found within the colon, and the gross macroscopic appearance of the colon. The distal colon between the 1^{st} and the 4^{th} centimeter was removed and placed in 10% neutral buffered formalin for later histological analysis.

For the following parameters, colon length, stool consistency and appearance, and macroscopic damage a scoring system is used to describe the changes. The 3 scores for each animal are added to provide a Total Macroscopic Score. Thus,

Stool Score: 0 = normal (well-formed fecal pellets); 1 = loosely-shaped moist pellets; 2 = amorphous, moist, sticky pellets; 3 = severe diarrhea.

Colon Damage Score: 0 = no inflammation ; 1 = reddening mild inflammation; 2 = moderate inflammation or more widely distributed; 3 = severe inflammation and / or extensively distributed.

Colon Length Score: 0 = < 5% shortening; 1 = 5-14% shortening; 2 = 15-24% shortening; 3 = 25 -35% shortening; 4 = > 35% shortening.

Histological analyses of tissues consisted of staining paraffin-embedded tissue sections with hematoxylin-eosin dye. Epithelial damage scores were determined as the fraction of the tissue section showing damaged epithelium. Scores were determined as follows: 0 = no damage; 1 = < 1/3 damaged, 2 = 1/3 to < 2/3 damaged, 3 = > 2/3 damaged. Resulting data is shown in Table VI and Table VII.

Statistical analysis performed in Graphpad Prism 4.0 using ANOVA with Dunnet's or Bonferroni's multiple comparison's test.

**Table VI**

| **Cpd** | **DOSE (mg/ kg)** | **Rte** | **Dose Regimen** | **% Inhibition of Total Macro scopic Score** | **SE** | **p value** | **n** | **mouse strain** |
|---|---|---|---|---|---|---|---|---|
| **55** | 30 | po | bid | 23.53 | 8.547 | p>0.05 | 10 | balb/c |
| | 60 | po | bid | 23.53 | 11.11 | p>0.05 | 10 | |

**Table VII**

| **Cpc** | **DOSE (mg/ kg)** | **Rte** | **Dose Regimen** | **% Inhibition of Epithelial Damage Score** | **SE** | **p-value** | **n** | **mouse strain** |
|---|---|---|---|---|---|---|---|---|
| **55** | 30 | po | bid | 0 | 27.22 | p > 0.05 | 10 | balb/ c |
| | 60 | po | bid | 58.34 | 13.89 | p > 0.05 | 10 | |

### Example 5

### In Vivo Model: Trinitrobenzenesulfonic acid (TNBS) Induced Colitis

The TNBS model of experimental colitis (Bobin-Dubigeon, C., Collin, X., Grimaud, N., Robert, J-M., Guillaume Le Baut, G., and Petit, J-Y. Effects of tumour necrosis factor-a synthesis inhibitors on rat trinitrobenzene sulphonic acid-induced chronic colitis. Eur. J. Pharmacology, 431: 103-110, 2001.), is characterized by shrinkage of the colon, intraperitoneal serosal adhesions, severe wounding and inflammatory damage, diarrhea, a continuous pattern of mucosal epithelial damage in the distal colon with infiltration of inflammatory cells. These symptomatic signs in the above - mentioned models are similar to what occur in human colitis.

Male Wistar rats (200-250 g) are inoculated with 500 microliters of a solution of 10 to 20 mg of TNBS in 30% ethanol delivered intracolonically via catheter or ball-tipped gavage needle to the 8^{th} cm from the anus. When Balb/c female mice (8-12 weeks of age) were used, the inoculation volume was 50 microliters containing 2-3 mg of TNBS in 30% ethanol delivered intracolonically via catheter or ball-tipped gavage needle to the 4^{th} cm from the anus. During the ensuing 7 days, test animals were administered a preparation of an experimental compound This material may be administered orally, subcutaenously or intraperitoneally, once or twice daily. At the end of this period, the animals were euthanized and their colons were collected for further analysis. Among the parameters analyzed were the length of the colon starting from the anus to the top of the cecum, the weight of the colon, the consistency of any stools found within the colon, the presence or absence of intraperitoneal adhesions on the serosal surfacr of the intestin, and the gross macroscopic appearance of the colon. The latter is scored for length and severity of inlfammatory damage using a 10 point score. In rats, The distal colon between the 5^{th} and the 8^{th} centimeter is dissected and placed in 10% neutral buffered formalin for later histological analysis. In mice, the 1^{st} to the 4^{th} cm was collected for histological analyses.

For the following parameters-colon length, colon weight, stool consistency and appearance and macroscopic damage-- a scoring system was used to describe the changes. The four scores for each animal were added to provide a Total Score.

Stool Score: 0 = normal (well-formed fecal pellets); 1 = loosely-shaped moist pellets; 2 = amorphous, moist, sticky pellets; 3 = bloody diarrhea. For the presence of blood in stool, one point was added to scores < 3.

Colon Damage Score: 0 = no inflammation; 1 = focal hyperemia; 2 = ulceration without hyperemia at one site; 3 = ulceration and hyperemia at one site; 4 = 2 or more sites of ulceration and hyperemia; 5 = multiple sites of damage extending to > 1 cm; 6-10 = multiple sites of damage extending to > 2 cm; one point was added for each additional cm of tissue involvement.

Colon Weight Score: 0 = < 5% weight gain; 1 = 5 - 14% weight gain; 2 = 15 - 24 % weight gain; 3 = 25 - 35% weight gain; 4 = > 35% weight gain.

Colon Length Score: 0 = < 5% shortening; 1 = 5-14% shortening; 2 = 15 - 24% shortening; 3 = 25 -35% shortening; 4 = > 35% shortening.

Histological analyses of tissues consisted of staining paraffin-tissues with hematoxylin-eosin dye. Epithelial damage scores were determined as the fraction of the tissue section showing damaged epithelium. Scores were determined as follows: 0 = no damage; 1 = < 1/3 damaged, 2 = 1/3 to < 2/3 damaged, 3 = > 2/3 damaged.

Resulting data is shown in Table VIII and Table IX. Statistic analyses for these experiments were performed with Graphpad Prism 4.0, using ANOVA, with Dunnets or Bonferroni's multiple comparisons test.

**Table VIII**

| **Cpd** | **DOSE (mg/kg)** | **Rte** | **Dose Regimen** | **% Inhibition of Total Macro scopic Score** | **SE** | **p value** | **n** | **Species** |
|---|---|---|---|---|---|---|---|---|
| **55** | 30 | po | bid | -7.321 | 12.57 | p>0.05 | 11 | rat |

**Table IX**

| **Cpd** | **DOSE (mg/ kg)** | **Rte** | **Dose Regimen** | **% Inhibition of Epithelial Damage Score** | **SE** | **p value** | **n** | **Species** |
|---|---|---|---|---|---|---|---|---|
| **55** | 30 | po | bid | 1.818 | 24.64 | p>0.05 | 11 | rat |

## Claims

1. A compound of Formula (I) 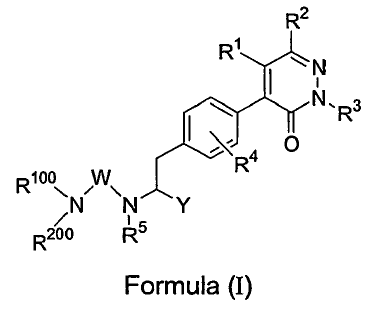 wherein
**R¹** is independently selected from the group consisting of hydrogen, C₁₋₆alkyl, C₁₋₆alkoxy, aryl, heteroaryl, heterocyclyl, benzo fused heterocyclyl, benzo fused cycloalkyl, heteroaryl fused heterocyclyl, heteroaryl fused cycloalkyl, aryloxy, heteroaryloxy, heterocyclyloxy, cycloalkyloxy, -NR¹⁰R²⁰, halogen, hydroxy, and -S(C₁₋₆)alkyl; wherein C₁₋₆alkoxy is optionally substituted with one to four substituents independently selected from R^{a}; wherein R^{a} is independently selected from the group consisting of aryl, heteroaryl, heterocyclyl, cycloalkyl, (C₁₋₆)alkoxycarbonyl, carboxy, amino, alkylamino, dialkylamino, hydroxy(C₁₋₆)alkoxy, one to three halogen atoms, and hydroxy;
wherein **R**¹⁰ and **R**²⁰ are independently selected from the group consisting of hydrogen, C₁₋₆alkyl, allyl, halogenated C₁₋₆alkyl, hydroxy, hydroxy(C₁₋₄)alkyl, aryl(C₁₋₄)alkyl, aryl, and cycloalkyl; additionally, R¹⁰ and R²⁰ are optionally taken together with the atoms to which they are attached to form a five to seven membered monocyclic ring;
wherein the aryl and aryloxy substituents of R¹ are optionally substituted with a substituent independently selected from the group consisting of one to three C₁₋₆alkyl substituents, hydroxy(C₁₋₆)alkyl, ary(C₁₋₆)alkyl, C₁₋₆alkoxy, aryl, heteroaryl, C₁₋₆alkoxycarbonyl, aryl(C₁₋₆)alkoxycarbonyl, C₁₋₆alkylcarbonyl, aminocarbonyl, alkylaminocarbonyl, dialkylaminocarbonyl, hydroxy, cyano, nitro, -SO₂(C₁₋₃)alkyl, -SO₂aryl, -SO₂heteroaryl, trifluoromethyl, trifluoromethoxy, and one to three halogen atoms;
and wherein the heteroaryl and heterocyclyl substituents of R¹ are optionally substituted with one to three substituents independently selected from the group consisting of C₁₋₆alkyl, C₁₋₆alkoxy, aryl, heteroaryl, halogen, and hydroxy;
additionally, R¹ and R² are optionally taken together with the atoms to which they are attached to form a five to seven membered carbocyclic or heterocyclic ring;
**R²** is independently selected from the group consisting of hydrogen, C₁₋₆alkyl, C₁₋₆alkoxy, C₂₋₆alkenyloxy, hydroxy, amino, alkylamino, dialkylamino, and halogen; wherein R¹ and R² are optionally taken together with the atoms to which they are attached to form a five to seven membered carbocyclic or heterocyclic ring;
**R³** is independently selected from the group consisting of hydrogen, C₁₋₆alkyl, C₂₋₆alkenyl, C₂₋₆alkynyl, aryl, heteroaryl, heterocyclyl, and cycloalkyl; wherein alkyl, alkenyl, and alkynyl are optionally substituted with a substituent independently selected from the group consisting of aminocarbonyl, alkylaminocarbonyl, dialkylaminocarbonyl, aryl, heteroaryl, heterocyclyl, cycloalkyl, carboxy, one to three halogen atoms, hydroxy, and -C(=O)C₁₋₆alkyl;
**R⁴** is independently selected from the group consisting of hydrogen, fluorine, chlorine, and methyl;
**R⁵** is hydrogen or C₁₋₃alkyl, provided that R⁵ is C₁₋₃alkyl only when taken with Y and the atoms to which R⁵ and Y are attached to form a five to seven membered heterocycle;
**Y** is independently selected from the group consisting of hydroxymethyl, -C(=O)NH₂, -C(=O)NH(OH), -C(=O)NH(C₁₋₆alkyl), -C(=O)NH(hydroxy(C₁₋₆)alkyl), -C(=O)N(C₁₋₆alkyl)₂, -C(=O)NHSO₂(C₁₋₄)alkyl, carboxy, tetrazolyl, and -C(=O)C₁₋₆alkoxy; wherein said alkoxy is optionally substituted with one to two substituents independently selected from the group consisting of hydroxy, -NR³⁰R⁴⁰, heterocyclyl, heteroaryl, halogen, and -OCH₂CH₂OCH₃; wherein R³⁰ and R⁴⁰ are independently selected from the group consisting of hydrogen, C₁₋₆alkyl, hydroxy, and hydroxy(C₁₋₄)alkyl, wherein said R³⁰ and R⁴⁰ are optionally taken together with the atoms to which they are attached to form a five to ten membered monocyclic ring;
**W** is independently selected from the group consisting of -C(=O)- and -C(=S)-;
**R¹⁰⁰** and **R²⁰⁰** are independently selected from the group consisting of hydrogen, C₁₋₆alkyl, C₁₋₆alkenyl, C₁₋₆alkynyl, aryl, heteroaryl, cycloalkyl, polycycloalkyl, heterocyclyl, hydroxy, and arylamino;
wherein the alkyl, C₁₋₆alkenyl, C₁₋₆alkynyl, and alkoxy substituents of R¹⁰⁰ and R²⁰⁰ are optionally substituted with a substituent independently selected from the group consisting of aryl, heteroaryl, heterocyclyl, hydroxy, one to three halogen atoms, amino, C₁₋₆₆alkylamino, hydroxy(C₁₋₆)alkylamino, di(C₁₋₆)alkylamino, -C(=O)amino, and -C(=O)C₁₋₆alkoxy;
wherein said aryl, heteroaryl, and heterocyclyl substituents may be optionally substituted with up to four substituents selected from the group consisting of C₁₋₆alkyl, C₁₋₆alkoxy, halogen, aryl, amino, alkylamino, dialkylamino, hydroxy, polycycloalkyl, and heteroaryl, additionally said heterocyclyl is optionally substituted with one to three oxo substituents;
wherein said aryl, heteroaryl, and heterocyclyl of R¹⁰⁰ and R²⁰⁰ are optionally substituted with one to four substituents independently selected from the group consisting of C₁₋₆alkyl, trifluoroalkyl, C₁₋₆alkoxy, trifluoroalkoxy, halogen, aryl, amino, alkylamino, dialkylamino, arylamino, hydroxy, polycycloalkyl, and heteroaryl; additionally heterocyclyl is optionally substituted with one to three oxo substituents;
and wherein R¹⁰⁰ and R²⁰⁰ are optionally taken with the nitrogen atom to which they are both attached to form a five to ten membered heterocycle or a nine to ten membered benzo-fused heterocycle;
wherein the heterocycle being is optionally substituted with up to four substituents independently selected from the group consisting of C₁₋₆alkyl, C₁₋₆alkoxy, halogen, aryl, heteroaryl, amino, alkylamino, dialkylamino, hydroxy, polycycloalkyl, or and oxo;
and an optical isomer, enantiomer, diastereomer, racemate, or pharmaceutically acceptable salt thereof.

2. The compounds of claim 1 wherein R¹ is independently selected from the group consisting of hydrogen, C₁₋₄alkyl, C₁₋₄alkoxy, aryl, heteroaryl, heterocyclyl, benzo fused cycloalkyl benzo fused heterocyclyl, aryloxy, heteroaryloxy, heterocyclyloxy, cycloalkyloxy, -NR¹⁰R²⁰, halogen, hydroxy, and -S(C₁₋₆)alkyl; wherein C₁₋₄alkoxy of R¹ is optionally substituted with one to three substituents independently selected from R^{a};
wherein R^{a} is independently selected from the group consisting of aryl, heteroaryl, heterocyclyl, cycloalkyl, dialkylamino, hydroxy(C₁₋₆)alkoxy, one to three halogen atoms, and hydroxy;
wherein R¹⁰ and R²⁰ are independently selected from the group consisting of hydrogen, C₁₋₆alkyl, allyl, halogenated C₁₋₆alkyl, and cycloalkyl; additionally, R¹⁰ and R²⁰ are optionally taken together with the atoms to which they are attached to form a five to seven membered monocyclic ring;
wherein the aryl and aryloxy substituents of R¹ are optionally substituted with a substituent independently selected from the group consisting of C₁₋₆alkyl, C₁₋₆alkoxy, phenyl, heteroaryl, aminocarbonyl, alkylaminocarbonyl, dialkylaminocarbonyl, hydroxy, cyano, nitro, -SO₂(C₁₋₃)alkyl, -SO₂aryl, trifluoromethyl, trifluoromethoxy, and halogen;
and wherein the heteroaryl and heterocyclyl substituents of R¹ are optionally substituted with one to three substituents independently selected from the group consisting of C₁₋₆alkyl, halogen, and hydroxy;
additionally, R¹ and R² are optionally taken together with the atoms to which they are attached to form a five to seven membered carbocyclic or heterocyclic ring.

3. The compounds of claim 1 wherein R¹ is independently selected from the group consisting of hydrogen, ethyl, methoxy, ethoxy, 2-hydroxyeth-1-oxy, iso-propoxy, iso-butoxy, difluoromethoxy, 2,2,2-trifluoro-eth-1-oxy, benzyloxy, cyclopropylmethoxy, pyridin-3-ylmethoxy, (1-methyl)-pyrrolidinyl-3-oxy, cyclobutyloxy, cyclopentyloxy, cyclohexyloxy, indazol-1-yl, thiophen-3-yl, [1,3]benzodioxol-5-yl, (2-methyl)-imidazol-1-yl, (1-methyl)-piperidin-4-yloxy, 2-(morpholin-4-yl)-ethoxy, (4-bromo)-pyrazol-1-yl, N-pyrrolidinyl, (3, 5-dimethyl)-pyrazol-1-yl, morpholin-4-yl, hydroxy, -(OCH₂CH₂)₂OH, phenyl (optionally substituted with a substituent independently selected from the group consisting of -SO₂Me, -C(=O)NH₂, -OCF₃, -CF₃, cyano, fluoro, and methoxy), amino, cyclopropylamino, allylamino, methylamino, hydroxy, chloro, and -SMe;
and wherein R¹ is optionally taken together with R² to form a 1,4-dioxanyl or a oxazinyl.

4. The compounds of claim 1 wherein R¹ is methoxy.

5. The compounds of claim 1 wherein R² is independently selected from the group consisting of hydrogen, C₁₋₄alkyl, C₁₋₄alkoxy, C₂₋₄alkenyloxy, hydroxy, amino, and halogen; wherein R¹ and R² are optionally taken together with the atoms to which they are attached to form a five to seven membered carbocyclic or heterocyclic ring.

6. The compounds of claim 1 wherein R² is independently selected from the group consisting of hydrogen, C₁₋₄alkyl, C₁₋₄alkoxy, hydroxy, amino, alkylamino, and halogen; wherein R² is optionally taken together with R¹ to form a 1,4-dioxanyl or a oxazinyl ring.

7. The compounds of claim 1 wherein R² is independently selected from the group consisting of hydrogen, C₁₋₄alkoxy, amino, or alkylamino; wherein R² is optionally taken together with R¹ to form a 1,4-dioxanyl or a oxazinyl ring.

8. The compounds of claim 1 wherein R³ is independently selected from the group consisting of hydrogen, C₁₋₆alkyl, aryl, heteroaryl, heterocyclyl, and cycloalkyl; wherein C₁₋₆alkyl is optionally substituted with a substituent independently selected from the group consisting of - C(=O)NH₂, aryl, heteroaryl, heterocyclyl, cycloalkyl, carboxy, one to three halogen atoms, hydroxy, and -C(=O)C₁₋₆alkyl.

9. The compounds of claim 1 wherein R³ is independently selected from the group consisting of hydrogen, C₁₋₄alkyl, cycloalkyl, and aryl; wherein C₁₋₄alkyl is optionally substituted with a substituent independently selected from the group consisting of -C(=O)C₁₋₄alkyl, -C(=O)NH₂, carboxy, heterocyclyl, phenyl, cyclopropyl, hydroxy, and one to three fluorine atoms.

10. The compounds of claim 1 wherein R³ is independently selected from the group consisting of hydrogen, C₁₋₄alkyl, and phenyl; wherein C₁₋₄alkyl is optionally substituted with a substituent independently selected from the group consisting of -C(=O)C₁₋₄alkyl, -C(=O)NH₂, carboxy, morpholinyl, cyclopropyl, hydroxy, and one to three fluorine atoms.

11. The compounds of claim 1 wherein R³ is independently selected from the group consisting of hydrogen, methyl, ethyl, and phenyl; wherein methyl and ethyl are optionally substituted with a substituent independently selected from the group consisting of -C(=O)C₁₋₄alkyl, -C(=O)NH₂, carboxy, morpholinyl, cyclopropyl, hydroxy, and one to three fluorine atoms.

12. The compounds of claim 1 wherein R³ is independently selected from the group consisting of hydrogen and 2-hydroxy-eth-1-yl.

13. The compounds of claim 1 wherein R⁴ is independently selected from the group consisting of hydrogen, fluorine, and chlorine.

14. The compounds of claim 1 wherein R⁴ is independently selected from the group consisting of hydrogen and fluorine.

15. The compounds of claim 1 wherein R⁴ is hydrogen.

16. The compounds of claim 1 wherein R⁵ is hydrogen or C₁₋₃alkyl, provided that R⁵ is C₁₋₃alkyl only when taken with Y and the atoms to which R⁵ and Y are attached to form a five to seven membered heterocycle.

17. The compounds of claim 1 wherein R⁵ is hydrogen or methylene, provided that R⁵ is methylene only when taken with Y and the atoms to which R⁵ and Y are attached to form a five membered heterocycle.

18. The compounds of claim 1 wherein R⁵ is hydrogen.

19. The compounds of claim 1 wherein Y is independently selected from the group consisting of hydroxymethyl, -C(=O)NH₂, -C(=O)NH(OH), -C(=O)NH(2-hydroxyeth-1-yl), carboxy, tetrazolyl, -C(=O)NHSO₂(C₁₋₄)alkyl, and -C(=O)C₁₋₆alkoxy; wherein said alkoxy is optionally substituted with one to two substituents independently selected from the group consisting of hydroxy, -NR³⁰R⁴⁰, heterocyclyl, heteroaryl, halogen, and
-OCH₂CH₂OCH₃; wherein R³⁰ and R⁴⁰ are independently selected from the group consisting of hydrogen and C₁₋₆alkyl.

20. The compounds of claim 1 wherein Y is independently selected from the group consisting of carboxy, tetrazolyl, -C(=O)NH(2-hydroxyeth-1-yl) and -C(=O)C₁₋₄alkoxy; wherein said alkoxy is optionally substituted with one to two substituents independently selected from the group consisting of hydroxy, -NH₂, -NH(C₁₋₄)alkyl, -N(C₁₋₄alkyl)₂, heterocyclyl, halogen, and -OCH₂CH₂OCH₃.

21. The compounds of claim 1 wherein Y is independently selected from the group consisting of carboxy, 1*H*-tetrazol-5-yl, and -C(=O)C₁₋₄alkoxy; wherein said alkoxy is optionally substituted with a substituent independently selected from the group consisting of hydroxy, -Nme₂, morpholin-1-yl, chloro, and -OCH₂CH₂OCH₃.

22. The compounds of claim 1 wherein Y is independently selected from the group consisting of carboxy, 1*H*-tetrazol-5-yl, or -C(=O)ethoxy; wherein ethoxy is optionally substituted with a substituent independently selected from the group consisting of hydroxy, chlorine, -NMe₂, and -OCH₂CH₂OCH₃.

23. The compounds of claim 1 wherein W is independently selected from the group consisting of -C(=O)-and -C(=S)-.

24. The compounds of claim 1 wherein W is -C(=O)-.

25. The compounds of claim 1 wherein R¹⁰⁰ and R²⁰⁰ are substituents independently selected from the group consisting of hydrogen, C₁₋₄alkyl, C₁₋₄alkynyl, aryl, heteroaryl, cycloalkyl, polycycloalkyl, heterocyclyl, phenylamino, and hydroxy; wherein said aryl, heteroaryl, and heterocyclyl may be optionally substituted with up to four substituents independently selected from the group consisting of C₁₋₆alkyl, hydroxy(C₁₋₄)alkyl, halogen, aryl, trifluoromethyl, trifluoromethoxy, heteroaryl, and hydroxy;
wherein C₁₋₄alkyl and C₁₋₄alkynyl of R¹⁰⁰ and R²⁰⁰ are optionally substituted with a substituent independently selected from the group consisting of heteroaryl, aryl, hydroxy, one to three halogen atoms, amino, alkylamino, and dialkylamino;
wherein R¹⁰⁰ and R²⁰⁰ are optionally taken with the nitrogen atom to which they are both attached to form a five to seven membered heterocycle or nine to ten membered benzo-fused heterocycle; optionally substituted with up to four substituents selected from C₁₋₆alkyl, halogen, aryl, and heteroaryl.

26. The compounds of claim 1 wherein R¹⁰⁰ and R²⁰⁰ are independently selected from the group consisting of hydrogen, C₁₋₄alkyl, aryl, cycloalkyl, and heterocyclyl; wherein said aryl and heterocyclyl are optionally substituted with up to four substituents independently selected from the group consisting of C₁₋₃alkyl, halogen, trifluoromethyl, trifluoromethoxy, heteroaryl, and hydroxy;
wherein R¹⁰⁰ and R²⁰⁰ are optionally taken with the nitrogen atom to which they are both attached to form a five to seven membered heterocycle optionally substituted with up to four substituents independently selected from the group consisting of C₁₋₆alkyl, halogen, aryl, and heteroaryl.

27. The compounds of claim 1 wherein R¹⁰⁰ and R²⁰⁰ are independently selected from the group consisting of hydrogen, C₁₋₄alkyl, aryl, cycloalkyl, and a five to seven membered heterocyclyl , said heterocyclyl is formed when R¹⁰⁰ and R²⁰⁰ are taken together with the nitrogen atom to which they are both attached);
wherein said aryl and heterocyclyl are optionally substituted with up to four substituents independently selected from the group consisting of C₁₋₃alkyl, halogen, trifluoromethyl, trifluoromethoxy, and hydroxy.

28. The compounds of claim 1 wherein R¹⁰⁰ and R²⁰⁰ are substituents independently selected from the group consisting of hydrogen, methyl, ethyl, *i*-but-1-yl, cyclohexyl, 2-methyl-piperadin-1-yl, and phenyl; wherein said phenyl is substituted with one to two substitutents independently selected from the group consisting of methyl, chloro, hydroxy, trifluoromethyl, and trifluoromethoxy.

29. A compound of Formula (la) according to claim 1 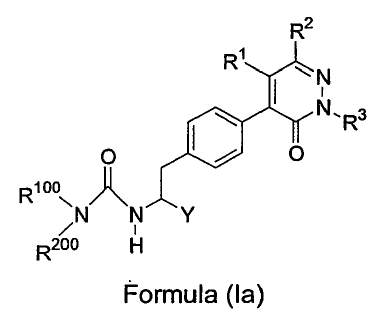 wherein:
R¹ is independently selected from the group consisting of hydrogen, C₁₋₄alkyl, C₁₋₄alkoxy, aryl, heteroaryl, heterocyclyl, benzo fused cycloalkyl benzo fused heterocyclyl, aryloxy, heteroaryloxy, heterocyclyloxy, cycloalkyloxy, -NR¹⁰R²⁰ , halogen, hydroxy, and -S(C₁₋₆)alkyl; wherein C₁₋₄alkoxy of R¹ is optionally substituted with one to three substituents independently selected from R^{a};
wherein R^{a} is independently selected from the group consisting of aryl, heteroaryl, heterocyclyl, cycloalkyl, dialkylamino, hydroxy(C₁₋₆)alkoxy, one to three halogen atoms, and hydroxy;
wherein R¹⁰ and R²⁰ are independently selected from the group consisting of hydrogen, C₁₋₆alkyl, allyl, halogenated C₁₋₆alkyl, and cycloalkyl; additionally, R¹⁰ and R²⁰ are optionally taken together with the atoms to which they are attached to form a five to seven membered monocyclic ring;
wherein the aryl and aryloxy substituents of R¹ are optionally substituted with a substituent independently selected from the group consisting of C₁₋₆alkyl, C₁₋₆alkoxy, phenyl, heteroaryl, aminocarbonyl, alkylaminocarbonyl, dialkylaminocarbonyl, hydroxy, cyano, nitro, -SO₂(C₁₋₃)alkyl, -SO₂aryl, trifluoromethyl, trifluoromethoxy, and halogen;
and wherein the heteroaryl and heterocyclyl substituents of R¹ are optionally substituted with one to three substituents independently selected from the group consisting of C₁₋₆alkyl, halogen, and hydroxy;
additionally, R¹ and R² are optionally taken together with the atoms to which they are attached to form a five to seven membered carbocyclic or heterocyclic ring;
R² is independently selected from the group consisting of hydrogen, C₁₋₄alkyl, C₁₋₄alkoxy, C₂₋₄alkenyloxy, hydroxy, amino, and halogen; wherein R¹ and R² are optionally taken together with the atoms to which they are attached to form a five to seven membered carbocyclic or heterocyclic ring;
R³ is independently selected from the group consisting of hydrogen, C₁₋₄alkyl, cycloalkyl, and aryl; wherein C₁₋₄alkyl is optionally substituted with a substituent independently selected from the group consisting of -C(=O)C₁₋₄alkyl, -C(=O)NH₂, carboxy, heterocyclyl, phenyl, cyclopropyl, hydroxy, and one to three fluorine atoms;
Y is independently selected from the group consisting of carboxy, tetrazolyl, -C(=O)NH(2-hydroxyeth-1-yl) and -C(=O)C₁₋₄alkoxy; wherein said alkoxy is optionally substituted with one to two substituents independently selected from the group consisting of hydroxy, -NH₂, -NH(C₁₋₄)alkyl, -N(C₁₋₄alkyl)₂, heterocyclyl, halogen, and -OCH₂CH₂OCH₃;
R¹⁰⁰ and R²⁰⁰ are independently selected from the group consisting of hydrogen, C₁₋₄alkyl, aryl, cycloalkyl, and heterocyclyl; wherein said aryl and heterocyclyl are optionally substituted with up to four substituents independently selected from the group consisting of C₁₋₃alkyl, halogen, trifluoromethyl, trifluoromethoxy, heteroaryl, and hydroxy;
wherein R¹⁰⁰ and R²⁰⁰ are optionally taken with the nitrogen atom to which they are both attached to form a five to seven membered heterocycle optionally substituted with up to four substituents independently selected from the group consisting of C₁₋₆alkyl, halogen, aryl, and heteroaryl; and an optical isomer, enantiomer, diastereomer, racemate, or pharmaceutically acceptable salt thereof.

30. A compound of Formula (la) according to claim 1 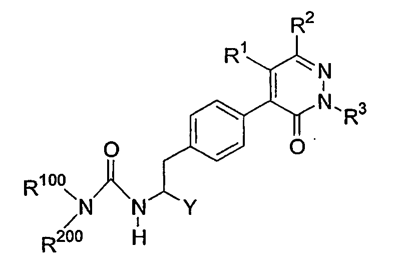 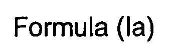 wherein:
R¹ is independently selected from the group consisting of hydrogen, ethyl, methoxy, ethoxy, 2-hydroxyeth-1-oxy, iso-propoxy, iso-butoxy, difluoromethoxy, 2,2,2-trifluoro-eth-1-oxy, benzyloxy, cyclopropylmethoxy, pyridin-3-ylmethoxy, (1-methyl)-pyrrolidinyl-3-oxy, cyclobutyloxy, cyclopentyloxy, cyclohexyloxy, indazol-1-yl, thiophen-3-yl, [1,3]benzodioxol-5-yl, (2-methyl)-imidazol-1-yl, (1-methyl)-piperidin-4-yloxy, 2-(morpholin-4-yl)-ethoxy, (4-bromo)-pyrazol-1-yl, N-pyrrolidinyl, (3, 5-dimethyl)-pyrazol-1-yl, morpholin-4-yl, hydroxy, -(OCH₂CH₂)₂OH, phenyl (optionally substituted with a substituent independently selected from the group consisting of -SO₂Me, -C(=O)NH₂, -OCF₃, -CF₃, cyano, fluoro, and methoxy), amino, cyclopropylamino, allylamino, methylamino, hydroxy, chloro, and -SMe;
and wherein R¹ is optionally taken together with R² to form a 1,4-dioxanyl or a, oxazinyl ring;
R² is independently selected from the group consisting of hydrogen, C₁₋₄alkyl, C₁₋₄alkoxy, hydroxy, amino, alkylamino, and halogen; wherein R² is optionally taken together with R¹ to form a 1,4-dioxanyl or an oxazinyl ring;
R³ is independently selected from the group consisting of hydrogen, C₁₋₄alkyl, and phenyl; wherein C₁₋₄alkyl is optionally substituted with a substituent independently selected from the group consisting of -C(=O)C₁₋₄alkyl, -C(=O)NH₂, carboxy, morpholinyl, cyclopropyl, hydroxy, and one to three fluorine atoms;
Y is independently selected from the group consisting of carboxy, 1 H-tetrazol-5-yl, and -C(=O)C₁₋₄alkoxy; wherein said alkoxy is optionally substituted with a substituent independently selected from the group consisting of hydroxy, -NMe₂, morpholin-1-yl, chloro, and -OCH₂CH₂OCH₃;
R¹⁰⁰ and R²⁰⁰ are independently selected from the group consisting o fhydrogen, C₁₋₄alkyl, aryl, cycloalkyl, and a five to seven membered heterocyclyl, said heterocyclyl being formed from R¹⁰⁰ and R²⁰⁰ taken together with the nitrogen atom to which they are both attached;
wherein said aryl and heterocyclyl are optionally substituted with up to four substituents independently selected from the group consisting of C₁₋₃alkyl, halogen, trifluoromethyl, trifluoromethoxy, and hydroxy; and an optical isomer, enantiomer, diastereomer, racemate, or pharmaceutically acceptable salt thereof.

31. A compound of Formula (Ib) according to claim 1 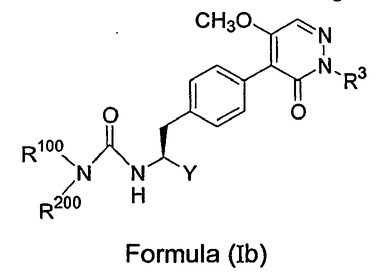 wherein Y, R³, R¹⁰⁰, and R²⁰⁰ are dependently selected from the group consisting of:
(d denotes a diastereomeric mixture)
| **R³** | **Y** | **R¹⁰⁰** | **R²⁰⁰** | **Stereochem. of R¹⁰⁰** |
|---|---|---|---|---|
| CH₃ | CO₂H | *i*-propyl | *i*-propyl | |
| (2-OH)-eth-1-yl | CO₂H | (2,6-Cl₂)phenyl | H | |
| (2-OH)-eth-1-yl | CO₂H | cyclohexyl | H | |
| (2-OH)-eth-1-yl | CO₂H | (2-F)phenyl | H | |
| CH₃ | CO₂H | (4-Me)piperazin-1-yl | | |
| CH₃ | CO₂H | (3-OH)pyrrolidin-1-yl | | d |
| CH₃ | CO₂H | (2-OH)-eth-1-yl | H | |
| CH₃ | CO₂H | (2-hydroxy methyl)-pyrrolidin-1-yl | H | S |
| CH₃ | CO₂H | benzyl | H | |
| CH₃ | CO₂H | phenyl | methyl | |
| CH₃ | CO₂H | cyclohexyl | methyl | |
| CH₃ | CO₂H | benzyl | methyl | |
| CH₃ | CO₂H | methyl | *i*-butyl | |
| CH₃ | CO₂H | (2-OH)-eth-1-yl | methyl | |
| CH₃ | CO₂H | benzyl | (2-OH)-eth-1-yl | |
| CH₃ | CO₂H | phenyl | (2-OH)-eth-1-yl | |
| CH₃ | CO₂H | cyclohexyl | (2-OH)-eth-1-yl | |
| CH₃ | CO₂H | *i*-butyl | benzyl | |
| CH₃ | CO₂H | methyl | methyl | |
| CH₃ | CO₂H | cyclohexyl | H | |
| CH₃ | 2-OH-ethoxy carbonyl | *i*-butyl | methyl | |
| CH₃ | CO₂H | 1-methyl-piperidin-4-yl | cyclopropyl | |
| CH₃ | CO₂H | norbornan-2-yl | H | S |
| CH₃ | CO₂H | 4-(3-chloro-5-trifluoromethyl-pyridin-2-yl)-piperazin-1-yl | | |
| CH₃ | CO₂H | 4-(adamantan-2-yl)-piperazin-1-yl | | |
| CH₃ | CO₂H | cyclohexyl | hydroxy | |
| CH₃ | CO₂H | benzyl | *i*-propyl | |
| CH₃ | CO₂H | 2-methyl-piperidin-1-yl | | d |
| CH₃ | CO₂H | (1-phenyl)-pyrazol-4-ylmethyl | methyl | |
| CH₃ | CO₂H | 4-(Benzooxazol-2-yl)-[1,4]diazepan-1-yl | | |
| CH₃ | CO₂H | furan-2-ylmethyl | but-2-yn-1-yl | |
| CH₃ | CO₂H | 10-methoxy-3,4,5,6-tetrahydro-2*H*-benzo[b][1,5]oxacinyl | | |
| CH₃ | CO₂H | cyclohexyl | *i*-propyl | |
| CH₃ | CO₂H | 4-(2-hyd roxy-eth-1-yl)-piperazin-1-ylamino | H | |
| CH₃ | CO₂H | 1-benzyl-pyrrolidin-3-yl | methyl | |
| CH₃ | CO₂H | (4-Hydroxy-1,1-dioxo-tetrahydro-1λ⁶-thiopheny -3-yl | H | 4S,3S |
| CH₃ | CO₂H | 2-(dimethylamin o)-eth-1-yl | ethyl | |
| CH₃ | CO₂H | 2-(phenylamino-methyl)-pyrrolidin-1-yl | | |
| CH₃ | CO₂H | 2-(pyrrolidin-1-ylmethyl)-pyrrolidin-1-yl | | S |
| CH₃ | CO₂H | 1-Aza-bicyclo [2.2.2]oct-3-yl | pyridin-3-ylmethyl | d |
| CH₃ | CO₂H | phenyl | *i*-butyl | |
| CH₃ | CO₂H | phenyl | *i*-propyl | |
| CH₃ | CO₂H | 4-(*i-*propylamino)-phenyl | *i*-propyl | |
| CH₃ | CO₂H | (2-methyl-5-hydroxy)-phenyl | ethyl | |
| CH₃ | CO₂H | naphth-1-yl | ethyl | |
| CH₃ | CO₂H | phenyl | phenylamino | |
| CH₃ | CO₂H | phenyl | phenyl | |
| CH₃ | CO₂H | benzyl | phenyl | |
| CH₃ | CO₂H | (2-methyl)-indolin-1-yl | | d |
| CH₃ | CO₂H | phenyl | n-propyl | |
| CH₃ | CO₂H | (2-trifluorometho xy)-phenyl | methyl | |
| CH₃ | CO₂H | (2-trifluoromethyl )-phenyl | methyl | |
| CH₃ | CO₂H | 6-fluoro-2-methyl-3,4-dihydro-2*H*-quinolin-1-yl | | d |
| CH₃ | CO₂H | pyridin-2-yl and | methyl | |
| CH₃ | -C(=O) O(CH₂)₂O H | s-but-1-yl | methyl | |

32. A compound according to claim 1 selected from the group consisting of
| |
|---|
| 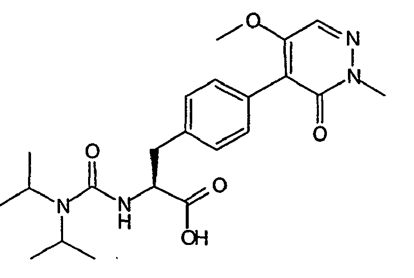 |
| 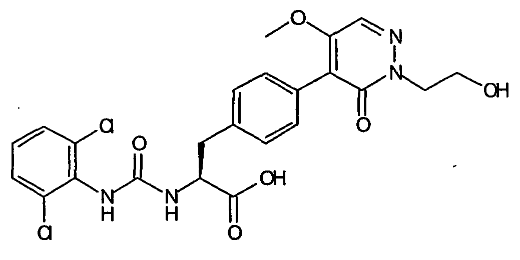 |
| 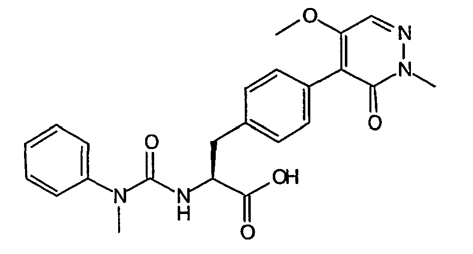 |
| 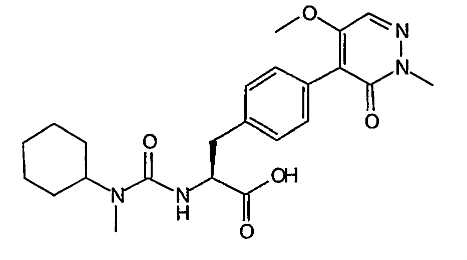 |
| 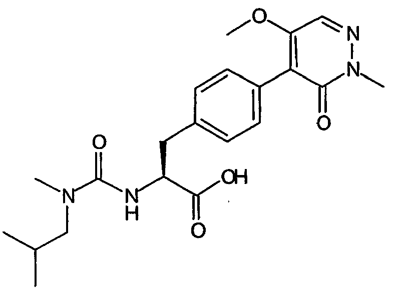 |
| 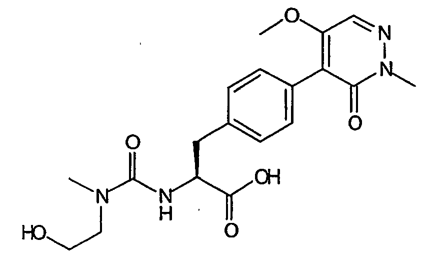 |
| 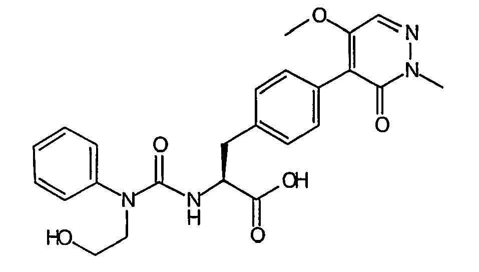 |
| 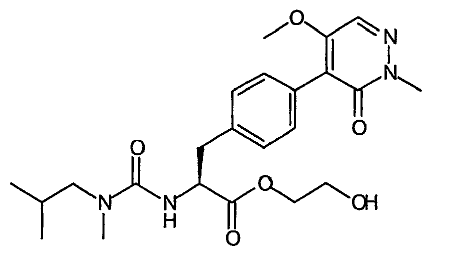 |
| 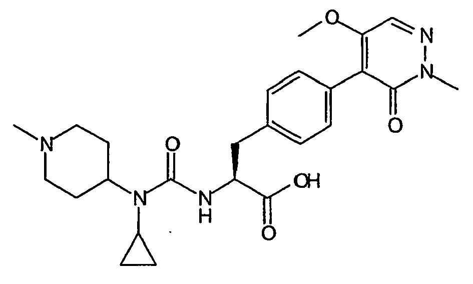 |
| 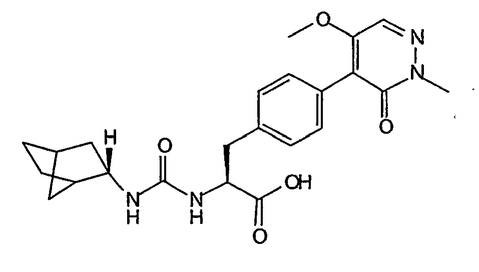 |
| 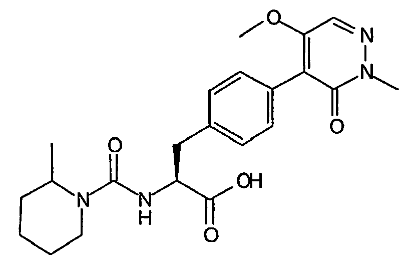 |
| 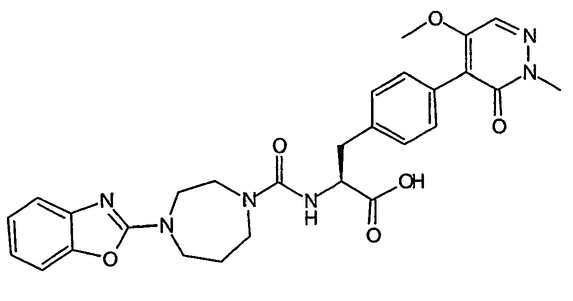 |
| 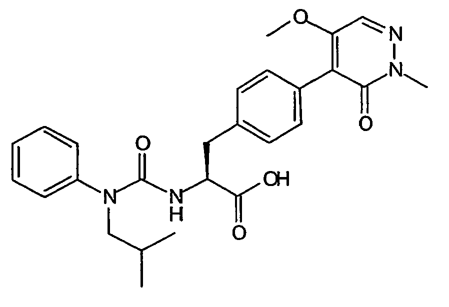 |
| 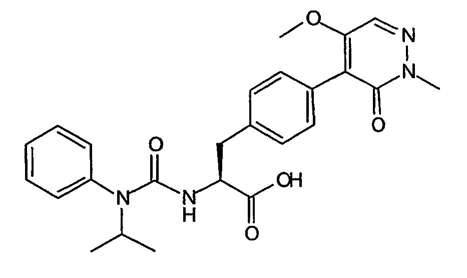 |
| 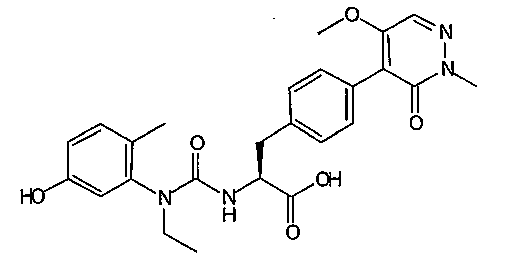 |
| 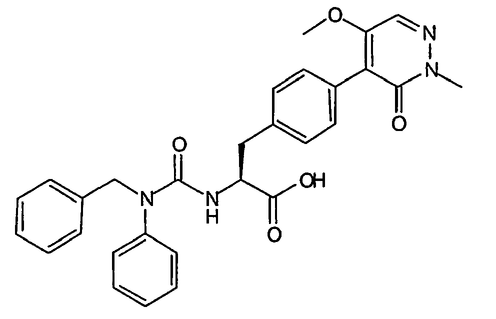 |
| 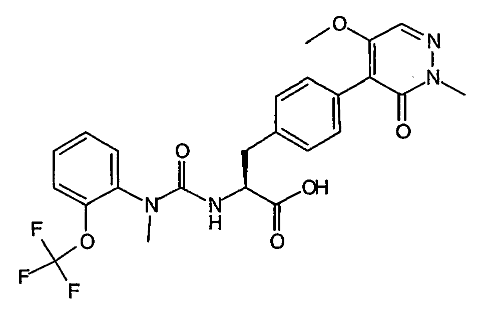 |
| 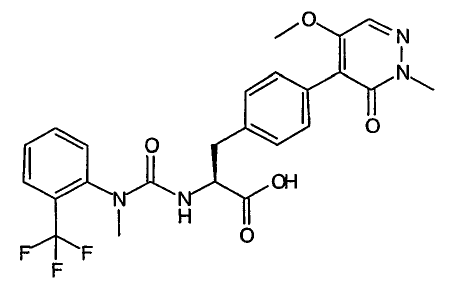 |
| 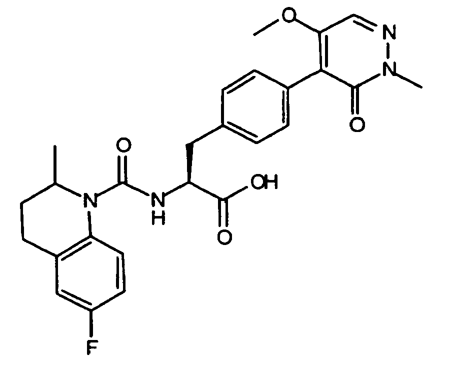 |

33. A composition comprising the compound of claim 1 and a pharmaceutically acceptable carrier.

34. A method of making a composition comprising admixing the compound of claim 1 and a pharmaceutically acceptable carrier.

35. The use of a compound according to claim 1 in the preparation of a medicament for treating or ameliorating an α4 integrin mediated disorder selected from the group consisting of multiple sclerosis, asthma, allergic rhinitis, allergic conjunctivitis, inflammatory lung diseases, rheumatoid arthritis, septic arthritis, type I diabetes, organ transplantation rejection, restenosis, autologous bone marrow transplantation, inflammatory sequelae of viral infections, myocarditis, inflammatory bowel disease, toxic and immune-based nephritis, contact dermal hypersensitivity, psoriasis, tumor metastasis, atherosclerosis and hepatitis in a subject in need thereof wherein said treatment comprises administering to the subject a therapeutically effective amount of the compound of claim 1.

36. The use of a compound according to claim 29 in the preparation of a medicament for treating or ameliorating an α4 integrin mediated disorder selected from the group consisting of multiple sclerosis, asthma, allergic rhinitis, allergic conjunctivitis, inflammatory lung diseases, rheumatoid arthritis, septic arthritis, type I diabetes, organ transplantation rejection, restenosis, autologous bone marrow transplantation, inflammatory sequelae of viral infections, myocarditis, inflammatory bowel disease, toxic and immune-based nephritis, contact dermal hypersensitivity, psoriasis, tumor metastasis, atherosclerosis and hepatitis in a subject in need thereof wherein said treatment comprises administering to the subject a therapeutically effective amount of the compound of claim 29.

37. The use of claims 35 and 36 wherein the inflammatory bowel disease is selected from the group consisting of including ulcerative colitis and Crohn's disease.

38. The use of claim 35 and 36 wherein the therapeutically effective amount of the compound of claim 1 is from about 0.001 mg/kg/day to about 1000 mg/kg/day.

39. A compound according to any one of claims 1 to 32 for use as a medicament.

## Patentansprüche

1. Verbindung von Formel (I) 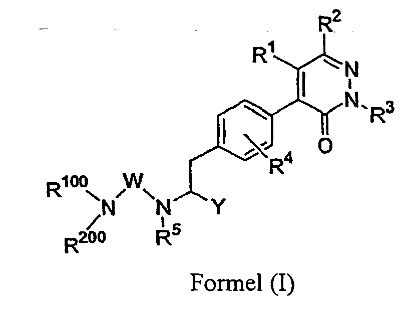 worin
R¹ unabhängig ausgewählt ist aus der Gruppe, bestehend aus Wasserstoff, C₁₋₆-Alkyl, C₁₋₆-Alkoxy, Aryl, Heteroaryl, Heterocyclyl, Benzo-kondensiertem Heterocyclyl, Benzo-kondensiertem Cycloalkyl, Heteroaryl-kondensiertem Heterocyclyl, Heteroaryl-kondensiertem Cycloalkyl, Aryloxy, Heteroaryloxy, Heterocyclyloxy, Cycloalkyloxy, -NR¹⁰R²⁰, Halogen, Hydroxy und -S(C₁₋₆)-Alkyl; wobei C₁₋₆-Alkoxy fakultativ mit einem bis vier Substituenten substituiert ist, die unabhängig ausgewählt sind aus R^{a}; wobei R^{a} unabhängig ausgewählt ist aus der Gruppe, bestehend aus Aryl, Heteroaryl, Heterocyclyl, Cycloalkyl, (C₁₋₆)-Alkoxycarbonyl, Carboxy, Amino, Alkylamino, Dialkylamino, Hydroxy-(C₁₋₆)-alkoxy, einem bis drei Halogenatomen und Hydroxy;
wobei R¹⁰ und R²⁰ unabhängig ausgewählt sind aus der Gruppe, bestehend aus Wasserstoff, C₁₋₆-Alkyl, Allyl, halogeniertem C₁₋₆-Alkyl, Hydroxy, Hydroxy-(C₁₋₄)-alkyl, Aryl-(C₁₋₄)-alkyl, Aryl und Cycloalkyl; zusätzlich R¹⁰ und R²⁰ fakultativ mit den Atomen zusammengenommen sind, an die sie gebunden sind, um einen fünf- bis siebengliedrigen monocyclischen Ring zu bilden;
wobei die Aryl- und Aryloxy-Substituenten von R¹ fakultativ mit einem Substituenten substituiert sind, der unabhängig ausgewählt ist aus der Gruppe, bestehend aus einem bis drei C₁₋₆-Alkyl-Substituenten, Hydroxy-(C₁₋₆)-alkyl, Aryl-(C₁₋₆)-alkyl, C₁₋₆-Alkoxy, Aryl, Heteroaryl, C₁₋₆-Alkoxycarbonyl, Aryl-(C₁₋₆)-alkoxycarbonyl, C₁₋₆-Alkylcarbonyl, Aminocarbonyl, Alkylaminocarbonyl, Dialkylaminocarbonyl, Hydroxy, Cyano, Nitro, -SO₂(C₁₋₃)-Alkyl, -SO₂-Aryl, -SO₂-Heteroaryl, Trifluormethyl, Trifluormethoxy und einem bis drei Halogenatomen;
und wobei die Heteroaryl- und Heterocyclyl-Substituenten von R¹ fakultativ mit einem bis drei Substituenten substituiert sind, die unabhängig ausgewählt sind aus der Gruppe, bestehend aus C₁₋₆-Alkyl, C₁₋₆-Alkoxy, Aryl, Heteroaryl, Halogen und Hydroxy;
zusätzlich R¹ und R² fakultativ mit den Atomen zusammengenommen sind, an die sie gebunden sind, um einen fünf- bis siebengliedrigen carbocyclischen oder heterocyclischen Ring zu bilden;
R² unabhängig ausgewählt aus der Gruppe, bestehend aus Wasserstoff, C₁₋₆-Alkyl, C₁₋₆-Alkoxy, C₂₋₆-Alkenyloxy, Hydroxy, Amino, Alkylamino, Dialkylamino und Halogen; wobei R¹ und R² fakultativ mit den Atomen zusammengenommen sind, an die sie gebunden sind, um einen fünf- bis siebengliedrigen carbocyclischen oder heterocyclischen Ring zu bilden;
R³ unabhängig ausgewählt ist aus der Gruppe, bestehend aus Wasserstoff, C₁₋₆-Alkyl, C₂₋₆-Alkenyl, C₂₋₆-Alkinyl, Aryl, Heteroaryl, Heterocyclyl und Cycloalkyl; wobei Alkyl, Alkenyl und Alkinyl fakultativ mit einem Substituenten substituiert sind, der unabhängig ausgewählt ist aus der Gruppe, bestehend aus Aminocarbonyl, Alkylaminocarbonyl, Dialkylaminocarbonyl, Aryl, Heteroaryl, Heterocyclyl, Cycloalkyl, Carboxy, einem bis drei Halogenatomen, Hydroxy und -C(=O)C₁₋₆-Alkyl;
R⁴ unabhängig ausgewählt ist aus der Gruppe, bestehend aus Wasserstoff, Fluor, Chlor und Methyl;
R⁵ Wasserstoff oder C₁₋₃-Alkyl ist, vorausgesetzt, daß R⁵ nur C₁₋₃-Alkyl ist, wenn zusammengenommen mit Y und den Atomen, an die R⁵ und Y gebunden sind, um einen fünf- bis siebengliedrigen Heterozyklus zu bilden;
Y unabhängig ausgewählt ist aus der Gruppe, bestehend aus Hydroxymethyl, -C(=O)NH₂, -C(=O)NH(OH), -C(=O)NH(C₁₋₆-Alkyl), -C(=O)NH(Hydroxy-(C₁₋₆)-alkyl), -C(=O)N(C₁₋₆-Alkyl)₂, -C(=O)NHSO₂(C₁₋₄)-Alkyl, Carboxy, Tetrazolyl und -C(=O)C₁₋₆-Alkoxy; wobei besagtes Alkoxy fakultativ mit einem bis zwei Substituenten substituiert ist, die unabhängig ausgewählt sind aus der Gruppe, bestehend aus Hydroxy, -NR³⁰R⁴⁰, Heterocyclyl, Heteroaryl, Halogen und -OCH₂CH₂OCH₃; wobei R³⁰ und R⁴⁰ unabhängig ausgewählt sind aus der Gruppe, bestehend aus Wasserstoff, C₁₋₆-Alkyl, Hydroxy und Hydroxy-(C₁₋₄)-alkyl, wobei besagte R³⁰ und R⁴⁰ fakultativ mit den Atomen zusammengenommen sind, an die sie gebunden sind, um einen fünf bis zehngliedrigen monocyclischen Ring zu bilden;
W unabhängig ausgewählt ist aus der Gruppe, bestehend aus -C(=O)- und -C(=S)-;
R¹⁰⁰ und R²⁰⁰ unabhängig ausgewählt sind aus der Gruppe, bestehend aus Wasserstoff, C₁₋₆-Alkyl, C₁₋₆-Alkenyl, C₁₋₆-Alkinyl, Aryl, Heteroaryl, Cycloalkyl, Polycycloalkyl, Heterocyclyl, Hydroxy und Arylamino;
wobei die Alkyl-, C₁₋₆-Alkenyl-, C₁₋₆-Alkinyl- und Alkoxy-Substituenten von R¹⁰⁰ und R²⁰⁰ fakultativ mit einem Substituenten substituiert sind, der unabhängig ausgewählt ist aus der Gruppe, bestehend aus Aryl, Heteroaryl, Heterocyclyl, Hydroxy, einem bis drei Halogenatomen, Amino, C₁₋₆-Alkylamino, Hydroxy-(C₁₋₆)-alkylamino, Di-(C₁₋₆)-alkylamino, -C(=O)Amino und -C(=O)C₁₋₆-Alkoxy;
wobei besagte Aryl-, Heteroaryl- und Heterocyclyl-Substituenten fakultativ mit bis zu vier Substituenten substituiert sein können, die ausgewählt sind aus der Gruppe, bestehend aus C₁₋₆-Alkyl, C₁₋₆-Alkoxy, Halogen, Aryl, Amino, Alkylamino, Dialkylamino, Hydroxy, Polycycloalkyl und Heteroaryl, zusätzliches besagtes Heterocyclyl fakultativ mit einem bis drei Oxo-Substituenten substituiert ist;
wobei besagtes Aryl, Heteroaryl und Heterocyclyl von R¹⁰⁰ und R²⁰⁰ fakultativ mit einem bis vier Substituenten substituiert sind, die unabhängig ausgewählt sind aus der Gruppe, bestehend aus C₁₋₆-Alkyl, Trifluoralkyl, C₁₋₆-Alkoxy, Trifluoralkoxy, Halogen, Aryl, Amino, Alkylamino, Dialkylamino, Arylamino, Hydroxy, Polycycloalkyl und Heteroaryl; zusätzlich Heterocyclyl fakultativ mit einem bis drei Oxo-Substituenten substituiert ist;
und wobei R¹⁰⁰ und R²⁰⁰ fakultativ mit dem Stickstoffatom zusammengenommen sind, an das sie beide gebunden sind, um einen fünf- bis zehngliedrigen Heterozyklus oder einen neun- bis zehngliedrigen Benzo-kondensierten Heterozyklus zu bilden; wobei der Heterozyklus fakultativ mit bis zu vier Substituenten substituiert ist, die unabhängig ausgewählt sind aus der Gruppe, bestehend aus C₁₋₆-Alkyl, C₁₋₆-Alkoxy, Halogen, Aryl, Heteroaryl, Amino, Alkylamino, Dialkylamino, Hydroxy, Polycycloalkyl oder und Oxo;
und ein optisches Isomer, Enantiomer, Diastereomer, Razemat oder pharmazeutisch annehmbares Salz davon.

2. Verbindungen nach Anspruch 1, **dadurch gekennzeichnet, daß** R¹ unabhängig ausgewählt ist aus der Gruppe, bestehend aus Wasserstoff, C₁₋₄-Alkyl, C₁₋₄-Alkoxy, Aryl, Heteroaryl, Heterocyclyl, Benzo-kondensiertem Cycloalkyl, Benzo-kondensiertem Heterocyclyl, Aryloxy, Heteroaryloxy, Heterocyclyloxy, Cycloalkyloxy, -NR¹⁰R²⁰, Halogen, Hydroxy und -S(C₁₋₆)-Alkyl; wobei C₁₋₄-Alkoxy von R¹ fakultativ mit einem bis drei Substituenten substituiert ist, die unabhängig ausgewählt sind aus R^{a};
wobei R^{a} unabhängig ausgewählt ist aus der Gruppe, bestehend aus Aryl, Heteroaryl, Heterocyclyl, Cycloalkyl, Dialkylamino, Hydroxy-(C₁₋₆)-alkoxy, einem bis drei Halogenatomen und Hydroxy;
wobei R¹⁰ und R²⁰ unabhängig ausgewählt sind aus der Gruppe, bestehend aus Wasserstoff, C₁₋₆-Alkyl, Allyl, halogeniertem C₁₋₆-Alkyl und Cycloalkyl; zusätzlich R¹⁰ und R²⁰ fakultativ mit den Atomen zusammengenommen sind, an die sie gebunden sind, um einen fünf- bis siebengliedrigen monocyclischen Ring zu bilden;
wobei die Aryl- und Aryloxy-Substituenten von R¹ fakultativ mit einem Substituenten substituiert sind, der unabhängig ausgewählt ist aus der Gruppe, bestehend aus C₁₋₆-Alkyl, C₁₋₆-Alkoxy, Phenyl, Heteroaryl, Aminocarbonyl, Alkylaminocarbonyl, Dialkylaminocarbonyl, Hydroxy, Cyano, Nitro, -SO₂(C₁₋₃)-Alkyl, -SO₂-Aryl, Trifluormethyl, Trifluormethoxy und Halogen;
und wobei die Heteroaryl- und Heterocyclyl-Substituenten von R¹ fakultativ mit einem bis drei Substituenten substituiert sind, die unabhängig ausgewählt sind aus der Gruppe, bestehend aus C₁₋₆-Alkyl, Halogen und Hydroxy;
zusätzlich R¹ und R² fakultativ mit den Atomen zusammengenommen sind, an die sie gebunden sind, um einen fünf bis siebengliedrigen carbocyclischen oder heterocyclischen Ring zu bilden.

3. Verbindungen nach Anspruch 1, **dadurch gekennzeichnet, daß** R¹ unabhängig ausgewählt ist aus der Gruppe, bestehend aus Wasserstoff, Ethyl, Methoxy, Ethoxy, 2-Hydroxyeth-1-oxy, Isopropoxy, Isobutoxy, Difluormethoxy, 2,2,2-Trifluoreth-1-oxy, Benzyloxy, Cyclopropylmethoxy, Pyridin-3-ylmethoxy, (1-Methyl)-pyrrolidinyl-3-oxy, Cyclobutyloxy, Cyclopentyloxy, Cyclohexyloxy, Indazol-1-yl, Thiophen-3-yl, [1,3]-Benzodioxol-5-yl, (2-Methyl)-imidazol-1-yl, (1-Methyl)-piperidin-4-yloxy, 2-(Morpholin-4-yl)-ethoxy, (4-Brom)-pyrazol-1-yl, N-Pyrrolidinyl, (3,5-Dimethyl)-pyrazol-1-yl, Morpholin-4-yl, Hydroxy, -(OCH₂CH₂)₂OH, Phenyl (fakultativ substituiert mit einem Substituenten, der unabhängig ausgewählt ist aus der Gruppe, bestehend aus -SO₂Me, -C(=O)NH₂, -OCF₃, -CF₃, Cyano, Fluor und Methoxy), Amino, Cyclopropylamino, Allylamino, Methylamino, Hydroxy, Chlor und -SMe;
und wobei R¹ fakultativ mit R² zusammengenommen ist, um ein 1,4-Dioxanyl oder ein Oxazinyl zu bilden.

4. Verbindungen nach Anspruch 1, **dadurch gekennzeichnet, daß** R¹ Methoxy ist.

5. Verbindungen nach Anspruch 1, **dadurch gekennzeichnet, daß** R² unabhängig ausgewählt ist aus der Gruppe, bestehend aus Wasserstoff, C₁₋₄-Alkyl, C₁₋₄-Alkoxy, C₂₋₄-Alkenyloxy, Hydroxy, Amino und Halogen; wobei R¹ und R² fakultativ mit den Atomen zusammengenommen sind, an die sie gebunden sind, um einen fünf- bis siebengliedrigen carbocyclischen oder heterocyclischen Ring zu bilden.

6. Verbindungen nach Anspruch 1, **dadurch gekennzeichnet, daß** R² unabhängig ausgewählt ist aus der Gruppe, bestehend aus Wasserstoff, C₁₋₄-Alkyl, C₁₋₄-Alkoxy, Hydroxy, Amino, Alkylamino und Halogen; wobei R² fakultativ mit R¹ zusammengenommen ist, um einen 1,4-Dioxanyl- oder einen Oxazinylring zu bilden.

7. Verbindungen nach Anspruch 1, **dadurch gekennzeichnet, daß** R² unabhängig ausgewählt ist aus der Gruppe, bestehend aus Wasserstoff, C₁₋₄-Alkoxy, Amino oder Alkylamino; wobei R² fakultativ mit R¹ zusammengenommen ist, um einen 1,4-Dioxanyl- oder einen Oxazinylring zu bilden.

8. Verbindungen nach Anspruch 1, **dadurch gekennzeichnet, daß** R³ unabhängig ausgewählt ist aus der Gruppe, bestehend aus Wasserstoff, C₁₋₆-Alkyl, Aryl, Heteroaryl, Heterocyclyl und Cycloalkyl; wobei C₁₋₆-Alkyl fakultativ mit einem Substituenten substituiert ist, der unabhängig ausgewählt ist aus der Gruppe, bestehend aus -C(=O)NH₂, Aryl, Heteroaryl, Heterocyclyl, Cycloalkyl, Carboxy, einem bis drei Halogenatomen, Hydroxy und -C(=O)C₁₋₆-Alkyl.

9. Verbindungen nach Anspruch 1, **dadurch gekennzeichnet, daß** R³ unabhängig ausgewählt ist aus der Gruppe, bestehend aus Wasserstoff, C₁₋₄-Alkyl, Cycloalkyl und Aryl; wobei C₁₋₄-Alkyl fakultativ mit einem Substituenten substituiert ist, der unabhängig ausgewählt ist aus der Gruppe, bestehend aus -C(=O)C₁₋₄-Alkyl, -C(=O)NH₂, Carboxy, Heterocyclyl, Phenyl, Cyclopropyl, Hydroxy und einem bis drei Fluoratomen.

10. Verbindungen nach Anspruch 1, **dadurch gekennzeichnet, daß** R³ unabhängig ausgewählt ist aus der Gruppe, bestehend aus Wasserstoff, C₁₋₄-Alkyl und Phenyl; wobei C₁₋₄-Alkyl fakultativ mit einem Substituenten substituiert ist, der unabhängig ausgewählt ist aus der Gruppe, bestehend aus -C(=O)C₁₋₄-Alkyl, -C(=O)NH₂, Carboxy, Morpholinyl, Cyclopropyl, Hydroxy und einem bis drei Fluoratomen.

11. Verbindungen nach Anspruch 1, **dadurch gekennzeichnet, daß** R³ unabhängig ausgewählt ist aus der Gruppe, bestehend aus Wasserstoff, Methyl, Ethyl und Phenyl; wobei Methyl und Ethyl fakultativ mit einem Substituenten substituiert sind, der unabhängig ausgewählt ist aus der Gruppe, bestehend aus -C(=O)C₁₋₄Alkyl, -C(=O)NH₂, Carboxy, Morpholinyl, Cyclopropyl, Hydroxy und einem bis drei Fluoratomen.

12. Verbindungen nach Anspruch 1, **dadurch gekennzeichnet, daß** R³ unabhängig ausgewählt ist aus der Gruppe, bestehend aus Wasserstoff und 2-Hydroxyeth-1-yl.

13. Verbindungen nach Anspruch 1, **dadurch gekennzeichnet, daß** R⁴ unabhängig ausgewählt ist aus der Gruppe, bestehend aus Wasserstoff, Fluor und Chlor.

14. Verbindungen nach Anspruch 1, **dadurch gekennzeichnet, daß** R⁴ unabhängig ausgewählt ist aus der Gruppe, bestehend aus Wasserstoff und Fluor.

15. Verbindungen nach Anspruch 1, **dadurch gekennzeichnet, daß** R⁴ Wasserstoff ist.

16. Verbindungen nach Anspruch 1, **dadurch gekennzeichnet, daß** R⁵ Wasserstoff oder C₁₋₃-Alkyl ist, vorausgesetzt, daß R⁵ nur C₁₋₃-Alkyl ist, wenn zusammengenommen mit Y und den Atomen, an die R⁵ und Y gebunden sind, um einen fünf- bis siebengliedrigen Heterozyklus zu bilden.

17. Verbindungen nach Anspruch 1, **dadurch gekennzeichnet, daß** R⁵ Wasserstoff oder Methylen ist, vorausgesetzt, daß R⁵ nur Methylen ist, wenn zusammengenommen mit Y und den Atomen, an die R⁵ und Y gebunden sind, um einen fünfgliedrigen Heterozyklus zu bilden.

18. Verbindungen nach Anspruch 1, **dadurch gekennzeichnet, daß** R⁵ Wasserstoff ist.

19. Verbindungen nach Anspruch 1, **dadurch gekennzeichnet, daß** Y unabhängig ausgewählt ist aus der Gruppe, bestehend aus Hydroxymethyl, -C(=O)NH₂, -C(=O)NH(OH), -C(=O)NH(2-Hydroxyeth-1-yl), Carboxy, Tetrazolyl, -C(=O)NHSO₂(C₁₋₄)-alkyl und -C(=O)C₁₋₆-Alkoxy; wobei besagtes Alkoxy fakultativ mit einem bis zwei Substituenten substituiert ist, die unabhängig ausgewählt sind aus der Gruppe, bestehend aus Hydroxy, -NR³⁰R⁴⁰, Heterocyclyl, Heteroaryl, Halogen und -OCH₂CH₂OCH₃; wobei R³⁰ und R⁴⁰ unabhängig ausgewählt sind aus der Gruppe, bestehend aus Wasserstoff und C₁₋₆-Alkyl.

20. Verbindungen nach Anspruch 1, **dadurch gekennzeichnet, daß** Y unabhängig ausgewählt ist aus der Gruppe, bestehend aus Carboxy, Tetrazolyl, -C(=O)NH(2-Hydroxyeth-1-yl) und -C(=O)C₁₋₄-Alkoxy; wobei besagtes Alkoxy fakultativ mit einem bis zwei Substituenten substituiert ist, die unabhängig ausgewählt sind aus der Gruppe, bestehend aus Hydroxy, -NH₂, -NH(C₁₋₄)-Alkyl, -N(C₁₋₄-Alkyl)₂, Heterocyclyl, Halogen und -OCH₂CH₂OCH₃.

21. Verbindungen nach Anspruch 1, **dadurch gekennzeichnet, daß** Y unabhängig ausgewählt ist aus der Gruppe, bestehend aus Carboxy, 1H-Tetrazol-5-yl und -C(=O)C₁₋₄-Alkoxy; wobei besagtes Alkoxy fakultativ mit einem Substituenten substituiert ist, der unabhängig ausgewählt ist aus der Gruppe, bestehend aus Hydroxy, -NMe₂, Morpholin-1-yl, Chlor und -OCH₂CH₂OCH₃.

22. Verbindungen nach Anspruch 1, **dadurch gekennzeichnet, daß** Y unabhängig ausgewählt ist aus der Gruppe, bestehend aus Carboxy, 1H-Tetrazol-5-yl und -C(=O)Ethoxy; wobei besagtes Ethoxy fakultativ mit einem Substituenten substituiert ist, der unabhängig ausgewählt ist aus der Gruppe, bestehend aus Hydroxy, Chlor, -NMe₂ und -OCH₂CH₂OCH₃.

23. Verbindungen nach Anspruch 1, **dadurch gekennzeichnet, daß** W unabhängig ausgewählt ist aus der Gruppe, bestehend aus -C(=O)- und -C(=S)-.

24. Verbindungen nach Anspruch 1, **dadurch gekennzeichnet, daß** W -C(=O)- ist.

25. Verbindungen nach Anspruch 1, **dadurch gekennzeichnet, daß** R¹⁰⁰ und R²⁰⁰ Substituenten sind, die unabhängig ausgewählt sind aus der Gruppe, bestehend aus Wasserstoff, C₁₋₄-Alkyl, C₁₋₄-Alkinyl, Aryl, Heteroaryl, Cycloalkyl, Polycycloalkyl, Heterocyclyl, Phenylamino und Hydroxy; wobei besagte Aryl, Heteroaryl und Heterocyclyl fakultativ mit bis zu vier Substituenten substituiert sein können, die unabhängig ausgewählt sind aus der Gruppe, bestehend aus C₁₋₆-Alkyl, Hydroxy-(C₁₋₄)-alkyl, Halogen, Aryl, Trifluormethyl, Trifluormethoxy, Heteroaryl und Hydroxy;
wobei C₁₋₄-Alkyl und C₁₋₄-Alkinyl von R¹⁰⁰ und R²⁰⁰ fakultativ mit einem Substituenten substituiert sind, der unabhängig ausgewählt ist aus der Gruppe, bestehend aus Heteroaryl, Aryl, Hydroxy, einem bis drei Halogenatomen, Amino, Alkylamino und Dialkylamino;
wobei R¹⁰⁰ und R²⁰⁰ fakultativ mit dem Stickstoffatom zusammengenommen sind, an das sie beide gebunden sind, um einen fünf- bis siebengliedrigen Heterozyklus oder neun- bis zehngliedrigen Benzo-kondensierten Heterozyklus zu bilden; fakultativ substituiert mit bis zu vier Substituenten, die ausgewählt sind aus C₁₋₆-Alkyl, Halogen, Aryl und Heteroaryl.

26. Verbindungen nach Anspruch 1, **dadurch gekennzeichnet, daß** R¹⁰⁰ und _{R}²⁰⁰ unabhängig ausgewählt sind aus der Gruppe, bestehend aus Wasserstoff, C₁₋₄-Alkyl, Aryl, Cycloalkyl und Heterocyclyl; wobei besagte Aryl und Heterocyclyl fakultativ mit bis zu vier Substituenten substituiert sind, die unabhängig ausgewählt sind aus der Gruppe, bestehend aus C₁₋₃-Alkyl, Halogen, Trifluormethyl, Trifluormethoxy, Heteroaryl und Hydroxy;
wobei R¹⁰⁰ und R²⁰⁰ fakultativ mit dem Stickstoffatom zusammengenommen sind, an das sie beide gebunden sind, um einen fünf- bis siebengliedrigen Heterozyklus zu bilden, der fakultativ mit bis zu vier Substituenten substituiert ist, die unabhängig ausgewählt sind aus der Gruppe, bestehend aus C₁₋₆-Alkyl, Halogen, Aryl und Heteroaryl.

27. Verbindungen nach Anspruch 1, **dadurch gekennzeichnet, daß** R¹⁰⁰ und R²⁰⁰ unabhängig ausgewählt sind aus der Gruppe, bestehend aus Wasserstoff, C₁₋₄-Alkyl, Aryl, Cycloalkyl und einem fünf- bis siebengliedrigen Heterocyclyl, wobei besagtes Heterocyclyl gebildet wird, wenn R¹⁰⁰ und R²⁰⁰ mit dem Stickstoffatom zusammengenommen sind, an das sie beide gebunden sind;
wobei besagte Aryl- und Heterocyclyl fakultativ mit bis zu vier Substituenten substituiert sind, die unabhängig ausgewählt sind aus der Gruppe, bestehend aus C₁₋₃-Alkyl, Halogen, Trifluormethyl, Trifluormethoxy und Hydroxy.

28. Verbindungen nach Anspruch 1, **dadurch gekennzeichnet, daß** R¹⁰⁰ und R²⁰⁰ Substituenten sind, die unabhängig ausgewählt sind aus der Gruppe, bestehend aus Wasserstoff, Methyl, Ethyl, i-But-1-yl, Cyclohexyl, 2-Methylpiperidin-1-yl und Phenyl; wobei besagtes Phenyl mit einem bis zwei Substituenten substituiert ist, die unabhängig ausgewählt sind aus der Gruppe, bestehend aus Methyl, Chlor, Hydroxy, Trifluormethyl und Trifluormethoxy.

29. Verbindung von Formel (Ia) nach Anspruch 1 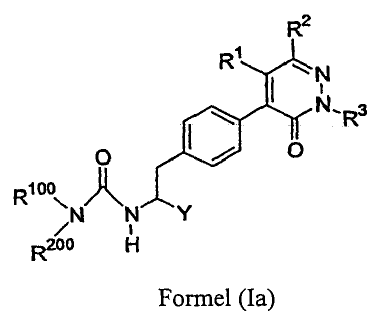 worin:
R¹ unabhängig ausgewählt ist aus der Gruppe, bestehend aus Wasserstoff, C₁₋₄-Alkyl, C₁₋₄-Alkoxy, Aryl, Heteroaryl, Heterocyclyl, Benzo-kondensiertem Cycloalkyl, Benzo-kondensiertem Heterocyclyl, Aryloxy, Heteroaryloxy, Heterocyclyloxy, Cycloalkyloxy, -NR¹⁰R²⁰, Halogen, Hydroxy und -S(C₁₋₆)-Alkyl; wobei C₁₋₄-Alkoxy von R¹ fakultativ mit einem bis drei Substituenten substituiert ist, die unabhängig ausgewählt sind aus R^{a};
wobei R^{a} unabhängig ausgewählt ist aus der Gruppe, bestehend aus Aryl, Heteroaryl, Heterocyclyl, Cycloalkyl, Dialkylamino, Hydroxy-(C₁₋₆)-alkoxy, einem bis drei Halogenatomen und Hydroxy;
wobei R¹⁰ und R²⁰ unabhängig ausgewählt sind aus der Gruppe, bestehend aus Wasserstoff, C₁₋₆-Alkyl, Allyl, halogeniertem C₁₋₆-Alkyl und Cycloalkyl; zusätzlich R¹⁰ und R²⁰ fakultativ mit den Atomen zusammengenommen sind, an die sie gebunden sind, um einen fünf- bis siebengliedrigen monocyclischen Ring zu bilden;
wobei die Aryl- und Aryloxy-Substituenten von R¹ fakultativ mit einem Substituenten substituiert sind, der unabhängig ausgewählt ist aus der Gruppe, bestehend aus C₁₋₆-Alkyl, C₁₋₆-Alkoxy, Phenyl, Heteroaryl, Aminocarbonyl, Alkylaminocarbonyl, Dialkylaminocarbonyl, Hydroxy, Cyano, Nitro, -SO₂(C₁₋₃)-Alkyl, -SO₂-Aryl, Trifluormethyl, Trifluormethoxy und Halogen;
und wobei die Heteroaryl- und Heterocyclyl-Substituenten von R¹ fakultativ mit einem bis drei Substituenten substituiert sind, die unabhängig ausgewählt sind aus der Gruppe, bestehend aus C₁₋₆-Alkyl, Halogen und Hydroxy;
zusätzlich R¹ und R² fakultativ mit den Atomen zusammengenommen sind, an die sie gebunden sind, um einen fünf- bis siebengliedrigen carbocyclischen oder heterocyclischen Ring zu bilden;
R² unabhängig ausgewählt ist aus der Gruppe, bestehend aus Wasserstoff, C₁₋₄-Alkyl, C₁₋₄-Alkoxy, C₂₋₄-Alkenyloxy, Hydroxy, Amino und Halogen; wobei R¹ und R² fakultativ mit den Atomen zusammengenommen sind, an die sie gebunden sind, um einen fünf- bis siebengliedrigen carbocyclischen oder heterocyclischen Ring zu bilden;
R³ unabhängig ausgewählt ist aus der Gruppe, bestehend aus Wasserstoff, C₁₋₄-Alkyl, Cycloalkyl und Aryl; wobei C₁₋₄-Alkyl fakultativ mit einem Substituenten substituiert ist, der unabhängig ausgewählt ist aus der Gruppe, bestehend aus -C(=O)C₁₋₄-Alkyl, -C(=O)NH₂, Carboxy, Heterocyclyl, Phenyl, Cyclopropyl, Hydroxy und einem bis drei Fluoratomen;
Y unabhängig ausgewählt ist aus der Gruppe, bestehend aus Carboxy, Tetrazolyl, -C(=O)NH(2-Hydroxyeth-1-yl) und -C(=O)C₁₋₄-Alkoxy; wobei besagtes Alkoxy fakultativ mit einem bis zwei Substituenten substituiert ist, die unabhängig ausgewählt sind aus der Gruppe, bestehend aus Hydroxy, -NH₂, -NH(C₁₋₄)-Alkyl, -N(C₁₋₄-Alkyl)₂, Heterocyclyl, Halogen und -OCH₂CH₂OCH₃;
R¹⁰⁰ und R²⁰⁰ unabhängig ausgewählt sind aus der Gruppe, bestehend aus Wasserstoff, C₁₋₄-Alkyl, Aryl, Cycloalkyl und Heterocyclyl; wobei besagte Aryl und Heterocyclyl fakultativ mit bis zu vier Substituenten substituiert sind, die unabhängig ausgewählt sind aus der Gruppe, bestehend aus C₁₋₃-Alkyl, Halogen, Trifluormethyl, Trifluormethoxy, Heteroaryl und Hydroxy;
wobei R¹⁰⁰ und R²⁰⁰ fakultativ mit dem Stickstoffatom zusammengenommen sind, an das sie beide gebunden sind, um einen fünf- bis siebengliedrigen Heterozyklus zu bilden, der fakultativ mit bis zu vier Substituenten substituiert ist, die unabhängig ausgewählt sind aus der Gruppe, bestehend aus C₁₋₆-Alkyl, Halogen, Aryl und Heteroaryl;
und ein optisches Isomer, Enantiomer, Diastereomer, Razemat oder pharmazeutisch annehmbares Salz davon.

30. Verbindung von Formel (Ia) nach Anspruch 1 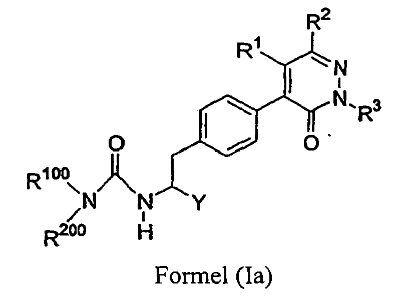 worin:
R¹ unabhängig ausgewählt ist aus der Gruppe, bestehend aus Wasserstoff, Ethyl, Methoxy, Ethoxy, 2-Hydroxyeth-1-oxy, Isopropoxy, Isobutoxy, Difluormethoxy, 2,2,2-Trifluoreth-1-oxy, Benzyloxy, Cyclopropylmethoxy, Pyridin-3-ylmethoxy, (1-Methyl)-pyrrolidinyl-3-oxy, Cyclobutyloxy, Cyclopentyloxy, Cyclohexyloxy, Indazol-1-yl, Thiophen-3-yl, [1,3]-Benzodioxol-5-yl, (2-Methyl)-imidazol-1-yl, (1-Methyl)-piperidin-4-yloxy, 2-(Morpholin-4-yl)-ethoxy, (4-Brom)-pyrazol-1-yl, N-Pyrrolidinyl, (3,5-Dimethyl)-pyrazol-1-yl, Morpholin-4-yl, Hydroxy, -(OCH₂CH₂)₂OH, Phenyl (fakultativ substituiert mit einem Substituenten, der unabhängig ausgewählt ist aus der Gruppe, bestehend aus -SO₂Me, -C(=O)NH₂, -OCF₃, -CF₃, Cyano, Fluor und Methoxy), Amino, Cyclopropylamino, Allylamino, Methylamino, Hydroxy, Chlor und -SMe;
und wobei R¹ fakultativ mit R² zusammengenommen ist, um einen 1,4-Dioxanyl- oder einen Oxazinylring zu bilden;
R² unabhängig ausgewählt ist aus der Gruppe, bestehend aus Wasserstoff, C₁₋₄-Alkyl, C₁₋₄-Alkoxy, Hydroxy, Amino, Alkylamino und Halogen; wobei R² fakultativ mit R¹ zusammengenommen ist, um einen 1,4-Dioxanyl- oder einen Oxazinylring zu bilden;
R³ unabhängig ausgewählt ist aus der Gruppe, bestehend aus Wasserstoff, C₁₋₄-Alkyl und Phenyl; wobei C₁₋₄-Alkyl fakultativ mit einem Substituenten substituiert ist, der unabhängig ausgewählt ist aus der Gruppe, bestehend aus -C(=O)C₁₋₄-Alkyl, -C(=O)NH₂, Carboxy, Morpholinyl, Cyclopropyl, Hydroxy und einem bis drei Fluoratomen;
Y unabhängig ausgewählt ist aus der Gruppe, bestehend aus Carboxy, 1H-Tetrazol-5-yl und -C(=O)C₁₋₄-Alkoxy; wobei besagtes Alkoxy fakultativ mit einem Substituenten substituiert ist, der unabhängig ausgewählt ist aus der Gruppe, bestehend aus Hydroxy, -NMe₂, Morpholin-1-yl, Chlor und -OCH₂CH₂OCH₃;
R¹⁰⁰ und R²⁰⁰ unabhängig ausgewählt sind aus der Gruppe, bestehend aus Wasserstoff, C₁₋₄-Alkyl, Aryl, Cycloalkyl und einem fünf- bis siebengliedrigen Heterocyclyl, wobei besagtes Heterocyclyl aus R¹⁰⁰ und R²⁰⁰ gebildet wird, die mit dem Stickstoffatom zusammengenommen sind, an das sie beide gebunden sind;
wobei besagte Aryl und Heterocyclyl fakultativ mit bis zu vier Substituenten substituiert sind, die unabhängig ausgewählt sind aus der Gruppe, bestehend aus C₁₋₃-Alkyl, Halogen, Trifluormethyl, Trifluormethoxy und Hydroxy;
und ein optisches Isomer, Enantiomer, Diastereomer, Razemat oder pharmazeutisch annehmbares Salz davon.

31. Verbindung von Formel (Ib) nach Anspruch 1 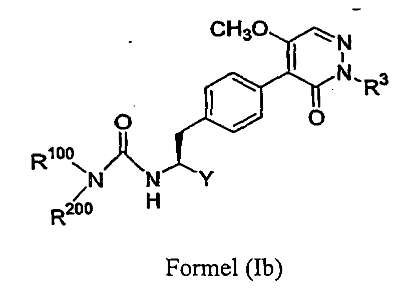 worin Y, R³, R¹⁰⁰ und R²⁰⁰ abhängig ausgewählt sind aus der Gruppe, bestehend aus:
(d bezeichnet eine Diastereomerenmischung)
| R³ | Y | R¹⁰⁰ | R²⁰⁰ | Stereochemie von R¹⁰⁰ |
|---|---|---|---|---|
| CH₃ | CO₂H | i-Propyl | i-Propyl | |
| (2-OH)-Eth-1-yl | CO₂H | (2,6-Cl₂)Phenyl | H | |
| (2-OH)-Eth-1-yl | CO₂H | Cyclohexyl | H | |
| (2-OH)-Eth-1-yl | CO₂H | (2-F)Phenyl | H | |
| CH₃ | CO₂H | (4-Me)Piperazin-1-yl | | |
| CH₃ | CO₂H | (3-OH)Pyrrolidin-1-yl | | d |
| CH₃ | CO₂H | (2-OH)-Eth-1-yl | H | |
| CH₃ | CO₂H | (2-Hydroxymethyl)-pyrrolidin-1-yl | H | S |
| CH₃ | CO₂H | Benzyl | H | |
| CH₃ | CO₂H | Phenyl | Methyl | |
| CH₃ | CO₂H | Cyclohexyl | Methyl | |
| CH₃ | CO₂H | Benzyl | Methyl | |
| CH₃ | CO₂H | Methyl | i-Butyl | |
| CH₃ | CO₂H | (2-OH)-Eth-1-yl | Methyl | |
| CH₃ | CO₂H | Benzyl | (2-OH)-Eth-1-yl | |
| CH₃ | CO₂H | Phenyl | (2-OH)-Eth-1-yl | |
| CH₃ | CO₂H | Cyclohexyl | (2-OH)-Eth-1-yl | |
| CH₃ | CO₂H | i-Butyl | Benzyl | |
| CH₃ | CO₂H | Methyl | Methyl | |
| CH₃ | CO₂H | Cyclohexyl | H | |
| CH₃ | 2-OH-Ethoxycarbonyl | i-Butyl | Methyl | |
| CH₃ | CO₂H | 1-Methylpiperidin-4-yl | Cyclopropyl | |
| CH₃ | CO₂H | Norbornan-2-yl | H | S |
| CH₃ | CO₂H | 4-(3-Chlor-5-trifluormethylpyridin-2-yl)-piperazin-1-yl | | |
| CH₃ | CO₂H | 4-(Adamantan-2-yl)-piperazin-1-yl | | |
| CH₃ | CO₂H | Cyclohexyl | Hydroxy | |
| CH₃ | CO₂H | Benzyl | i-Propyl | |
| CH₃ | CO₂H | 2-Methylpiperidin-1-yl | | d |
| CH₃ | CO₂H | (1-Phenyl)- pyrazol-4-ylmethyl | Methyl | |
| CH₃ | CO₂H | 4-(Benzooxazol-2-yl)-[1,4]diazepan-1-yl | | |
| CH₃ | CO₂H | Furan-2-ylmethyl | But-2-in-1-yl | |
| CH₃ | CO₂H | 10-Methoxy-3,4,5,6-tetrahydro-2H-benzo [b] [1,5]oxacinyl | | |
| CH₃ | CO₂H | Cyclohexyl | i-Propyl | |
| CH₃ | CO₂H | 4-(2-Hydroxyeth-1-yl)-piperazin-1-ylamino | H | |
| CH₃ | CO₂H | 1- Benzylpyrrolidin-3-yl | Methyl | |
| CH₃ | CO₂H | (4-Hydroxy-1,1-dioxotetrahydro-1λ⁶-thiophenyl-3-yl | H | 4S,3S |
| CH₃ | CO₂H | 2- (Dimethylamino)-eth-1-yl | Ethyl | |
| CH₃ | CO₂H | 2-(Phenylaminomethyl)-pyrrolidin-1-yl | | |
| CH₃ | CO₂H | 2-(Pyrrolidin-1-ylmethyl)- pyrrolidin-1-yl | | S |
| CH₃ | CO₂H | 1-Azabicyclo- [2.2.2]oct-3-yl | Pyridin-3-lmethyl | d |
| CH₃ | CO₂H | Phenyl | i-Butyl | |
| CH₃ | CO₂H | Phenyl | i-Propyl | |
| CH₃ | CO₂H | 4-(i-Propylamino)-phenyl | i-Propyl | |
| CH₃ | CO₂H | (2-Methyl-5-hydroxy)phenyl | Ethyl | |
| CH₃ | CO₂H | Naphth-1-yl | Ethyl | |
| CH₃ | CO₂H | Phenyl | Phenylamino | |
| CH₃ | CO₂H | Phenyl | Phenyl | |
| CH₃ | CO₂H | Benzyl | Phenyl | |
| CH₃ | CO₂H | (2-Methyl)-indolin-1-yl | | d |
| CH₃ | CO₂H | Phenyl | n-Propyl | |
| CH₃ | CO₂H | (2- Trifluormethoxy)-phenyl | Methyl | |
| CH₃ | CO₂H | (2- Trifluormethyl)-phenyl | Methyl | |
| CH₃ | CO₂H | 6-Fluor-2-methyl-3,4-dihydro-2H- chinolin-1-yl | | d |
| CH₃ | CO₂H | Pyridin-2-yl | Methyl | |
| und | | | | |
| CH₃ | -C(=O)O- (CH₂)₂OH | s-But-1-yl | Methyl | |

32. Verbindung nach Anspruch 1, ausgewählt aus der Gruppe, bestehend aus
| |
|---|
| 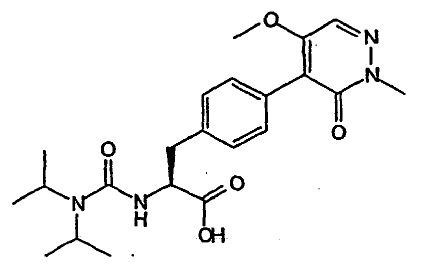 |
| 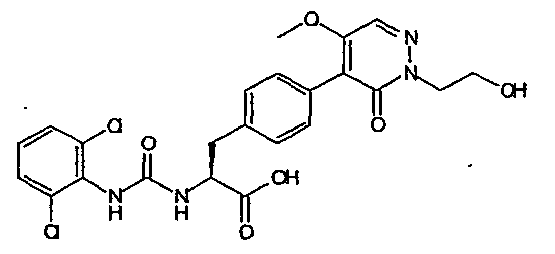 |
| 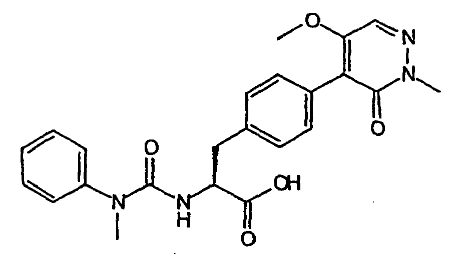 |
| 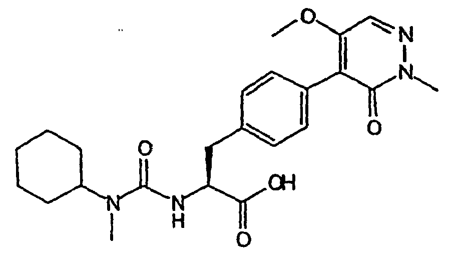 |
| 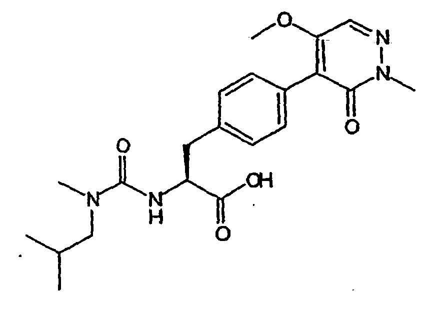 |
| 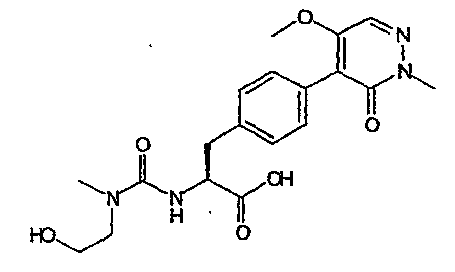 |
| 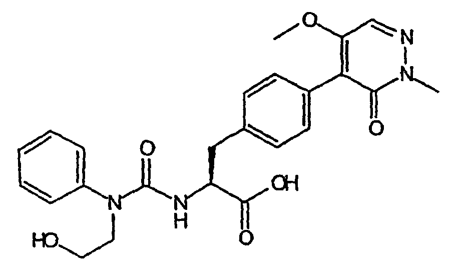 |
| 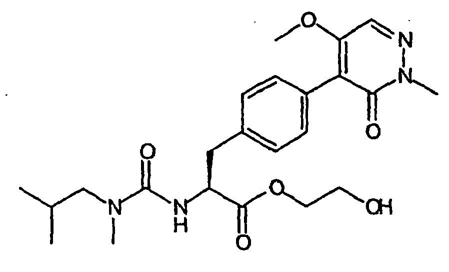 |
| 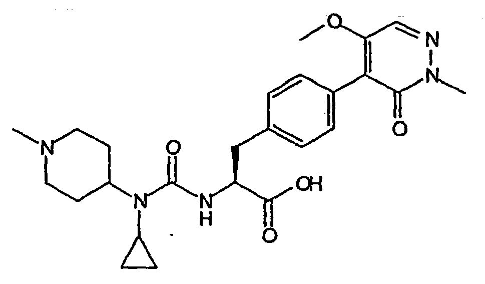 |
| 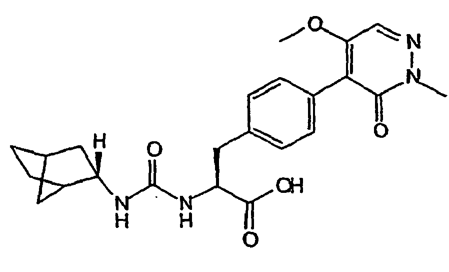 |
| 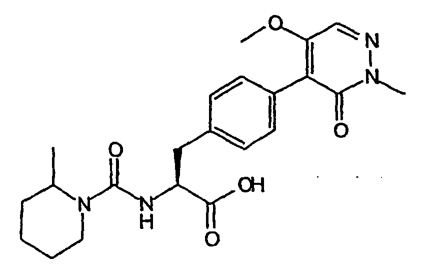 |
| 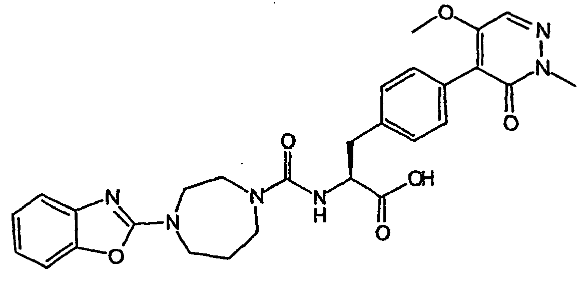 |
| 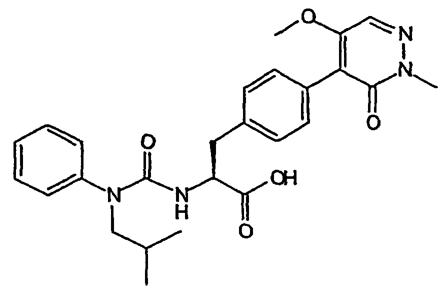 |
| 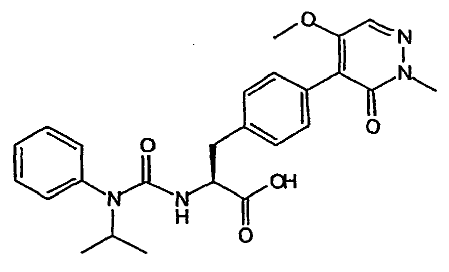 |
| 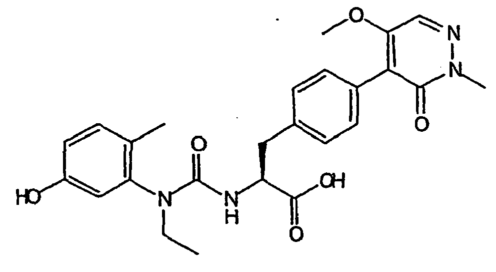 |
| 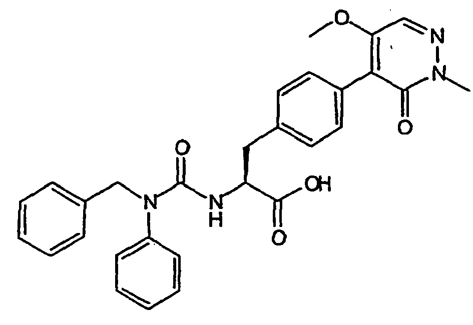 |
| 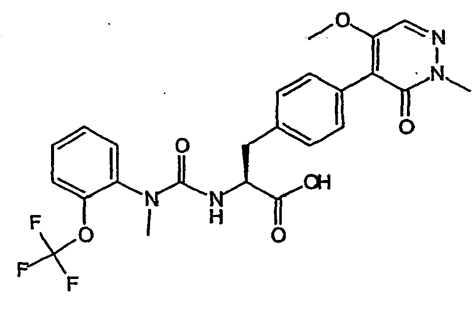 |
| 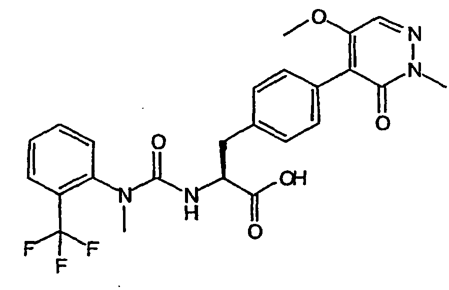 |
| 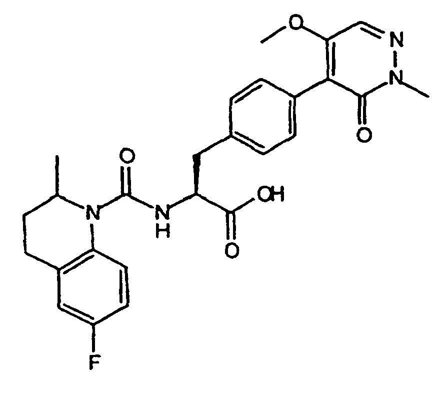 |

33. Zusammensetzung, die die Verbindung nach Anspruch 1 und einen pharmazeutisch annehmbaren Trägerstoff umfaßt.

34. Verfahren zur Herstellung einer Zusammensetzung, welches das Vermischen der Verbindung nach Anspruch 1 und eines pharmazeutisch annehmbaren Trägerstoffes umfaßt.

35. Verwendung einer Verbindung nach Anspruch 1 zur Herstellung eines Arzneimittels zur Behandlung oder Linderung einer α4-Integrin-vermittelten Störung, ausgewählt aus der Gruppe, bestehend aus multipler Sklerose, Asthma, allergischer Rhinitis, allergischer Konjunktivitis, entzündlicher Lungenerkrankungen, rheumatoider Arthritis, septischer Arthritis, Diabetes Typ I, Organtransplantationsabstoßung, Restenose, autologer Knochenmarktransplantation, entzündlicher Folgeerkrankungen viraler Infektionen, Myokarditis, entzündlicher Darmerkrankung, toxischer und immunbasierter Nephritis, Kontakthautüberempfindlichkeit, Psoriasis, Tumormetastase, Atherosklerose und Hepatitis, bei einem Patienten, der derselben bedarf, wobei besagte Behandlung die Verabreichung einer therapeutisch wirksamen Menge der Verbindung nach Anspruch 1 an den Patienten umfaßt.

36. Verwendung einer Verbindung nach Anspruch 29 zur Herstellung eines Arzneimittels zur Behandlung oder Linderung einer α4-Integrin-vermittelten Störung, ausgewählt aus der Gruppe, bestehend aus multipler Sklerose, Asthma, allergischer Rhinitis, allergischer Konjunktivitis, entzündlicher Lungenerkrankungen, rheumatoider Arthritis, septischer Arthritis, Diabetes Typ I, Organtransplantationsabstoßung, Restenose, autologer Knochenmarktransplantation, entzündlicher Folgeerkrankungen viraler Infektionen, Myokarditis, entzündlicher Darmerkrankung, toxischer und immunbasierter Nephritis, Kontakthautüberempfindlichkeit, Psoriasis, Tumormetastase, Atherosklerose und Hepatitis, bei einem Patienten, der derselben bedarf, wobei besagte Behandlung die Verabreichung einer therapeutisch wirksamen Menge der Verbindung nach Anspruch 29 an den Patienten umfaßt.

37. Verwendung nach Anspruch 35 und 36, **dadurch gekennzeichnet, daß** die entzündliche Darmerkrankung ausgewählt ist aus der Gruppe, bestehend aus einschließlich Colitis ulcerosa und Crohn-Krankheit.

38. Verwendung nach Anspruch 35 und 36, **dadurch gekennzeichnet, daß** die therapeutisch wirksame Menge der Verbindung nach Anspruch 1 von etwa 0,001 mg/kg/Tag bis etwa 1.000 mg/kg/Tag liegt.

39. Verbindung nach einem der Ansprüche 1 bis 32 zur Verwendung als ein Arzneimittel.

## Revendications

1. Composé de Formule (I) 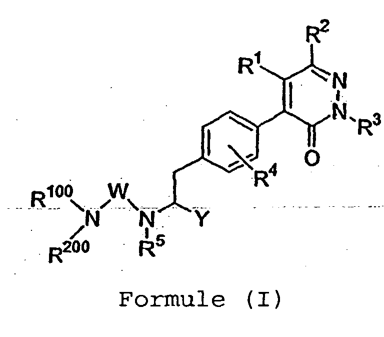 où
R¹ est indépendamment sélectionné dans le groupe consistant en hydrogène, alkyleC₁₋₆, alcoxyC₁₋₆, aryle, hétéroaryle, hérérocyclyle, hétérocyclyle benzo fusionné, cycloalkyle benzo fusionné, hétérocyclyle hétéroaryle fusionné, cycloalkyle hétéroaryle fusionné, aryloxy, hétéroaryloxy, hétérocyclyloxy, cycloalkyloxy, -NR¹⁰R²⁰, halogène, hydroxy, et -Salkyle (C₁₋₆); où alcoxyC₁₋₆ est facultativement substitué par un à quatre substituants indépendamment sélectionnés parmi R^{a};
où R^{a} est indépendamment sélectionné dans le groupe consistant en aryle, hétéroaryle, hétérocyclyle, cycloalkyle, alcoxycarbonyle(C₁₋₆), carboxy, amino, alkylamino, dialkylamino, hydroxyalcoxy(C₁₋₆), un à trois atomes d'halogène, et hydroxy;
où R¹⁰ et R²⁰ sont indépendamment sélectionnés dans le groupe consistant en hydrogène, alkyleC₁₋₆, allyle, alkyleC₁₋₆ halogéné, hydroxy, hydroxyalkyle(C₁₋₄), arylalkyle(C₁₋₄), aryle et cycloalkyle; additionnellement R¹⁰ et R²⁰ sont facultativement pris avec les atomes auquel ils sont attachés pour former un cycle monocyclique à cinq à sept membres;
où les substituants aryle et aryloxy de R¹ sont facultativement substitués par un substituant indépendamment sélectionnés dans le groupe consistant en un à trois substituants alkyleC₁₋₆, hydroxyalkyle(C₁₋₆), arylalkyle(C₁₋₆), alcoxyC₁₋₆, aryle, hétéroaryle, alcoxycarbonyleC₁₋₆, arylalcoxycarbonyl(C₁₋₆), alkyl-carbonyleC₁₋₆, aminocarbonyle, alkylaminocarbonyle, dialkylaminocarbonyle, hydroxy, cyano, nitro, -SO₂alkyle(C₁₋₃), -SO₂aryle, -SO₂hétéroaryle, trifluorométhyle, trifluorométhoxy, et un à trois atomes d'halogène;
et où les substituants hétéroaryle et hétérocyclyle de R¹ sont facultativement substitués par un à trois substituants indépendamment sélectionnés dans le groupe consistant en alkyleC₁₋₆, alcoxyC₁₋₆, aryle, hétéroaryle, halogène, et hydroxy;
additionnellement, R¹ et R² sont facultativement pris avec les atomes auquel ils sont attachés pour former un cycle carbocyclique ou hétérocyclique à cinq à sept membres;
R² est indépendamment sélectionné dans le groupe consistant en hydrogène, alkyleC₁₋₆, alcoxyC₁₋₆, alcényloxyC₂₋₆, hydroxy, amino, alkylamino, dialkylamino, et halogène; où R¹ et R² sont facultativement pris avec les atomes auquel ils sont attachés pour former un cycle carbocyclique ou hétérocyclique à cinq à sept membres;
R³ est indépendamment sélectionné dans le groupe consistant en hydrogène, alkyleC₁₋₆, alcényleC₂₋₆, alkynyleC₂₋₆, aryle, hétéroaryle, hétérocyclyle, et cycloalkyle; où alkyle, alcényle, et alkynyle sont facultativement substitués par un substituant indépendamment sélectionné dans le groupe consistant en aminocarbonyle, alkylaminocarbonyle, dialkylaminocarbonyle, aryle, hétéroaryle, hétérocyclyle, cycloalkyle, carboxy, un à trois atomes d'halogène, hydroxy, et -C(=O)alkyleC₁₋₆;
R⁴ est indépendamment sélectionné dans le groupe consistant en hydrogène, fluor, chlore, et méthyle;
R⁵ est hydrogène ou alkyleC₁₋₃, à condition que R⁵ soit alkyleC₁₋₃ uniquement quand il est pris avec Y et les atomes auquel R⁵ et Y sont attachés pour former un hétérocycle à cinq à sept membres;
Y est indépendamment sélectionné dans le groupe consistant en hydroxyméthyle, -C(=O)NH₂, -C(=O)NH(OH), -C(=O) NH (alkyleC₁₋₆), -C(=O)NH(hydroxyalkyle(C₁₋₆). -C(=O)N(alkyleC₁₋₆)₂, -C(=O)NHSO₂alkyle(C₁₋₄), carboxy, tétrazolyle, et -C(=O) alcoxyC₁₋₆; où ledit alcoxy est facultativement substitué par un à deux substituants indépendamment sélectionnés dans le groupe consistant en hydroxy, -NR³⁰R⁴⁰, hétérocyclyle, hétéroaryle, halogène, et -OCH₂CH₂OCH₃; où R³⁰ et R⁴⁰ sont indépendamment sélectionnés dans le groupe consistant en hydrogène, alkyleC₁₋₆, hydroxy, et hydroxyalkyle (C₁₋₄), où lesdits R³⁰ et R⁴⁰ sont facultativement pris ensemble avec les atomes auquel ils sont attachés pour former un cycle monocyclique à cinq à dix membres;
W est indépendamment sélectionné dans le groupe consistant en -C(=O)- et -C(=S)-;
R¹⁰⁰ et R²⁰⁰ sont indépendamment sélectionnés dans le groupe consistant en hydrogène, alkyleC₁₋₆, alcényleC₁₋₆, alkynyleC₁₋₆, aryle, hétéroaryle, cycloalkyle, polycycloalkyle, hétérocyclyle, hydroxy, et arylamino;
où les substituants alkyle, alcényleC₁₋₆, alkynyleC₁₋₆, et alcoxy de R¹⁰⁰ et R²⁰⁰ sont facultativement substitués par un substituant indépendamment sélectionnés dans le groupe consistant en aryle, hétéroaryle, hétérocyclyle, hydroxy, un à trois atomes d'halogène, amino, alkylaminoC₁₋₆, hydroxyalkylamino(C₁₋₆), dialkylamino(C₁₋₆), -C (=O) amino, et -C(=O)alcoxyC₁₋₆;
où lesdits substituants aryle, hétéroaryle, et hétérocyclyle peuvent facultativement être substitués par jusqu'à quatre substituants sélectionnés dans le groupe consistant en alkyleC₁₋₆, alcoxyC₁₋₆, halogène, aryle, amino, alkylamino, dialkylamino, hydroxy, polycycloalkyle, et hétéroaryle, additionnellement ledit hétérocyclyle est facultativement substitué par un à trois substituants oxo;
où ledit aryle, hétéroaryle, et hétérocyclyle de R¹⁰⁰ et R²⁰⁰ sont facultativement substitués par un à quatre substituants indépendamment sélectionnés dans le groupe consistant en alkyle C₁₋₆, trifluoroalkyle, alcoxyC₁₋₆, trifluoroalcoxy, halogène, aryle, amino, alkylamino, dialkylamino, arylamino, hydroxy, polycycloalkyle, et hétéroaryle; additionnement hétérocyclyle est facultativement substitué par un à trois substituants oxo;
et où R¹⁰⁰ et R²⁰⁰ sont facultativement pris avec l'atome d'azote auquel ils sont tous deux attachés pour former un hétérocycle à cinq à dix membres ou bien un hétérocycle benzo fusionné à neuf à dix membres; où l'hétérocycle est facultativement substitué par jusqu'à quatre substituants indépendamment sélectionnés dans le groupe consistant en alkyleC₁₋₆, alcoxyC₁₋₆, halogène, aryle, hétéroaryle, amino, alkylamino, dialkylamino, hydroxy, polycycloalkyle, ou et oxo;
et un isomère, énantiomère, diastéréomère, racémate, ou sel pharmaceutiquement acceptable.

2. Composés de la revendication 1, où R¹ est indépendamment sélectionné dans le groupe consistant en hydrogène, alkyleC₁₋₄, alcoxyC₁₋₄, aryle, hétéroaryle, hétérocyclyle, cycloalkyle benzo fusionné hétérocyclyle benzo fusionné, aryloxy, hétéroaryloxy, hétérocyclyloxy, cycloalkyloxy, -NR¹⁰R²⁰, halogène, hydroxy, et - Salkyle (C₁₋₆) ; où alcoxyC₁₋₄ de R¹ est facultativement substitué par un à trois substituants indépendamment sélectionnés parmi R^{a};
où R^{a} est indépendamment sélectionné dans le groupe consistant en aryle, hétéroaryle, hétérocyclyle, cycloalkyle, dialkylamino, hydroxyalcoxy(C₁₋₆), un à trois atomes d'halogène, et hydroxy;
où R¹⁰ et R²⁰ sont indépendamment sélectionnés dans le groupe consistant en hydrogène, alkyleC₁₋₆, allyle, alkyleC₁₋₆ halogéné, et cycloalkyle; additionnellement R¹⁰ et R²⁰ sont facultativement pris avec les atomes auquel ils sont attachés pour former un cycle monocyclique à cinq à sept membres;
où les substituants aryle et aryloxy de R¹ sont facultativement substitués par un substituant indépendamment sélectionné dans le groupe consistant en alkyleC₁₋₆, alcoxyC₁₋₆, phényle, hétéroaryle, aminocarbonyle, alkylaminocarbonyle, dialkylamino-carbonyle, hydroxy, cyano, nitro, - SO₂alkyle (C₁₋₃), -SO₂ aryle, trifluorométhyle, trifluorométhoxy, et halogène;
et où les substituants hétéroaryle et hétérocyclyle de R¹ sont facultativement substitués par un à trois substituants indépendamment sélectionnés dans le groupe consistant en alkyleC₁₋₆, halogène, et hydroxy;
additionnellement, R¹ et R² sont facultativement pris avec les atomes auquel ils sont attachés pour former un cycle carbocyclique ou hétérocyclique à cinq à sept membres.

3. Composés de la revendication 1 où R¹ est indépendamment sélectionné dans le groupe consistant en hydrogène, éthyle, méthoxy, éthoxy, 2-hydroxyéth-1-oxy, iso-propoxy, iso-butoxy, difluorométhoxy, 2,2,2-trifluoro-éth-1-oxy, benzyloxy, cyclopropylméthoxy, pyridin-3-ylméthoxy, (1-méthyl)-pyrrolidinyl-3-oxy, cyclobutyloxy, cyclopentyloxy, cyclohexyloxy, indazol-1-yle, thiophén-3-yle, [1,3]benzodioxol-5-yle, (2-méthyl)-imidazol-1-yle, (1-méthyl)-pipéridin-4-yloxy, 2-(morpholin-4-yl)-éthoxy, (4-bromo)-pyrazol-1-yl, *N*-pyrrolidinyle, (3,5-diméthyl)-pyrazol-1-yle, morpholin-4-yle, hydroxy, -(OCH₂CH₂)₂OH, phényle (facultativement substitué par un substituant indépendamment sélectionné dans le groupe consistant en -SO₂Me, -C(=O)NH₂, -OCF₃, -CF₃, cyano, fluoro , et méthoxy), amino, cyclopropylamino, allylamino, méthylamino, hydroxy, chloro, et -SMe;
et où R¹ est facultativement pris avec R² pour former un 1,4-dioxanyle ou un oxazinyle.

4. Composés de la revendication 1 où R¹ est méthoxy.

5. Composés de la revendication 1 où R² est indépendamment sélectionné dans le groupe consistant en hydrogène, alkyleC₁₋₄, alcoxyC₁₋₄, alcényloxyC₂₋₄, hydroxy, amino, et halogène; où R¹ et R² sont facultativement pris avec les atomes auquel ils sont attachés pour former un cycle carbocyclique ou hétérocyclique à cinq à sept membres.

6. Composés de la revendication 1, où R² est indépendamment sélectionné dans le groupe consistant en hydrogène, alkyleC₁₋₄, alcoxyC₁₋₄, hydroxy, amino, alkylamino, et halogène; où R² est facultativement pris avec R¹ pour former un cycle 1,4-dioxanyle ou oxazinyle.

7. Composés de la revendication 1 où R² est indépendamment sélectionné dans le groupe consistant en hydrogène, alcoxyC₁₋₄, amino, ou alkylamino; où R² est facultativement pris avec R¹ pour former un cycle 1,4-dioxanyle ou oxazinyle.

8. Composés de la revendication 1 où R³ est indépendamment sélectionné dans le groupe consistant en hydrogène, alkyleC₁₋₆, aryle, hétéroaryle, hétérocyclyle et cycloalkyle; où alkyleC₁₋₆ est facultativement substitué par un substituant indépendamment sélectionné dans le groupe consistant en -C(=O)NH₂, aryle, hétéroaryle, hétérocyclyle, cycloalkyle, carboxy, un à trois atomes d'halogène, hydroxy, et - C (=O) alkyleC₁₋₆.

9. Composés de la revendication 1 où R³ est indépendamment sélectionné dans le groupe consistant en hydrogène, alkyle C₁₋₄, cycloalkyle, et aryle; où alkyleC₁₋₄ est facultativement substitué par un substituant indépendamment sélectionné dans le groupe consistant en -C (=O) alkyleC₁₋₄, -C(=O) NH₂, carboxy, hétérocyclyle, phényle, cyclopropyle, hydroxy, et un à trois atomes de fluor.

10. Composés de la revendication 1, où R³ est indépendamment sélectionné dans le groupe consistant en hydrogène, alkyleC₁₋₄, et phényle; où alkyleC₁₋₄ est facultativement substitué par un substituant indépendamment sélectionné dans le groupe consistant en -C(=O)alkyleC₁₋₄, -C(=O)NH₂, carboxy, morpholinyle, cyclopropyle, hydroxy, et un à trois atomes de fluor.

11. Composés de la revendication 1 où R³ est indépendamment sélectionnés dans le groupe consistant en hydrogène, méthyle, éthyle, et phényle; où méthyle et éthyle sont facultativement substitués par un substituant indépendamment sélectionné dans le groupe consistant en -C(=O)alkyleC₁₋₄, -C(=O)NH₂, carboxy, morpholinyle, cyclopropyle, hydroxy, et un à trois atomes de fluor.

12. Composés de la revendication 1 où R³ est indépendamment sélectionné dans le groupe consistant en hydrogène et 2-hydroxy-éth-1-yle.

13. Composés de la revendication 1 où R⁴ est indépendamment sélectionné dans le groupe consistant en hydrogène, fluor, et chlore.

14. Composés de la revendication 1 où R⁴ est indépendamment sélectionné dans le groupe consistant en hydrogène et fluor.

15. Composés de la revendication 1 où R⁴ est hydrogène.

16. Composés de la revendication 1 où R⁵ est hydrogène ou alkyleC₁₋₃, à condition que R⁵ soit alkyleC₁₋₃ uniquement quand il est pris avec Y et les atomes auquel R⁵ et Y sont attachés pour former un hétérocycle à cinq à sept membres.

17. Composés de la revendication 1 où R⁵ est hydrogène ou méthylène, à condition que R⁵ soit méthylène uniquement quand il est pris avec Y et les atomes auquel R⁵ et Y sont attachés pour former un hétérocycle à cinq membres.

18. Composés de la revendication 1 où R⁵ est hydrogène.

19. Composés de la revendication 1 où Y est indépendamment sélectionné dans le groupe consistant en hydroxyméthyle, -C(=O)NH₂, -C(=O)NH(OH), -C (=O)NH(2-hydroxyéth-1-yle), carboxy, tétrazolyle, -C(=O)NHSO₂ alkyle(C₁₋₄), et -C(=O)alcoxyC₁₋₆; où ledit alcoxy est facultativement substitué par un à deux substituants indépendamment sélectionnés dans le groupe consistant en hydroxy, -NR³⁰R⁴⁰, hétérocyclyle, hétéroaryle, halogène, et -OCH₂CH₂OCH₃; où R³⁰ et R⁴⁰ sont indépendamment sélectionnés dans le groupe consistant en hydrogène et alkyleC₁₋₆.

20. Composés de la revendication 1 où Y est indépendamment sélectionné dans le groupe consistant en carboxy, tétrazolyle, -C(=O)NH(2-hydroxyéth-1-yle) et -C(=O) alcoxyC₁₋₄; où ledit alcoxy est facultativement substitué par un à deux substituants indépendamment sélectionnés dans le groupe consistant en hydroxy, -NH₂, -NHalkyle (C₁₋₄), -Nalkyle(C₁₋₄)₂, hétérocyclyle, halogène, et -OCH₂CH₂OCH₃ .

21. Composés de la revendication 1 où Y est indépendamment sélectionné dans le groupe consistant en carboxy, 1*H*-tétrazol-5-yle, et -C(=O)alcoxyC₁₋₄; où ledit alcoxy est facultativement substitué par un substituant indépendamment sélectionné dans le groupe consistant en hydroxy, -Nme₂, morpholin-1-yle, chloro, et -OCH₂CH₂OCH₃.

22. Composés de la revendication 1 où Y est indépendamment sélectionné dans le groupe consistant en carboxy, 1*H*-tétrazol-5-yle, ou C(=O) éthoxy; où éthoxy est facultativement substitué par un substituant indépendamment sélectionné dans le groupe consistant en hydroxy, chlore, -NMe₂, et -OCH₂CH₂OCH₃-.

23. Composés de la revendication 1 où W est indépendamment sélectionné dans le groupe consistant en -C (=O)- et -C(=S)-.

24. Composés de la revendication 1 où W est -C(=O)-.

25. Composés de la revendication 1 où R¹⁰⁰ et R²⁰⁰ sont des substituants indépendamment sélectionnés dans le groupe consistant en hydrogène, alkyleC₁₋₄, alkynyleC₁₋₄, aryle, hétéroaryle, cycloalkyle, polycycloalkyle, hétérocyclyle, phénylamino, et hydroxy; où ledit aryle, hétéroaryle, et hétérocyclyle peuvent facultativement être substitués par un à quatre substituants indépendamment sélectionnés dans le groupe consistant en alkyleC₁₋₆, hydroxyalkyle (C₁₋₄), halogène, aryle, trifluorométhyle, trifluorométhoxy, hétéroaryle, et hydroxy;
où alkyleC₁₋₄ et alkynyleC₁₋₄ de R¹⁰⁰ et R²⁰⁰ sont facultativement subtitués par un substituant indépendamment sélectionné dans le groupe consistant en hétéroaryle, aryle, hydroxy, un à trois atomes d'halogène, amino, alkylamino et dialkylamino;
où R¹⁰⁰ et R²⁰⁰ sont facultativement pris avec l'atome d'azote auquel ils sont tous deux attachés pour former un hétérocycle à cinq à sept membres ou un hétérocycle benzofusionné à neuf à dix membres; facultativement substitué avec jusqu'à quatre substituants sélectionnés parmi alkyleC₁₋₆, halogène, aryle,et hétéroaryle.

26. Composés de la revendication 1 où R¹⁰⁰ et R²⁰⁰ sont indépendamment sélectionnés dans le groupe consistant en hydrogène, alkyleC₁₋₄, aryle, cycloalkyle, et hétérocyclyle; où ledit aryle et hétérocyclyle sont facultativement substitués par jusqu'à quatre substituants indépendamment sélectionnés dans le groupe consistant en alkyleC₁₋₃, halogène, trifluorométhyle, trifluorométhoxy, hétéroaryle, et hydroxy;
où R¹⁰⁰ et R²⁰⁰ sont facultativement pris avec l'atome d'azote auquel ils sont tous deux attachés pour former un hétérocycle à cinq à sept membres facultativement substitué par jusqu'à quatre substituants indépendamment sélectionnés dans le groupe consistant en alkyleC₁₋₆, halogène, aryle, et hétéroaryle.

27. Composés de la revendication 1 où R¹⁰⁰ et R²⁰⁰ sont indépendamment sélectionnés dans le groupe consistant en hydrogène, alkyleC₁₋₄, aryle, cycloalkyle et un hétérocyclyle à cinq à sept membres, ledit hétérocyclyle est formé quand R¹⁰⁰ et R²⁰⁰ sont pris ensemble avec l'atome d'azote auquel ils sont tous deux attachés);
où ledits aryle et hétérocyclyle sont facultativement substitués par jusqu'à quatre substituants indépendamment sélectionnés dans le groupe consistant en alkyleC₁₋₃, halogène, trifluorométhyle, trifluorométhoxy, et hydroxy.

28. Composés de la revendication 1 où R¹⁰⁰ et R²⁰⁰ sont des substituants indépendamment sélectionnés dans le groupe consistant en hydrogène, méthyle, éthyle, *i*-but-1-yle, cyclohexyle, 2-méthyl-pipéradin-1-yle, et phényle; où ledit phényle est substitué par un ou deux substituants indépendamment sélectionnés dans le groupe consistant en méthyle, chloro, hydroxy, trifluorométhyle, et trifluorométhoxy.

29. Composé de la Formule (Ia) selon la revendication 1 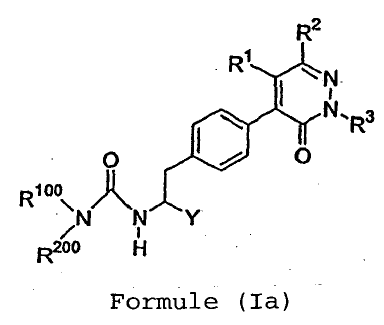 où:
R¹ est indépendamment sélectionné dans le groupe consistant en hydrogène, alkyleC₁₋₄, alcoxyC₁₋₄, aryle, hétéroaryle, hétérocyclyle, cycloalkyle benzo fusionné, hétérocyclyle benzo fusionné, aryloxy, hétéroaryloxy, hétérocyclyloxy, cycloalcoxy, -NR¹⁰R²⁰, halogène, hydroxy, et -Salkyle(C₁₋₆); où alcoxyC₁₋₄ de R¹ est facultativement substitué par un à trois substituants indépendamment sélectionnés parmi R^{a};
où R^{a} est indépendamment sélectionné dans le groupe consistant en aryle, hétéroaryle, cycloalkyle, dialkylamino, hydroxyalcoxy(C₁₋₆), un à trois atomes d'halogène, et hydroxy;
où R¹⁰ et R²⁰ sont indépendamment sélectionnés dans le groupe consistant en hydrogène, alkyleC₁₋₆, allyle, alkyleC₁₋₆ halogéné et cycloalkyle; additionnellement R¹⁰ et R²⁰ sont facultativement pris ensemble avec les atomes auquel ils sont attachés pour former un cycle monocyclique à cinq à sept membres;
où les substituants aryle et aryloxy de R¹ sont facultativement substitués par un substituant indépendamment sélectionné dans le groupe consistant en alkyleC₁₋₆, alcoxyC₁₋₆, phényle, hétéroaryle, aminocarbonyle, alkylaminocarbonyle, dialkylaminocarbonyle, hydroxy, cyano, nitro, -SO₂alkyle(C₁₋₃), -SO₂ aryle, trifluorométhyle, trifluorométhoxy, et halogène;
et où les substituants hétéroaryle et hétérocyclyle de R¹ sont facultativement substitués par un à trois substituants indépendamment sélectionnés dans le groupe consistant en alkyleC₁₋₆, halogène, et hydroxy;
additionnellement, R¹ et R² sont facultativement pris avec les atomes auquel ils sont attachés pour former un cycle carbocyclique ou hétérocyclique à cinq à sept membres;
R² est indépendamment sélectionné dans le groupe consistant en hydrogène, alkyleC₁₋₄, alcoxyC₁₋₄, alcényloxyC₂₋₄, hydroxy, amino, et halogène; où R¹ et R² sont facultativement pris avec les atomes auquel ils sont attachés pour former un cycle carbocyclique ou hétérocyclique à cinq à sept membres;
R³ est indépendamment sélectionné dans le groupe consistant en hydrogène, alkyleC₁₋₄, cycloalkyle, et aryle; où alkyleC₁₋₄ est facultativement substitué par un substituant indépendamment sélectionné dans le groupe consistant en -C(=O)alkyleC₁₋₄, -C(=O)NH₂, carboxy, hétérocyclyle, phényle, cyclopropyle, hydroxy, et un à trois atomes de fluor;
Y est indépendamment sélectionné dans le groupe consistant en carboxy, tétrazolyle, -C(=O)NH(2-hydroxyéth-1-yle) et -C(=O)alcoxyC₁₋₄; où ledit alcoxy est facultativement substitué par un à deux substituants indépendamment sélectionnés dans le groupe consistant en hydroxy, -NH₂, -NH(alkyleC₁₋₄), -N(alkyleC₁₋₄)₂, hétérocyclyle, halogène, et -OCH₂CH₂OCH₃;
R¹⁰⁰ et R²⁰⁰ sont indépendamment sélectionnés dans le groupe consistant en hydrogène,alkyleC₁₋₄, aryle, cycloalkyle, et hétérocyclyle; où lesdits aryle et hétérocyclyle sont facultativement substitués par jusqu'à quatre substituants indépendamment sélectionnés dans le groupe consistant en alkyleC₁₋₃, halogène, trifluorométhyle, trifluorométhoxy, hétéroaryle, et hydroxy;
où R¹⁰⁰ et R²⁰⁰ sont facultativement pris avec l'atome d'azote auquel ils sont tous deux attachés pour former un hétérocycle à cinq à sept membres facultativement substitués par jusqu'à quatre substituants indépendamment sélectionnés dans le groupe consistant en alkyleC₁₋₆, halogène, aryle, et hétéroaryle;
et un isomère optique, énantiomère, diastéréomère, racémate, ou sel pharmaceutiquement acceptable.

30. Composé de Formule (Ia) selon la revendication 1 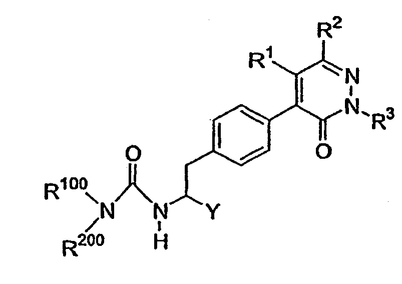 où:
R¹ est indépendamment sélectionné dans le groupe consistant en hydrogène, éthyle, méthoxy, éthoxy, 2-hydroxyéth-1-oxy, iso-propoxy, iso-butoxy, difluorométhoxy, 2,2,2-trifluoro-éth-1-oxy, benzyloxy, cyclopropylméthoxy, pyridin-3-ylméthoxy, (1-méthyl)-pyrrolidinyl-3-oxy, cyclobutyloxy, cyclopentyloxy, cyclohexyloxy, indazol-1-yle, thiophén-3-yle, [1,3]benzodioxol-5-yle, (2-méthyl)-imidazol-1-yle, (1-méthyl)-pipéridin-4-yloxy, 2-(morpholin-4-yl)-éthoxy, (4-bromo)-pyrazol-1-yle, *N-*pyrrolidinyle, (3,5-diméthyl)-pyrazol-1-yle, morpholin-4-yle, hydroxy, -(OCH₂CH₂)₂OH, phényle (facultativement subtitué par un substituant indépendamment sélectionné dans le groupe consistant en -SO₂Me, -C(=O)NH₂, -OCF₃, -CF₃, cyano, fluoro, et méthoxy), amino, cyclopropylamino, allylamino, méthylamino, hydroxy, chloro, et -SME;
et où R¹ est facultativement pris avec R² pour former un 1,4-dioxanyle ou un cycle oxazinyle;
R² est indépendamment sélectionné dans le groupe consistant en hydrogène, alkyleC₁₋₄, alcoxyC₁₋₄, hydroxy, amino, alkylamino, et halogène; où R² est facultativement pris avec R¹ pour former 1,4-dioxanyle ou un cycle oxazinyle;
R³ est indépendamment sélectionné dans le groupe consistant en hydrogène, alkyleC₁₋₄, et phényle; où alkyleC₁₋₄ est facultativement substitué par un substituant indépendamment sélectionné dans le groupe consistant en -C(=O)alkyleC₁₋₄, -C(=O)NH₂, carboxy, morpholinyle, cyclopropyle, hydroxy, et un à trois atomes de fluor;
Y est indépendamment sélectionné dans le groupe consistant en carboxy, 1*H*-tétrazol-5-yle, et -C(=O)alcoxyC₁₋₄; où ledit alcoxy est facultativement substitué par un substituant indépendamment sélectionné dans le groupe consistant en hydroxy, -NMe₂, morpholin-1-yle, chloro, et -OCH₂CH₂OCH₃;
R¹⁰⁰ et R²⁰⁰ sont indépendamment sélectionnés dans le groupe consistant en hydrogène, alkyleC₁₋₄, aryle, cycloalkyle, et un hétérocyclyle à cinq à sept membres, ledit hétérocyclyle étant formé de R¹⁰⁰ et R²⁰⁰ pris avec l'atome d'azote auquel ils sont tous deux attachés;
où lesdits aryle et hétérocyclyles sont facultativement substitués par jusqu'à quatre substituants indépendamment sélectionnés dans le groupe consistant en alkyleC₁₋₃, halogène, trifluorométhyle, trifluorométhoxy, et hydroxy;
et un isomère optique, énantiomère, diastéréomère, racémate, ou sel pharmaceutiquement acceptable.

31. Composé de la Formule (Ib) selon la revendication 1 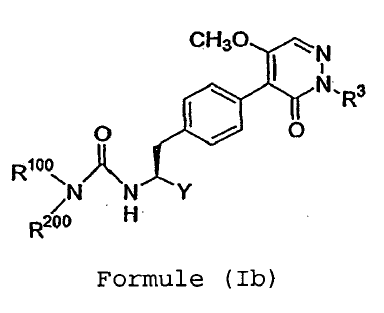 où Y, R³ ,R¹⁰⁰, et R²⁰⁰ sont dépendamment sélectionnés dans le groupe consistant en:
(d désigne un mélange diastéréomère)
| **R³** | **Y** | **R¹⁰⁰** | **R²⁰⁰** | **Stéréochimie de R¹⁰⁰** |
|---|---|---|---|---|
| CH₃ | CO₂H | *i*-propyl | *i*-propyl | |
| (2-OH)-eth-1-yl | CO₂H | (2,6-Cl₂)phenyl | H | |
| (2-OH)-eth-1-yl | CO₂H | cyclohexyl | H | |
| (2-OH)-eth-1-yl | CO₂H | (2-F)phenyl | H | |
| ₃ | CHCO₂H | (4-Me)piperazin-1-yl | | |
| CH₃ | CO₂H | (3-OH)pyrrolidin-1-yl | | d |
| CH₃ | CO₂H | (2-OH)-, eth-1-yl | H | |
| CH₃ | CO₂H | (2-hydroxy methyl)-pyrrolidin-1-yl | H | S |
| CH₃ | CO₂H | benzyl | H | |
| CH₃ | CO₂H | phenyl | methyl | |
| CH₃ | CO₂H | cyclohexyl | methyl | |
| CH₃ | CO₂H | benzyl | methyl | |
| CH₃ | CO₂H | methyl | *i*-butyl | |
| CH₃ | CO₂H | (2-OH)-eth-1-yl | methyl | |
| CH₃ | CO₂H | benzyl, | (2-OH)-eth-1-yl | |
| CH₃ | CO₂H | phenyl | (2-OH)-eth-1-yl | |
| CH₃ | CO₂H | cyclohexyl | (2-OH)-eth-1-yl | |
| CH₃ | CO₂H | *i*-butyl | benzyl | |
| CH₃ | CO₂H | methyl | methyl | |
| CH₃ | CO₂H | cyclohexyl | H | |
| CH₃ | 2-OH-ethoxy carbonyl | *i*-butyl | methyl | |
| CH₃ | CO₂H | 1-methy-piperidin-4-yl | cyclopropyl | |
| CH₃ | CO₂H | norbornan-2-yl | H | S |
| CH₃ | CO₂H | 4-(3-chloro-5-trifluoromethyl-pyridin-2-yl)-piperazin-1-yl | | |
| CH₃ | CO₂H | 4-(adamantan-2-yl)-piperazin-1-yl | | |
| CH₃ | CO₂H | cyclohexyl | hydroxy | |
| CH₃ | CO₂H | benzyl | *i*-propyl | |
| CH₃ | CO₂H | 2-methyl-piperidin-1-yl | | d |
| CH₃ | CO₂H | (1-phenyl)-pyrazol-4-ylmethyl | methyl | |
| CH₃ | CO₂H | 4-(Benzooxazol-2-yl)-[1,4]diazepan-1-yl | | |
| CH₃ | CO₂H | furan-2-ylmethyl | but-2-yn-1-yl | |
| CH₃ | CO₂H | 10-methoxy-3,4,5,6-tetrahydro-2*H*-benzo[b][1,5]oxacinyl | | |
| CH₃ | CO₂H | cyclohexyl | *i*-propyl | |
| CH₃ | CO₂H | 4-(2-hydroxy-eth-1-yl) piperazin-1-ylamino | H | |
| CH₃ | CO₂H | 1-benzyl-pyrrolidin-3-yl | methyl | |
| CH₃ | CO₂H | (4-Hydroxy-1,1-dioxo-tetrahydro-1λ⁶-thiopheny -3-yl | H | 4S,3S |
| CH₃ | CO₂H | 2-(dimethylamin o)-eth-1-yl | ethyl | |
| CH₃ | CO₂H | 2-(phenylamino-methyl)-pyrrolidin-1-yl | | |
| CH₃ | CO₂H | 2-(pyrrolidin-1-yl methyl)-pyrrolidin-1-yl | | S |
| CH₃ | CO₂H | 1-Aza-bicyclo [2.2.2]oct-3- yl | pyridin-3-ylmethyl | d |
| CH₃ | CO₂H | phenyl | *i*-butyl | |
| CH₃ | CO₂H | phenyl | *i*-propyl | |
| CH₃ | CO₂H | 4*-*(*i-*propylamirio)-phenyl | *i*-propyl | |
| CH₃ | CO₂H | (2-methyl-5-hydroxy)-phenyl | ethyl | |
| CH₃ | CO₂H | naphth-1-yl | ethyl | |
| CH₃ | CO₂H | phenyl | phenylamino | |
| CH₃ | CO₂H | phenyl | phenyl | |
| CH₃ | CO₂H | benzyl | phenyl | |
| CH₃ | CO₂H | (2-methyl)-indolin-1-yl | | d |
| CH₃ | CO₂H | phenyl | *n*-propyl | |
| CH₃ | CO₂H | (2-trifluorometho xy)-phenyl | methyl | |
| CH₃ | CO₂H | (2-trifluoromethyl )-phenyl | methyl | |
| CH₃ | CO₂H | 6-fluoro-2-methyl-3,4-dihydro-2*H*-quinolin-1-yl | | d |
| CH₃ | CO₂H | pyridin-2-yl et | methyl | |
| CH₃ | -C(=O) O(CH₂)₂O H | s-but-1-yl | methyl | |

32. Composé selon la revendication 1 sélectionné dans le groupe consistant en
| |
|---|
| 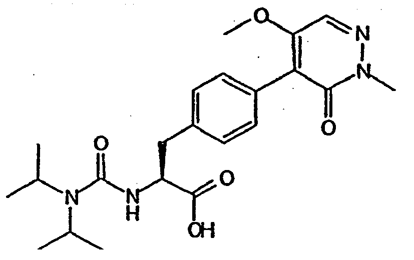 |
| 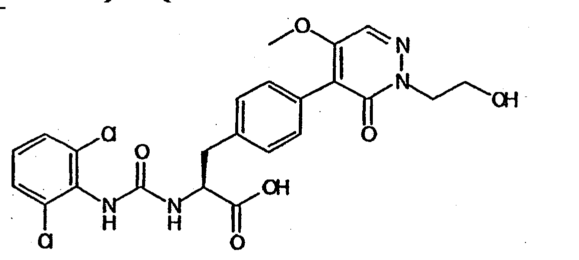 |
| 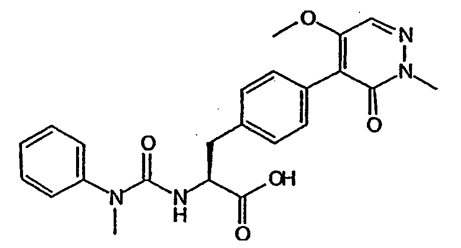 |
| 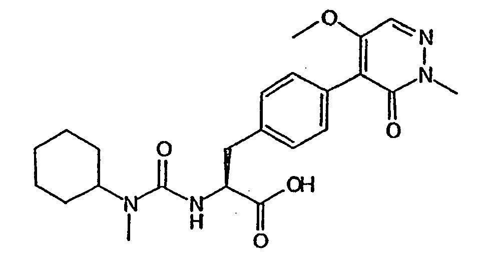 |
| 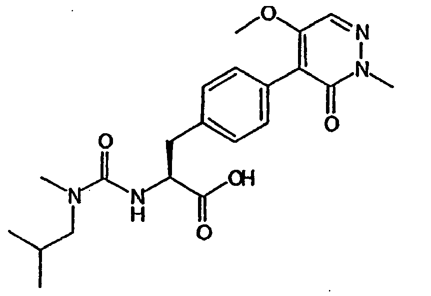 |
| 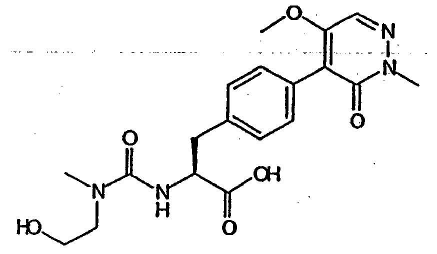 |
| 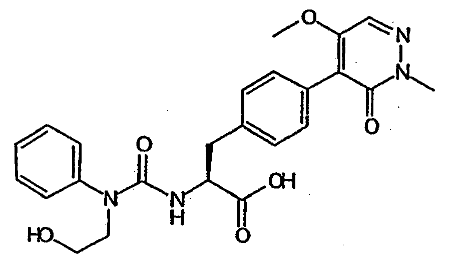 |
| 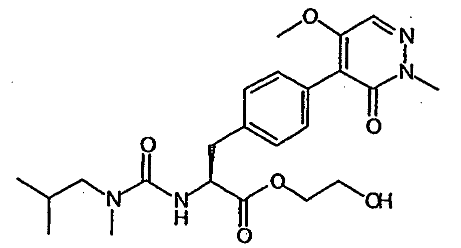 |
| 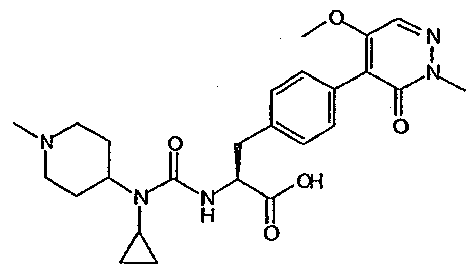 |
| 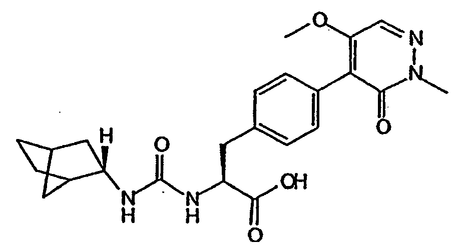 |
| 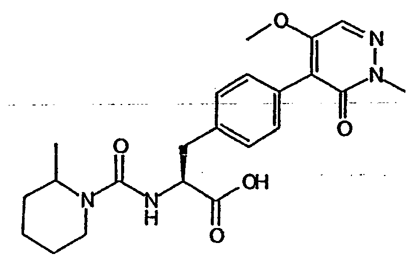 |
| 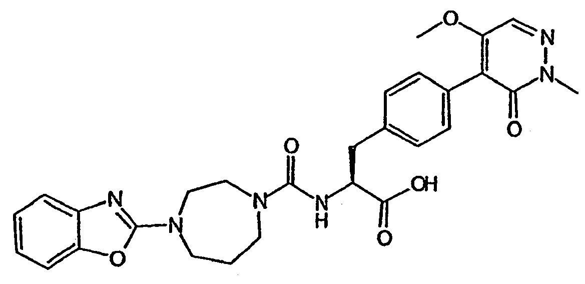 |
| 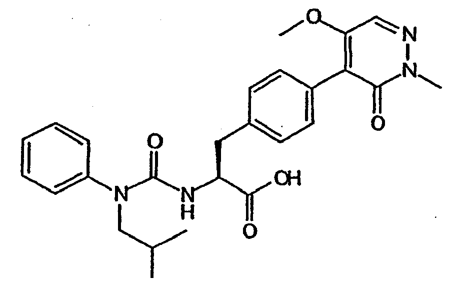 |
| 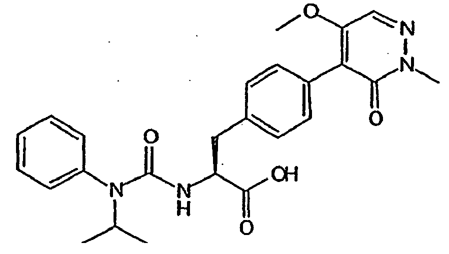 |
| 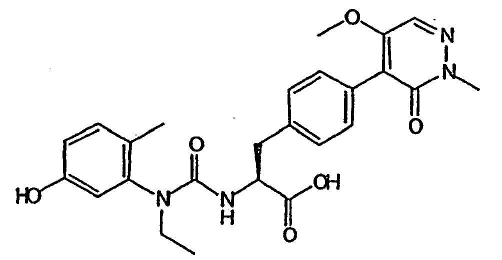 |
| 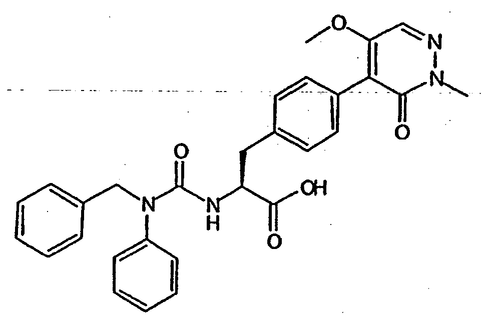 |
| 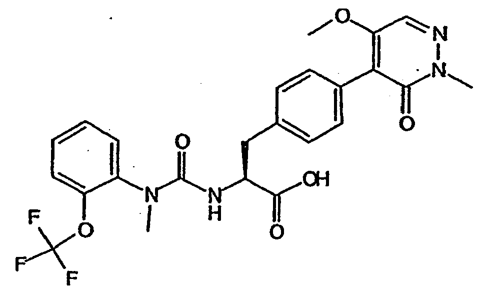 |
| 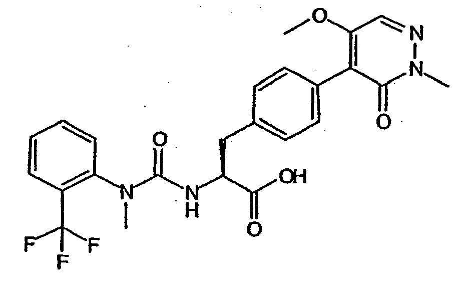 |
| 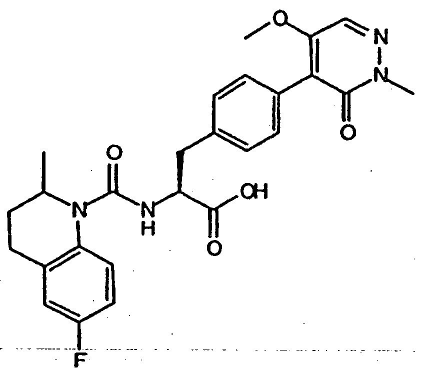 |

33. Composition comprenant le composé de la revendication 1 et un support pharmaceutiquement acceptable.

34. Méthode de production d'une composition comprenant le mélange du composé de la revendication 1 et d'un support pharmaceutiquement acceptable.

35. Utilisation d'un composé selon la revendication 1 dans la préparation d'un médicament pour le traitement ou l'amélioration d'un trouble provoqué par l'intégrine α4, sélectionné dans le groupe consistant en sclérose en plaque, asthme, rhinite allergique, conjonctivite allergique, maladies inflammatoires des poumons, arthrite rhumatoïde, arthrite septique, diabète type I, rejet de transplantation d'organe, resténose, transplantation autologue de moëlle osseuse, séquelles inflammatoires d'infections virales, myocardite, maladie inflammatoire des intestins, néphrite toxique et de base immune, hypersensibilité dermique de contact, psoriasis, métastases de tumeur, athérosclérose et hépatite chez un sujet le nécessitant, où ledit traitement comprend l'administration au sujet d'une quantité thérapeutiquement efficace du composé de la revendication 1.

36. Utilisation d'un composé selon la revendication 29 dans la préparation d'un médicament pour le traitement ou l'amélioration d'un trouble provoqué par l'intégrine α4, sélectionné dans le groupe consistant en sclérose en plaques, asthme, rhinite allergique, conjonctivite allergique, maladies inflammatoires des poumons, arthrite rhumatoïde, arthrite septique, diabète type I, rejet de transplantation d'organe, resténose, transplantation autologue de moëlle osseuse, séquelles inflammatoires d'infections virales, myocardite, maladie inflammatoire des intestins, néphrite toxique et de base immune, hypersensibilité dermique de contact, psoriasis, métastases de tumeur, athérosclérose et hépatite chez un sujet le nécessitant, où ledit traitement comprend l'administration au sujet d'une quantité thérapeutiquement efficace du composé de la revendication 29.

37. Utilisation des revendications 35 et 36 où la maladie inflammatoire des intestins est sélectionnée dans le groupe consistant et comprenant la colite ulcérative et la maladie de Crohn.

38. Utilisation des revendications 35 et 36 où la quantité thérapeutiquement efficace du composé de la revendication 1 est d'environ 0,001 mg/kg/jour à environ 1000 mg/kg/jour.

39. Composé selon l'une quelconque des revendications 1 à 32 à utiliser comme médicament.
